# EUROPEAN PATENT APPLICATION

(11) **EP 4 807 002 A1**
(43) Date of publication of application: **16.09.2026**
(21) Application number: 24887730.0
(22) Date of filing: 18.10.2024
(51) Int. Cl.: C12N 15/113, A61K 31/713, A61K 47/61

(54) **RNAI REAGENT AND COMPOSITION FOR INHIBITING EXPRESSION OF TRANSTHYRETIN (TTR), AND USE**

(30) Priority: 06.11.2023 CN 202311470890
(71) Applicant: Acurna Ltd., Chengdu, Sichuan 610094 (CN); Chengdu Brilliant Pharmaceutical Co., Ltd., Chengdu, Sichuan 610094 (CN)
(72) Inventor: LUO, Feng, Chengdu, Sichuan 610094 (CN); ZANG, Chao, Chengdu, Sichuan 610094 (CN); HUANG, Guobing, Chengdu, Sichuan 610094 (CN); YU, Shuowen, Chengdu, Sichuan 610094 (CN); XU, Ke, Chengdu, Sichuan 610094 (CN); HUANG, Haoxi, Chengdu, Sichuan 610094 (CN); SU, Zhonghai, Chengdu, Sichuan 610094 (CN)
(74) Representative: Kador & Partner Part mbB
(86) International application number: PCT/CN2024/125847
(87) International publication number: WO 2025/098117

(57) **Abstract**

An RNAi agent and composition for inhibiting the expression of transthyretin (TTR), which can be used for treating and/or preventing ATTR-related diseases comprising ATTR-PN (transthyretin amyloidosis polyneuropathy), ATTR-CM (transthyretin amyloidosis cardiomyopathy), senile systemic amyloidosis diseases, and leptomeningeal/central nervous system amyloidosis diseases.

## Description

### TECHNICAL FIELD

The present disclosure pertains to the field of biopharmaceuticals, and relates to an RNAi reagent and a composition for inhibiting transthyretin (TTR) expression, and use, for example, in treating and preventing a transthyretin amyloidosis disease.

### BACKGROUND

According to the central dogma, proteins are translated and synthesized using messenger RNA (mRNA) as a template. Scientists Andrew Fire and Craig Mello elucidated the mechanism of RNA interference (abbreviated as RNAi) in Caenorhabditis elegans, and were awarded the Nobel Prize in Physiology or Medicine in 2006 for this pioneering discovery. RNAi interference can be triggered by exogenous double-stranded RNA (dsRNA) molecules, and the dsRNA is about 20-25 bp in length and is referred to as small or short interference RNA (siRNA). The siRNAs selectively block or cleave complementary mRNA sequences to induce post-transcriptional gene silencing. At present, Alnylam Pharmaceuticals has 5 siRNA drugs approved by the FDA and the like, namely Patisiran, Givosiran, Lumasiran, Inclisiran, and Vutrisiran.

Transthyretin (TTR) (also known as prealbumin) is a serum protein that is expressed primarily in the liver, with other sites of significant expression including the choroid plexus, the retina (specifically retinal pigment epithelial cells), and the pancreas. It participates in the transport of a thyroid hormone (T4) and a retinol-binding protein (RBP). Since the retinol-binding protein binds to retinol (vitamin A), TTR also exerts the transport function of retinol. TTR is a tetramer formed by four identical subunits (monomers), each subunit containing 127 amino acids and being rich in β-sheet structures. Potential factors of a thyroid-stimulating hormone transport protein amyloidosis may be related to its extensive β-sheet structure; X-ray crystallography studies have shown that mutations in certain amino acids may destabilize the tetrameric structure of the protein. The transthyretin tetramer dissociates into monomers, misfolds into an amyloid substance, and deposits in tissues, organs, and extracellular spaces, ultimately progressing to an amyloidosis.

An amyloidosis is a general term for a group of amyloid diseases characterized by amyloid deposits. The amyloid diseases are classified based on their precursor proteins, and the name of an amyloid begins with "A", followed by the abbreviation of the precursor protein; for example, the abbreviation of a transthyretin amyloidosis is ATTR. ATTR includes two types. One type is senile, wild type transthyretin amyloidosis (ATTR wt), in which the transthyretin gene sequence has no mutation; the other type is hereditary, mutant transthyretin amyloidosis (ATTR m), in which the transthyretin gene sequence has pathogenic mutations. ATTR often causes lesions in the heart, peripheral nerves, autonomic nerves, eyes, and meninges, renal lesions are uncommon, and the diseases often accompany one another. The transthyretin (TTR) amyloid deposits in the myocardial interstitium and ultimately progresses to progressive heart failure, namely transthyretin amyloid cardiomyopathy (ATTR-CM); the transthyretin (TTR) amyloid deposits in peripheral nerves, mainly causing peripheral nerve damage, namely transthyretin amyloid polyneuropathy (ATTR-PN). ATTR wt is associated with cardiac amyloidosis in the elderly and is also referred to as senile cardiac amyloidosis (SCA). ATTR wt is associated with systemic multi-organ amyloidosis in the elderly and is also referred to as senile systemic amyloidosis (SSA). When ATTR m is associated with ATTR-PN, it is also referred to as familial amyloidotic polyneuropathy (FAP); when ATTR m is associated with ATTR-CM, it is also referred to as familial amyloidotic cardiomyopathy (FAC).

At present, therapeutic drugs for ATTR (transthyretin amyloidosis) mainly include transthyretin stabilizers and gene silencing drugs. For example, diflunisal is a non-steroidal anti-inflammatory drug that has only been approved domestically for the treatment of arthritis and pain. At present, only tafamidis has obtained approval from the National Medical Products Administration for the treatment of ATTR-PN and ATTR-CM, and has been included in a medical insurance. Tafamidis can stabilize the tetrameric structure of transthyretin, inhibit its dissociation into unstable monomers, and reduce the formation of an amyloid substance. For small interference RNA drugs, all of them reduce the production of transthyretin by inhibiting the expression level of transthyretin messenger RNA (mRNA), thereby ultimately reducing or eliminating the deposition of the pathogenic transthyretin amyloid *in vivo*. For example, the international patent application publication No. WO2013075035A1 of R. G. Kallanthottathil et al. is the patent family of Revusiran and Vutrisiran (which is incorporated herein by reference in its entirety), and has shown that certain other TTR-specific RNA interference (RNAi) reagents inhibit the expression of the TTR gene. The TTR RNAi reagents disclosed herein are previously undisclosed and unknown, and provide highly efficient inhibition of TTR gene expression.

### SUMMARY

The present disclosure provides a novel TTR RNA interference (RNAi) reagent and a composition capable of selectively and efficiently inhibiting TTR gene expression, which can be used for treating and/or preventing a transthyretin amyloidosis disease.

In general, the present disclosure is characterized by a TTR gene-specific RNAi reagent and a composition comprising the RNAi reagent, and a method for inhibiting TTR gene expression *in vitro* and/or *in vivo* using the TTR RNAi reagent of the present disclosure and the composition comprising the TTR RNAi reagent. The TTR RNAi reagent disclosed in the present disclosure can selectively and effectively reduce or inhibit TTR gene expression, thereby reducing a TTR protein level in a subject (e.g., a human or animal subject).

The TTR RNAi reagent may be used in a method for treating and/or preventing an ATTR-related symptom and disease, wherein the symptom and disease include, but are not limited to, ATTR-PN (transthyretin amyloid polyneuropathy), ATTR-CM (transthyretin amyloid cardiomyopathy), senile systemic amyloidosis diseases, and leptomeningeal/CNS (central nervous system) amyloidosis diseases. The method disclosed in the present disclosure comprises administering to a subject (e.g., a human or animal subject) one or more TTR RNAi reagents using methods known in the art, such as subcutaneous injection or intravenous administration.

The TTR RNAi reagent provided by the present disclosure comprises a sense strand (also referred to as a passenger strand) and an antisense strand (also referred to as a guide strand), wherein the sense strand and the antisense strand may be partially, substantially, or completely reversely complementary to each other. The sense strand and the antisense strand of the RNAi reagent described in the present disclosure may each be 16 to 30 nucleotides in length.

In some embodiments, the double-stranded RNAi reagent for inhibiting transthyretin (TTR) gene expression provided by the present disclosure comprises a sense strand and an antisense strand capable of forming a double-stranded region, wherein the antisense strand is complementary to at least a portion of an mRNA encoding transthyretin (TTR). In some embodiments, the antisense strand comprises a sequence motif, the sequence motif being complementary to a portion of an mRNA sequence of a TTR gene. In some embodiments, the complementarity is partial, substantial, or complete reverse complementarity. In some embodiments, the sequence motif is complementary to a fragment at positions 118-140, a fragment at positions 411-433, a fragment at positions 542-566, and/or a fragment at positions 581-603 of the TTR mRNA sequence as shown in SEQ ID NO: 884. In some preferred embodiments, the motif comprises at least 15, 16, 17, 18, 19, 20, 21, 22, or 23 consecutive nucleotides differing by no more than 3, 2, or 1 nucleotide from any one of the following sequences: SEQ ID NOs: 90, 340, 424, 432, 444, 914, and 915.

In some more specific embodiments, the antisense strand comprises at least 15, 16, 17, 18, 19, 20, 21, 22, or 23 consecutive nucleotides differing by no more than 5, 4, 3, 2, or 1 nucleotide from any one of antisense strand nucleotide sequence among the antisense strand nucleotide sequences listed in Table 1.

In some more specific embodiments, the sense strand comprises at least 15, 16, 17, 18, 19, 20, or 21 consecutive nucleotides differing by no more than 5, 4, 3, 2, or 1 nucleotide from any nucleotide sequence among the sense strand nucleotide sequences in Table 1.

In some more specific embodiments, the antisense strand is complementary to a fragment at positions 118-140, a fragment at positions 411-433, a fragment at positions 542-566, and/or a fragment at positions 581-603 of the TTR mRNA sequence as shown in SEQ ID NO: 884, and/or the sense strand comprises at least 15, 16, 17, 18, 19, 20, or 21 consecutive nucleotides from a fragment at positions 118-140, a fragment at positions 411-433, a fragment at positions 542-566, and/or a fragment at positions 581-603 of the TTR mRNA sequence as shown in SEQ ID NO: 884.

In some specific embodiments, the double-stranded RNAi reagent provided by the present disclosure is a combination of sense and antisense strands of siRNAs selected from the pairs listed in Table 2. In some preferred embodiments, the double-stranded RNAi reagent provided by the present disclosure is a combination of sense and antisense strands of siRNAs selected from the pairs listed in Table 1.

In some preferred embodiments of the double-stranded RNAi reagent provided by the present disclosure, the sense strand/antisense strand comprise, or consist of, the nucleotide sequences as shown in SEQ ID NOs: 89/90, SEQ ID NOs: 339/340, SEQ ID NOs: 417/418, SEQ ID NOs: 419/420, SEQ ID NOs: 421/422, SEQ ID NOs: 423/424, SEQ ID NOs: 425/426, SEQ ID NOs: 427/428, SEQ ID NOs: 429/430, SEQ ID NOs: 431/432, SEQ ID NOs: 431/914, SEQ ID NOs: 431/915, SEQ ID NOs: 433/434, SEQ ID NOs: 435/436, SEQ ID NOs: 437/438, SEQ ID NOs: 439/440, SEQ ID NOs: 441/442, SEQ ID NOs: 443/444, SEQ ID NOs: 445/446, SEQ ID NOs: 447/448, SEQ ID NOs: 449/450, SEQ ID NOs: 451/452, SEQ ID NOs: 453/454, SEQ ID NOs: 455/456, SEQ ID NOs: 457/458, SEQ ID NOs: 459/460, SEQ ID NOs: 461/462, SEQ ID NOs: 463/464, SEQ ID NOs: 465/466, SEQ ID NOs: 467/468, or SEQ ID NOs: 469/470, respectively.

In some more specific embodiments, the antisense strand comprises at least 15, 16, 17, 18, 19, 20, 21, 22, or 23 consecutive nucleotides differing by no more than 3, 2, or 1 nucleotide from any one of the following sequences: SEQ ID NOs: 90, 340, 424, 432, 444, 914, and 915.

In some more specific embodiments, the sense strand comprises at least 15, 16, 17, 18, 19, 20, or 21 consecutive nucleotides differing by no more than 3, 2, or 1 nucleotide from any one of the following sequences: SEQ ID NOs: 89, 339, 423, 431, and 443.

In some more preferred embodiments of the double-stranded RNAi reagent provided by the present disclosure, the sense strand/antisense strand comprise, or consist of, the nucleotide sequences as shown in SEQ ID NOs: 89/90, SEQ ID NOs: 339/340, SEQ ID NOs: 423/424, SEQ ID NOs: 431/432, SEQ ID NOs: 431/914, SEQ ID NOs: 431/915, or SEQ ID NOs: 443/444, respectively.

In yet another aspect, the present disclosure provides a double-stranded RNAi reagent comprising at least one modified nucleotide.

In some embodiments, the RNAi reagent of the present disclosure comprises a 4'-modified threose nucleic acid. In some embodiments, the 4'-modified threose nucleic acid has a structure of formula (A1) below: 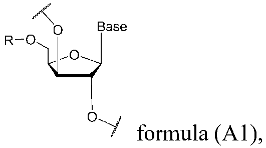 wherein Base represents a natural or modified nucleobase, the natural nucleobase being A, T, C, G, or U, and R represents an alkyl group having 1-30 carbon atoms. In some preferred embodiments, the threose nucleic acid is located at the 5'-terminal end of the sense strand of the RNAi active agent, and R represents an alkyl group having 10-30 carbon atoms, preferably a linear alkyl group having 12 carbon atoms; more preferably, the threose nucleic acid is a 4'-modified threose nucleic acid having a structure of formula (A1') below: 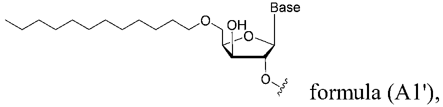 wherein Base is a natural nucleobase A, T, C, G, or U.

In some embodiments, the double-stranded RNAi reagent for inhibiting transthyretin (TTR) gene expression provided by the present disclosure comprises a sense strand and an antisense strand, wherein at least 18 nucleotides are reverse complementary between the sense strand and the antisense strand;
the sense strand comprises, consists of, or consists substantially of a sequence represented by general formula (I) below:
   5'-n₁-n₂-n₃-n₄-n₅-n₆-n₇-n₈-n₉-n₁₀-n₁₁-n₁₂-n₁₃-n₁₄-n₁₅-n₁₆₋n₁₇-n₁₈-n₁₉-n₂₀-n₂₁-3' general formula (I);
the antisense strand comprises, consists of, or consists substantially of a sequence represented by general formula (II) below:
   5'-N₁-N₂-N₃-N₄-N₅-N₆-N₇-N₈-N₉-N₁₀-N₁₁-N₁₂-N₁₃-N₁₄-N₁₅-N₁₆-N₁₇-N₁₈-N₁₉-N₂₀-N₂₁-N₂₂-N₂₃-3' general formula (II),
wherein n₁ to n₂₁ in general formula (I) represent 21 consecutive nucleotides comprised in the sense strand, N₁ to N₂₃ in general formula (II) represent 23 consecutive nucleotides comprised in the antisense strand, and each n and N is independently a modified nucleotide, wherein the modification includes a ribosyl group modification, a backbone modification (e.g., a phosphate group modification), a base modification, and the like.

In some embodiments, in the RNAi reagent described above, the modified nucleotide is selected from: a 2'-O-methyl-modified nucleotide, a 2'-fluoro-modified nucleotide, a 2'-O-methoxyethyl-modified nucleotide, an L-2'-fluoro-modified nucleotide, a 2'-deoxyribonucleotide, a 2'-amino-modified nucleotide, a 2'-alkoxy-modified nucleotide, a 2',3'-seco-nucleotide mimic, an L-2'-O-methyl-modified nucleotide, an L-2'-deoxyribonucleotide, a locked nucleic acid, a threose nucleic acid, a 4'-modified threose nucleic acid, a reverse nucleotide, a reverse 2'-O-methyl-modified nucleotide, a reverse 2'-deoxyribonucleotide, a glycol nucleotide, and a 5'-vinylphosphate nucleotide; more preferably, at least one modified nucleotide in the modified nucleotides is selected from the group consisting of the following: a 4'-modified threose nucleic acid, a 2'-O-methyl-modified nucleotide, a 2'-fluoro-modified nucleotide, a 2'-deoxyribonucleotide, and a combination thereof, wherein the nucleotides are linked via a phosphoester group or a phosphorothioate group.

The 4'-modified threose nucleic acid may have a structure as previously defined herein.

Specific definitions and structures of these modifications can be found in the "siRNA and modified siRNA" section herein.

In some embodiments, in any one of the RNAi reagents described above, nucleotides at positions n₁ and n₂₁ are a threose nucleic acid, a 4'-modified threose nucleic acid, a reverse 2'-deoxyribonucleotide, a reverse nucleotide, an L-2'-O-methyl-modified nucleotide, an L-2'-deoxyribonucleotide, or a 2'-O-methyl-modified nucleotide.

In some embodiments, in any one of the RNAi reagents described above, positions n₇, n₉, n₁₀, and n₁₁ are a 2'-deoxyribonucleotide or a 2'-fluoro-modified nucleotide.

In some embodiments, in any one of the RNAi reagents described above, positions n₂, n₃, n₄, n₅, n₆, n₈, n₁₂, n₁₃, n₁₄, n₁₅, n₁₆, n₁₇, n₁₈, n₁₉, and n₂₀ are a 2'-O-methyl-modified nucleotide.

In some embodiments, in any one of the RNAi reagents described above, the 5' end of the sequence represented by general formula (I) comprises no less than 1 phosphorothioate group; preferably, one or more of a linkage between n₁ and n₂ and a linkage between n₂ and n₃ are achieved by a phosphorothioate group.

Specific definitions and structures of the phosphorothioate group can be found in the "siRNA and modified siRNA" section herein.

In some embodiments, in any one of the RNAi reagents described above, position N₁ is a 5'-vinylphosphate-2'-O-methyl-modified nucleotide, a 2'-O-methyl-modified nucleotide, a threose nucleic acid, a 4'-modified threose nucleic acid, or a reverse nucleotide.

In some embodiments, in any one of the RNAi reagents described above, position N₃ is a 2'-O-methoxyethyl-modified nucleotide or a 2'-O-methyl-modified nucleotide.

In some embodiments, in any one of the RNAi reagents described above, position N₅ is a 2'-deoxyribonucleotide or a 2'-O-methyl-modified nucleotide.

In some embodiments, in any one of the RNAi reagents described above, positions N₆ and N₁₁ are each a 2'-O-methyl-modified nucleotide, a 2'-O-methoxyethyl-modified nucleotide, or a 2'-fluoro-modified nucleotide.

In some embodiments, in any one of the RNAi reagents described above, position N₇ is a 2'-deoxyribonucleotide, a 2'-O-methyl-modified nucleotide, a 2',3'-seco-nucleotide mimic, or a glycol nucleotide.

In some embodiments, in any one of the RNAi reagents described above, one or two of positions N₂₂ to N₂₃ are each an L-2'-O-methyl-modified nucleotide, an L-2'-deoxyribonucleotide, a threose nucleic acid, a 4'-modified threose nucleic acid, a reverse 2'-deoxyribonucleotide, a reverse nucleotide, a reverse 2'-O-methyl-modified nucleotide, or a 2'-O-methyl-modified nucleotide.

In some embodiments, in any one of the RNAi reagents described above, positions N₂, N₁₄, and N₁₆ are each a 2'-deoxyribonucleotide or a 2'-fluoro-modified nucleotide.

In some embodiments, in any one of the RNAi reagents described above, N₄, N₈, N₉, N₁₀, N₁₂, N₁₃, N₁₅, N₁₇, N₁₈, N₁₉, N₂₀, and N₂₁ are each a 2'-O-methyl-modified nucleotide.

In some embodiments, in any one of the RNAi reagents described above, the 5' end and the 3' end of the sequence represented by general formula (II) each have no less than 1 phosphorothioate group; preferably, one or more of a linkage between N₁ and N₂, a linkage between N₂ and N ₃, a linkage between N₂₁ and N₂₂, and a linkage between N₂₂ and N₂₃ are achieved by a phosphorothioate group.

In some embodiments, in any one of the RNAi reagents described above, the sense strand consists of 21 nucleotides, and the antisense strand consists of 23 nucleotides.

In some embodiments, in any one of the RNAi reagents described above, the RNAi reagent is obtained by independently modifying nucleotides at each position of the sense strand and the antisense strand shown in Table 2.

In some embodiments, in any one of the RNAi reagents described above, the antisense strand comprises at least 15 consecutive nucleotides (preferably at least 16, 17, 18, or 19 consecutive nucleotides, and more preferably at least 20, 21, 22, or 23 consecutive nucleotides) from any one of the modified antisense strands listed in Table 6; or the antisense strand differs by no more than 3, 2, or 1 nucleotide within at least 15 consecutive nucleotides (preferably at least 16, 17, 18, or 19 consecutive nucleotides, and more preferably at least 20, 21, 22, or 23 consecutive nucleotides) from the modified antisense strand; or the antisense strand comprises at least 15 consecutive nucleotides differing by no more than 3, 2, or 1 nucleotide from the modified antisense strand; preferably, the antisense strand comprises, consists of, or consists substantially of any one of the modified antisense strands listed in Table 6.

In some embodiments, in any one of the RNAi reagents described above, the sense strand comprises at least 15 consecutive nucleotides (preferably at least 16, 17, 18, or 19 consecutive nucleotides, and more preferably at least 20 or 21 consecutive nucleotides) from any one of the modified sense strands listed in Table 7; or the sense strand differs by no more than 3, 2, or 1 nucleotide within at least 15 consecutive nucleotides (preferably at least 16, 17, 18, or 19 consecutive nucleotides, and more preferably at least 20 or 21 consecutive nucleotides) from the modified sense strand; or the sense strand comprises at least 15 consecutive nucleotides differing by no more than 3, 2, or 1 nucleotide from the modified sense strand; preferably, the sense strand comprises, consists of, or consists substantially of any one of the modified sense strands listed in Table 7.

In some embodiments, in any one of the RNAi reagents described above, the sense strand and the antisense strand are as shown in Table 7 and Table 6, respectively.

In some embodiments, in any one of the RNAi reagents described above, the antisense strand comprises, consists of, or consists substantially of a sequence obtained by independently modifying nucleotides at each position of a nucleotide sequence differing by 0, 1, 2, or 3 nucleotides from one of the following nucleotide sequences:
5'-AUCUAGAACUUUGACCAUCAGAG-3' (SEQ ID NO: 90)
5'-UAGGAGUAGGGGCUCAGCAGGGC-3' (SEQ ID NO: 340)
5'-UAGGUGAAAACACUGCUUUAGGG-3' (SEQ ID NO: 424)
5'-AUAUGAGGUGAAAACACUGCUGG-3' (SEQ ID NO: 432)
5'-AAUGUUUUAUUGUCUCUGCCUGG-3' (SEQ ID NO: 444)
5'-AUAUGAGGUGAAAACACUGCUUU-3' (SEQ ID NO: 914)
5'-AUAUGAGGUGAAAACACUGCUAU-3' (SEQ ID NO: 915);
the difference is, for example, caused by a substitution, an insertion, a deletion, or an inversion of a nucleotide in the listed nucleotide sequence.

In some embodiments, in any one of the RNAi reagents described above, the sense strand comprises, consists of, or consists substantially of a sequence obtained by independently modifying nucleotides at each position of a nucleotide sequence differing by 0, 1, 2, or 3 nucleotides from one of the following nucleotide sequences:
5'-CUGAUGGUCAAAGUUCUAGAU-3' (SEQ ID NO: 89)
5'-CCUGCUGAGCCCCUACUCCUA-3' (SEQ ID NO: 339)
5'-CUAAAGCAGUGUUUUCACCUA-3' (SEQ ID NO: 423)
5'-AGCAGUGUUUUCACCUCAUAU-3' (SEQ ID NO: 431)
5'-AGGCAGAGACAAUAAAACAUU-3' (SEQ ID NO: 443);
the difference is, for example, caused by a substitution, an insertion, a deletion, or an inversion of a nucleotide in the listed nucleotide sequence.

In some embodiments, in any one of the RNAi reagents described above, the RNAi reagent comprises a duplex obtained by independently modifying nucleotides at each position of each strand of any pair of the following double strands:
sense strand: 5'-CUGAUGGUCAAAGUUCUAGAU-3' (SEQ ID NO: 89) and
antisense strand: 5'-AUCUAGAACUUUGACCAUCAGAG-3' (SEQ ID NO: 90);
sense strand: 5'-CCUGCUGAGCCCCUACUCCUA-3' (SEQ ID NO: 339) and
antisense strand: 5'-UAGGAGUAGGGGCUCAGCAGGGC-3' (SEQ ID NO: 340);
sense strand: 5'-CUAAAGCAGUGUUUUCACCUA-3' (SEQ ID NO: 423) and
antisense strand: 5'-UAGGUGAAAACACUGCUUUAGGG-3' (SEQ ID NO: 424);
sense strand: 5'-AGCAGUGUUUUCACCUCAUAU-3' (SEQ ID NO: 431) and
antisense strand: 5'-AUAUGAGGUGAAAACACUGCUGG-3' (SEQ ID NO: 432);
sense strand: 5'-AGCAGUGUUUUCACCUCAUAU-3' (SEQ ID NO: 431) and
antisense strand: 5'-AUAUGAGGUGAAAACACUGCUUU-3' (SEQ ID NO: 914);
sense strand: 5'-AGCAGUGUUUUCACCUCAUAU-3' (SEQ ID NO: 431) and
antisense strand: 5'-AUAUGAGGUGAAAACACUGCUAU-3' (SEQ ID NO: 915);
sense strand: 5'-AGGCAGAGACAAUAAAACAUU-3' (SEQ ID NO: 443) and
antisense strand: 5'-AAUGUUUUAUUGUCUCUGCCUGG-3' (SEQ ID NO: 444).

In some embodiments, in any one of the RNAi reagents described above, the antisense strand comprises, consists of, or consists substantially of a modified nucleotide sequence differing by 0, 1, 2, or 3 nucleotides from one of the following modified nucleotide sequences:
5'-UmsAfsGmGmUmGfAmAmAmAmCmAmCmUfGmCfUmUmUmAmGmsiGsiG-3' (SEQ ID NO: 597)
5'-UmsAfsGmGmdTGmdAAmAmAmCmAmCmUfGmCfUmUmUmAmGmsGmsGm-3' (SEQ ID NO: 604)
5'-AmsUfsAmUmGmAfGmGmUmGmAmAmAmAfCmAfCmUmGmCmUmsGmsGm-3' (SEQ ID NO: 606)
5'-AmsUfsAmUmdGAmdGGmUmGmAmAmAmAfCmAfCmUmGmCmUmsGmsGm-3' (SEQ ID NO: 620)
5'-VpUmsUfsAmUmdGAmdGGmUmGmAmAmAmAfCmAfCmUmGmCmUmsGmsGm-3' (SEQ ID NO: 621)
5'-AmsAfsUmGmUmUfUmUmAmUmU(moe)GmUmCfUmCfUmGmCmCmUmsGmsGm-3' (SEQ ID NO: 644)
5'-AmsAfsUmGmUmUfdTUmAmUmUmGmUmCfUmCfUmGmCmCmUmsGmsGm-3' (SEQ ID NO: 655)
5'-AmsUfsAmUmdGAmdGGmUmGmAmAmAmAfCmAfCmUmGmCmUmsUmsUm-3' (SEQ ID NO: 889)
5'-AmsUfsAmUmdGAmdGGmUmGmAmAmAmAfCmAfCmUmGmCmUmsAmsUm-3' (SEQ ID NO: 891)

wherein the uppercase letters "G", "C", "A", and "U" represent ribonucleotides containing guanine, cytosine, adenine, and uracil as bases, respectively; "T" represents a deoxyribonucleotide containing thymine as a base; the lowercase letter "m" represents that one nucleotide adjacent to the letter m on the left side is a 2'-O-methyl-modified nucleotide; the lowercase letter "f" represents that one nucleotide adjacent to the letter f on the left side is a 2'-fluoro-modified nucleotide; "moe" represents that one nucleotide adjacent to the letter moe on the left side is a 2'-O-methoxyethyl-modified nucleotide; "Vp" represents that one nucleotide adjacent to the letter Vp on the right side is a 5'-vinylphosphate nucleotide; the lowercase letter "i" represents that one nucleotide adjacent to the letter i on the right side is a reverse nucleotide; the lowercase letter "d" represents that one nucleotide adjacent to the letter d on the right side is a 2'-deoxyribonucleotide; the lowercase letter "s" represents that a linkage between two nucleotides adjacent to the letter s on the left and right sides is a phosphorothioate group linkage; the meanings of these symbols in the sequences may also be found in Table 7 herein;
the difference is, for example, caused by a substitution, an insertion, a deletion, or an inversion of a nucleotide in the listed nucleotide sequence.

In some embodiments, in any one of the RNAi reagents described above, the sense strand comprises, consists of, or consists substantially of a modified nucleotide sequence differing by 0, 1, 2, or 3 nucleotides from one of the following modified nucleotide sequences:
5'-ChsUmsAmAmAmGmCfAmGfUfGfUmUmUmUmCmAmCmCmUmAm-3' (SEQ ID NO: 601)
5'-iCsUmsAmAmAmGmCfAmGfUfGfUmUmUmUmCmAmCmCmUmAm-3' (SEQ ID NO: 603)
5'-AmsGmsCmAmGmUmGfUmUfUfUfCmAmCmCmUmCmAmUmAmUm-3' (SEQ ID NO: 605)
5'-AhsGmsCmAmGmUmGfUmUfUfUfCmAmCmCmUmCmAmUmAmUm-3' (SEQ ID NO: 617)
5'-AmsGmsGmCmAmGmAfGmAfCfAfAmUmAmAmAmAmCmAmUmUm-3' (SEQ ID NO: 641)
5'-AhsGmsGmCmAmGmAfGmAfCfAfAmUmAmAmAmAmCmAmUmUm-3' (SEQ ID NO: 653)
5'-AhsGmsCmAmGmUmGfUmUfUfUfCmAmCmCmUmCmAmUmAmAm-3' (SEQ ID NO: 744)

wherein the uppercase letters "G", "C", "A", and "U" represent ribonucleotides containing guanine, cytosine, adenine, and uracil as bases, respectively; the lowercase letter "m" represents that one nucleotide adjacent to the letter m on the left side is a 2'-O-methyl-modified nucleotide; the lowercase letter "f" represents that one nucleotide adjacent to the letter f on the left side is a 2'-fluoro-modified nucleotide; the lowercase letter "h" represents that one nucleotide adjacent to the letter h on the left side is a 4'-modified threose nucleic acid; "i" represents that one nucleotide adjacent to the letter i on the right side is a reverse nucleotide; the lowercase letter "s" represents that a linkage between two nucleotides adjacent to the letter s on the left and right sides is a phosphorothioate group linkage; the meanings of these symbols in the sequences may also be found in Table 7 herein;
the difference is, for example, caused by a substitution, an insertion, a deletion, or an inversion of a nucleotide in the listed nucleotide sequence.

In some embodiments, in any one of the RNAi reagents described above, the RNAi reagent comprises any one of the following groups: SEQ ID NOs: 601 and 597, SEQ ID NOs: 601 and 604, SEQ ID NOs: 603 and 597, SEQ ID NOs: 603 and 604, SEQ ID NOs: 605 and 606, SEQ ID NOs: 605 and 620, SEQ ID NOs: 605 and 889, SEQ ID NOs: 617 and 606, SEQ ID NOs: 617 and 620, SEQ ID NOs: 744 and 621, SEQ ID NOs: 641 and 644, SEQ ID NOs: 641 and 655, SEQ ID NOs: 653 and 644, and SEQ ID NOs: 653 and 655.

In some embodiments, in any one of the RNAi reagents described above, the sense strand and/or the antisense strand are/is linked to a targeting ligand to form a conjugate.

In some embodiments, in the conjugate of the RNAi reagent described above, the targeting ligand comprises one or more (e.g., 1, 2, 3, or 4) N-acetylgalactosamine (GalNAc) and/or a derivative thereof; for example, N-acetylgalactosamine is covalently conjugated to the sense strand and/or the antisense strand in a monovalent, bivalent, trivalent, or tetravalent manner;
N-acetylgalactosamine (GalNAc) has a structural formula represented by formula (B-I):

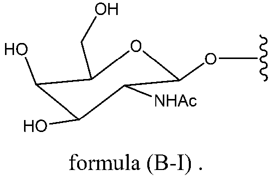

In some embodiments, in any one of the RNAi reagents described above, in the conjugate, the targeting ligand is L96 (GalNAc₃), which has a structural formula represented by formula (B-II):

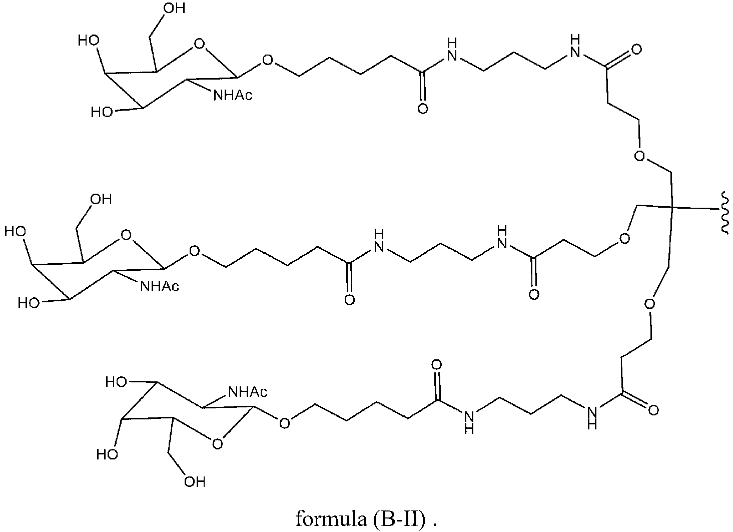

In some embodiments, in any one of the RNAi reagents described above, in the conjugate, the targeting ligand is conjugated to the sense strand.

In some embodiments, in any one of the RNAi reagents described above, in the conjugate, the targeting ligand is conjugated to the 3' end or the 5' end of the sense strand.

In some embodiments, in any one of the RNAi reagents described above, the conjugate has a structural formula represented by formula (B-III): 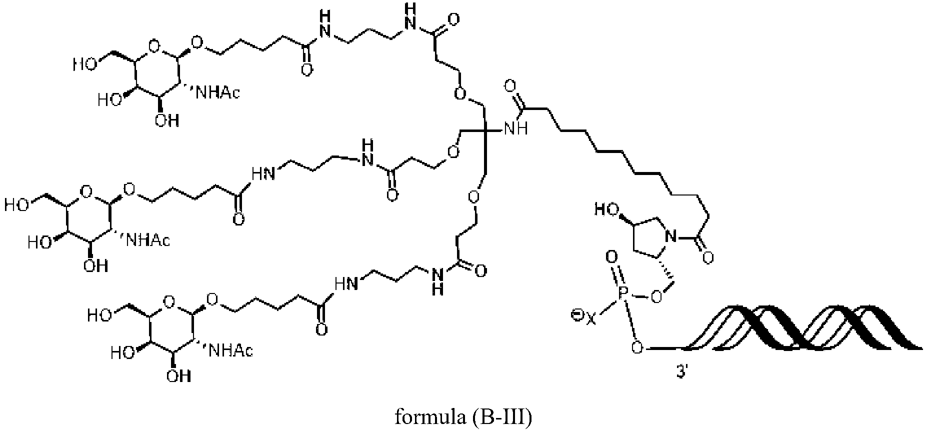 wherein 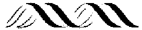 represents a double-stranded oligonucleotide of the RNAi, 3' represents the 3' end of the sense strand, and X is O or S.

In some embodiments, in any one of the RNAi reagents described above, the conjugate is as shown in Table 5.

In some embodiments, in any one of the RNAi reagents described above, the conjugate is BPR-30221218, BPR-30221221, BPR-30221223, BPR-30221228, BPR-30221686, BPR-30221617, BPR-30221618, BPR-30221620, BPR-30221672, BPR-30221676, BPR-30221678, BPR-30222203, BPR-30222212, or BPR-30222218 of the present disclosure.

The present disclosure provides a cell comprising the RNAi reagent described in the present disclosure.

The TTR RNAi reagent disclosed in the present disclosure may be incorporated into a composition. Accordingly, the present disclosure further provides a composition comprising one or more TTR RNAi reagents disclosed in the present disclosure and at least one pharmaceutically acceptable diluent, carrier, and/or excipient. In some embodiments, the composition comprising one or more pharmaceutically acceptable diluents, carriers, and/or excipients in the disclosed TTR RNAi reagent disclosed in the present disclosure is a pharmaceutical composition.

The pharmaceutically acceptable diluent, carrier, or excipient may be any suitable diluent, carrier, and/or excipient conventionally used in the art.

In some embodiments, in the composition described above, the diluent is a PBS buffer, normal saline, or water.

In some embodiments, in any one of the compositions described above, the composition may further comprise one or more additional therapeutic agents, e.g., any therapeutic agent effective in treating and/or preventing a disease and/or condition mediated at least in part by TTR gene expression.

The present disclosure further provides a method for preparing any one of the compositions described above, comprising mixing one or more TTR RNAi reagents disclosed in the present disclosure with at least one pharmaceutically acceptable diluent, carrier, and/or excipient. In some embodiments, the method comprises mixing the TTR RNAi reagent disclosed in the present disclosure with a diluent. In some embodiments, the method comprises mixing the TTR RNAi reagent disclosed in the present disclosure with water or normal saline or a PBS buffer.

The present disclosure further provides a method for inhibiting TTR gene expression in a cell *in vivo* or *in vitro*, which comprises introducing an effective amount of any one of the RNAi reagents described above or any one of the compositions described above into the cell.

In some embodiments, in any one of the methods described above, the cell is in a subject.

In some embodiments, in any one of the methods described above, the subject is a human.

In some embodiments, in any one of the methods described above, the TTR gene expression is inhibited by at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or about 100%.

The present disclosure further provides use of any one of the RNAi reagents described above or any one of the compositions described above in preparing a medicament for treating and/or preventing a disease, condition, or symptom mediated at least in part by TTR gene expression.

In some embodiments, in the use described above, the disease includes ATTR-PN (transthyretin amyloid polyneuropathy), ATTR-CM (transthyretin amyloid cardiomyopathy), senile systemic amyloidosis diseases, and leptomeningeal/central nervous system amyloidosis diseases.

The present disclosure further provides a method for treating and/or preventing a disease, condition, or symptom mediated at least in part by TTR gene expression, which comprises administering to a patient in need thereof a therapeutically effective amount of any one of the RNAi reagents described above or any one of the compositions described above.

In some embodiments, in the method described above, the patient is a human.

In some embodiments, in any one of the methods described above, the disease includes ATTR-PN (transthyretin amyloid polyneuropathy), ATTR-CM (transthyretin amyloid cardiomyopathy), senile systemic amyloidosis diseases, and leptomeningeal/central nervous system amyloidosis diseases.

In some embodiments, in any one of the methods described above, the RNAi reagent or the composition is administered to the patient by subcutaneous, intravenous, intramuscular, intrabronchial, intrapleural, intraperitoneal, intraarterial, lymphatic, and/or cerebrospinal administration.

In some embodiments, in any one of the methods described above, the RNAi reagent or the composition is delivered to the liver, choroid plexus, retina, and/or pancreas of the patient.

In some embodiments, in any one of the methods described above, an administration dose of the RNAi reagent is about 1-300 mg/kg body weight.

In some embodiments, in any one of the methods described above, the administration dose of the RNAi reagent is about 1-250 mg/kg body weight, about 1-200 mg/kg body weight, about 1-150 mg/kg body weight, about 1-100 mg/kg body weight, about 1-90 mg/kg body weight, about 1-80 mg/kg body weight, about 1-70 mg/kg body weight, about 1-60 mg/kg body weight, about 1-50 mg/kg body weight, about 1-40 mg/kg body weight, about 1-30 mg/kg body weight, about 1-20 mg/kg body weight, about 1-10 mg/kg body weight, about 1-9 mg/kg body weight, about 1-8 mg/kg body weight, about 1-7 mg/kg body weight, about 1-6 mg/kg body weight, about 1-5 mg/kg body weight, about 1-4 mg/kg body weight, about 1-3 mg/kg body weight, or about 1-2 mg/kg body weight.

In some embodiments, in any one of the methods described above, the administration dose of the RNAi reagent is about 2-300 mg/kg body weight, about 3-300 mg/kg body weight, about 4-300 mg/kg body weight, 5-300 mg/kg body weight, about 6-300 mg/kg body weight, about 7-300 mg/kg body weight, about 8-300 mg/kg body weight, about 9-300 mg/kg body weight, about 10-300 mg/kg body weight, about 20-300 mg/kg body weight, about 30-300 mg/kg body weight, about 40-300 mg/kg body weight, about 50-300 mg/kg body weight, about 60-300 mg/kg body weight, about 70-300 mg/kg body weight, about 80-300 mg/kg body weight, about 90-300 mg/kg body weight, about 100-300 mg/kg body weight, about 150-300 mg/kg body weight, about 200-300 mg/kg body weight, or about 250-300 mg/kg body weight.

In some embodiments, in any one of the methods described above, the administration dose of the RNAi reagent is about 1 mg/kg body weight, about 2 mg/kg body weight, about 3 mg/kg body weight, about 4 mg/kg body weight, about 5 mg/kg body weight, about 6 mg/kg body weight, about 7 mg/kg body weight, about 8 mg/kg body weight, about 9 mg/kg body weight, about 10 mg/kg body weight, about 20 mg/kg body weight, about 30 mg/kg body weight, about 40 mg/kg body weight, about 50 mg/kg body weight, about 60 mg/kg body weight, about 70 mg/kg body weight, about 80 mg/kg body weight, about 90 mg/kg body weight, about 100 mg/kg body weight, about 150 mg/kg body weight, about 200 mg/kg body weight, about 250 mg/kg body weight, about 300 mg/kg body weight, or any range and value between these values.

In some embodiments, in any one of the methods described above, an administration cycle of the RNAi reagent is once or more times every day, every week, every two weeks, every three weeks, every month, every 2 months, every 3 months, every 4 months, every 5 months, every 6 months, every 7 months, every 8 months, every 9 months, every 10 months, every 11 months, or every 12 months, e.g., 1 time, 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times, 21 times, 22 times, 23 times, 24 times, 25 times, 26 times, 27 times, 28 times, 29 times, 30 times, 31 times, 32 times, 33 times, 34 times, 35 times, 36 times, 37 times, 38 times, 39 times, 40 times, 41 times, 42 times, 43 times, 44 times, 45 times, 46 times, 47 times, 48 times, 49 times, or 50 times every day, every week, every two weeks, every three weeks, every month, every 2 months, every 3 months, every 4 months, every 5 months, every 6 months, every 7 months, every 8 months, every 9 months, every 10 months, every 11 months, or every 12 months.

In some embodiments, in any one of the methods described above, the total number of administrations of the RNAi reagent or composition may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50. For example, the RNAi reagent or composition may be administered about 1, 2, 3, or 4 times.

The present disclosure further provides a kit comprising any one of the RNAi reagents described above or any one of the compositions described above, and optionally instructions for use.

### Beneficial Effects

The novel TTR RNA interference (RNAi) reagents and compositions provided by the present disclosure can selectively and efficiently inhibit TTR gene expression, and can be used for treating and preventing a transthyretin amyloidosis disease, including but not limited to ATTR-PN (transthyretin amyloid polyneuropathy), ATTR-CM (transthyretin amyloid cardiomyopathy), senile systemic amyloidosis diseases, and leptomeningeal/CNS (central nervous system) amyloidosis diseases. In addition, the novel transthyretin RNA interference (RNAi) reagents and compositions provided by the present disclosure can also efficiently inhibit TTR gene expression in the evaluation of free uptake activity in primary hepatocytes, and can also relatively effectively inhibit TTR gene expression *in vivo* in the efficacy evaluation in humanized TTR mice and cynomolgus monkeys. The modification scheme provided by the present disclosure is applicable to all sequences provided in Table 2 and is not limited to the sequences specifically verified in the examples (such as those shown in Table 5). Other unverified RNAi reagents comprising modified sequences have the same or similar technical effects as the RNAi reagent sequences verified in the examples.

Transfection activity evaluation *in vitro* of 235 pairs of siRNAs shown in Table 2 in liver cancer cells HepG2 and Hep3B is performed in the present disclosure. It is found that the TTR inhibition rates of the siRNAs in HepG2 are about 10%, 20%, 30%, 40%, 50%, 60%, 70%, 80%, and 90%, with the maximum inhibition rate being 98.60%; the TTR inhibition rates of the siRNAs in Hep3B are about 10%, 20%, 30%, 40%, 50%, 60%, 70%, 80%, and 90%, with the maximum inhibition rate being 95.85%, as shown in Table 3.

For GalNAc-siRNA, evaluation of free uptake activity in primary hepatocytes is further performed in the present disclosure. In the free uptake assay of primary human hepatocytes, at a dose of 20 nM, the mean value of the maximum inhibition rate of the TTR gene is 94.45%; at a dose of 10 nM, the mean value of the maximum inhibition rate of the TTR gene is 92.05%; at a dose of 5 nM, the mean value of the maximum inhibition rate of the TTR gene is 88.55%, as shown in Table 8. In the free uptake assay of primary cynomogus hepatocytes, at a dose of 20 nM, the mean value of the maximum inhibition rate of the TTR gene is 92.61%; at a dose of 10 nM, the mean value of the maximum inhibition rate of the TTR gene is 84.26%, as shown in Table 9.

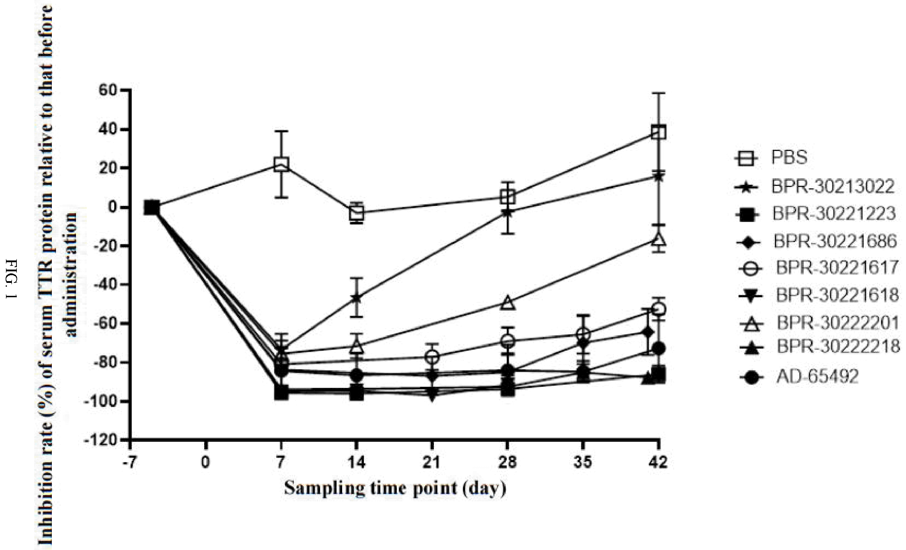

Efficacy evaluation in humanized TTR mice is further performed for the GalNAc-siRNAs, and the results are shown in FIG. 1. On day 7 after administration, BPR-30213022, BPR-30221223, BPR-30221686, BPR-30221617, BPR-30221618, BRP-30222201, and BPR-30222218 can all knock down the serum TTR protein content in mice, with a minimum knockdown rate of 61% and a maximum knockdown rate of 96%. On day 42 after administration, the maximum knockdown rate of the serum TTR protein in mice is 90%.

The efficacy evaluation of GalNAc-siRNAs is also performed in cynomolgus monkeys. Experimental results show that BPR-30221223, BPR-30221228, BPR-30221686, BPR-30221617, BPR-30221618, BPR-30221620, BRP-30222201, BPR-3022218, etc., can all well knock down the serum TTR protein content in cynomolgus monkeys after administration.

The GalNAc-siRNAs of the present disclosure can effectively inhibit TTR gene expression, and can be used for treating and/or preventing an ATTR-related symptom and disease, including but not limited to ATTR-PN (transthyretin amyloid polyneuropathy), ATTR-CM (transthyretin amyloid cardiomyopathy), senile systemic amyloidosis diseases, and leptomeningeal/CNS (central nervous system) amyloidosis diseases.

### BRIEF DESCRIPTION OF THE DRAWINGS

FIG. 1 is a curve graph showing the change in the inhibition rate of the hTTR protein content in the serum of humanized mice relative to that before administration at different time points, after the GalNAc-siRNA conjugate-containing pharmaceutical compositions in Example 6 were administered to the TTR gene humanized mice by single-dose subcutaneous injection of 3 mg/kg.

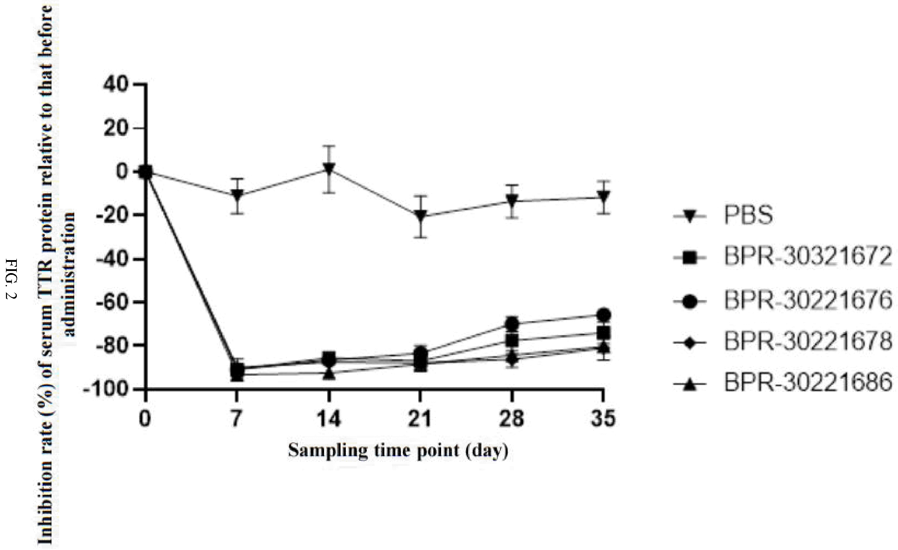

FIG. 2 is a curve graph showing the change in the mean value of the inhibition rate of the serum TTR protein in mice relative to that before administration at different time points for each experimental group, after the GalNAc-siRNA conjugate-containing pharmaceutical compositions in Example 6 were administered to humanized mice B6-hTTR by single-dose subcutaneous administration of 1 mg/kg.

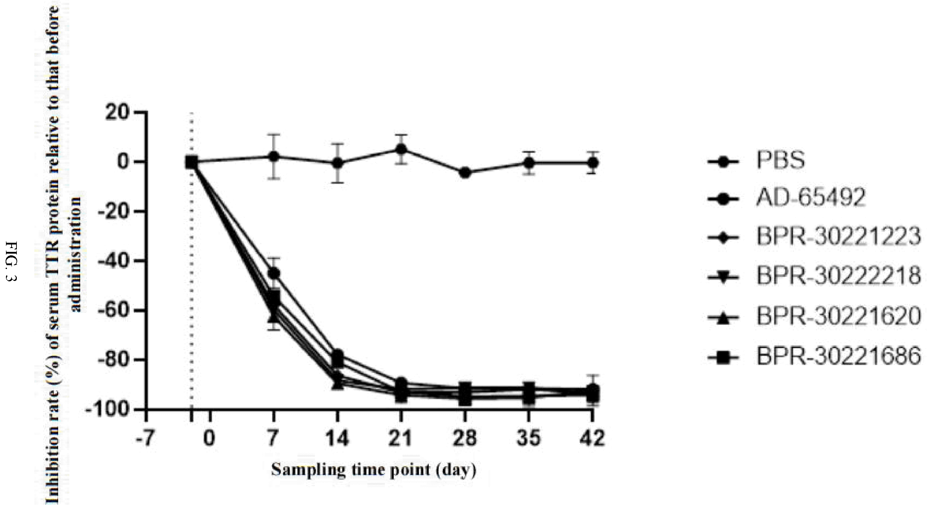

FIG. 3 is a curve graph showing the change in the mean value of the inhibition rate of the serum TTR protein in each group of mice relative to that before administration at different time points from administration to day 42, after the GalNAc-siRNA conjugate-containing pharmaceutical compositions in Example 7 were administered to cynomolgus monkeys by single-dose subcutaneous administration.

### DETAILED DESCRIPTION

The present disclosure will be further illustrated with reference to the following specific examples. It should be understood that the examples below are merely intended to illustrate the present disclosure rather than limit the scope of the present disclosure. Unless otherwise stated, the techniques used in the examples are all conventional operations in the art or experimental methods recommended by the manufacturers of the kits, instruments, and equipment. The reagents and biomaterials used in the examples are commercially available unless otherwise stated.

As used herein, the terms "deoxyribonucleotide" and "DNA" refer to a nucleotide or polynucleotide containing at least one sugar moiety having H instead of OH at its 2' position.

As used herein, "transthyretin" (TTR) is well known in the art. TTR is also referred to as prealbumin, HsT2651, PALB, and TBPA. TTR binds to thyroxine (T4) and retinol-binding protein (RBP), performs the transport functions of thyroxine (T4) and retinol, and it also acts as a protease. The liver secretes TTR into the blood, and the choroid plexus secretes TTR into the cerebrospinal fluid. TTR is also expressed in the pancreas and retinal pigment epithelial cells. The greatest clinical relevance of TTR is that both normal and mutant TTR proteins can form amyloid fibrils and aggregate extracellularly, causing amyloidosis. The sequence of human TTR mRNA can be found in the National Center for Biotechnology Information (NCBI) RefSeq accession number NM_000371.4 (SEQ ID NO: NM_000371.4).The sequence of mouse TTR mRNA can be found in RefSeq accession number NM_013697.2, and the sequence of rat TTR mRNA can be found in RefSeq accession number NM_012681.1. Additional examples of TTR mRNA sequences can be easily obtained using publicly available databases (e.g., GenBank, UniProt, and OMIM).

As used herein, "target sequence" refers to a consecutive portion of a nucleotide sequence of an mRNA molecule formed during transcription of the TTR gene, including mRNA as a product after RNA processing of the initial transcript. In one example, the target portion of the sequence is at least long enough to serve as a substrate for RNAi-guided cleavage at or near the nucleotide sequence portion of the mRNA molecule formed during transcription of the TTR gene. In one example, the target sequence is within the protein-coding region of the TTR gene. In another example, the target sequence is within the 3' UTR of the TTR gene.

The term "interference RNA" or "RNAi" or "interference RNA sequence" includes single-stranded RNA (e.g., mature miRNA or ssRNAi oligonucleotide) or double-stranded RNA (i.e., duplex RNA, such as siRNA, dsRNA, shRNA, aiRNA, or miRNA), which, when the interference RNA is in the same cell as the target gene or sequence, is capable of reducing or inhibiting the target gene expression (e.g., by mediating degradation and inhibiting translation of the mRNA complementary to the interference RNA sequence). Interference RNA thus refers to a single-stranded RNA complementary to a target sequence or a double-stranded RNA formed by two complementary strands or by a single self-complementary strand.

As used herein, the term "siRNA" refers to a small inhibitory RNA duplex that induces the RNA interference (RNAi) pathway, mediating targeted cleavage of RNA transcripts through the RNA-induced silencing complex (RISC) pathway and guiding sequence-specific degradation of mRNA. siRNAs may vary in length (generally between 18-30 base pairs) and have varying degrees of complementarity with the target mRNA.

Generally, the siRNA comprises a sense strand (SS) and an antisense strand (AS). The majority of nucleotides of each strand of the siRNA are ribonucleotides; one or both of the strands may further comprise one or more non-ribonucleotides, e.g., deoxyribonucleotides and/or modified nucleotides. Additionally, as used herein, "siRNA" may comprise ribonucleotides with chemical modifications; siRNA may comprise substantial modifications at a plurality of nucleotides. As used herein, the term "modified nucleotide" refers to a nucleotide independently having a modified sugar moiety, a modified internucleotide linkage, and/or a modified nucleobase. Therefore, the term "modified nucleotide" encompasses a substitution, an addition, or a removal (e.g., of functional groups or atoms) on the internucleotide linkage, sugar moiety, or base. Modifications suitable for use in the reagents of the present disclosure include all types of modifications disclosed herein or known in the art.

The term "antisense strand" or "guide strand" refers to a strand that is substantially complementary to a target sequence (e.g., TTR mRNA). As used herein, the term "region of complementarity" refers to a region where the antisense strand is substantially complementary to the target sequence, wherein mismatches may be present within the internal region or the terminal region of the molecule.

The term "sense strand" or "passenger strand" refers to an RNAi strand comprising a region substantially complementary to the antisense strand as defined herein.

The term "nucleotide overhang" refers to at least one unpaired nucleotide which protrudes from the duplex structure (e.g., dsRNA) of the RNAi. For example, a nucleotide overhang exists when the 3' end of one strand of dsRNA extends beyond the 5' end of the other strand, or vice versa. dsRNA may comprise an overhang having at least one nucleotide; the overhang may comprise at least one nucleotide, at least two nucleotides, at least three nucleotides, at least four nucleotides, at least five nucleotides, or more. A nucleotide overhang can comprise or consist of a nucleotide/nucleoside analog (including a deoxyribonucleotide/nucleoside). One or more overhangs can be on the sense strand, the antisense strand, or a combination thereof. Additionally, one or more nucleotides of the overhang can be present at the 5' end, the 3' end, or both ends of the antisense strand of the dsRNA.

The uppercase letters "G", "C", "A", and "U" represent ribonucleotides containing guanine, cytosine, adenine, and uracil as bases, respectively; "T" represents a deoxyribonucleotide containing thymine as a base.

The term "complementary" or "complementarity" refers to the ability of polynucleotides to pair with each other to form base pairs. Base pairs are typically formed by hydrogen bonding between nucleotide units in antiparallel polynucleotide strands. Complementary polynucleotide strands can base pair in a Watson-Crick manner (e.g., A with T, A with U, and C with G), or in any other manner that allows for the formation of a duplex, such as a Hoogsteen base-pairing manner or a wobble base-pairing manner.

The term "conjugate" refers to a new compound generated after the molecules of two or more compounds are covalently linked (conjugated) through a bivalent or multivalent compound molecule having a linking function. The conjugate can be represented by GalNAc-siRNA, wherein GalNAc can be L96 (i.e., a GalNAc₃ ligand), serving as a liver-targeting delivery vehicle. L96 can form a conjugate by conjugating with the 3' end of the sense strand of the siRNA, or it can form a conjugate by conjugating with the 5' end of the sense strand of the siRNA.

As used herein, the phrase "inhibiting TTR gene expression" includes inhibiting the expression of any TTR gene (such as a mouse TTR gene, a rat TTR gene, a monkey TTR gene, or a human TTR gene) as well as variants or mutants of the TTR gene encoding a TTR protein.

Inhibiting TTR gene expression includes any level of TTR gene inhibition, for example, at least partial inhibition of TTR gene expression, such as at least about 20% inhibition. In certain embodiments, the inhibition is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

TTR gene expression can be evaluated based on the level of any variable related to TTR gene expression, such as the TTR mRNA level or the TTR protein level. Inhibition can be evaluated by a decrease in the absolute or relative level of one or more of these variables compared with a control level. The control level can be any type of control level used in the art, such as a pre-administration baseline level or a level determined from a similar subject, cell, or sample that is untreated or treated with a control (e.g., a buffer-only control or an inert agent control).

The subject includes any human or non-human animal. The term "non-human animal" includes all vertebrates, for example, mammals and non-mammals, such as non-human primates, sheep, dogs, cats, horses, cattle, chickens, amphibians, and reptiles. As used herein, the terms "cyno" or "monkey" both refer to cynomolgus monkeys.

The terms "therapeutically effective amount", "therapeutically effective dose", and "effective amount" refer to an amount of the siRNA, GalNAc-siRNA, or pharmaceutical composition of the present disclosure, when administered to a cell, tissue, or subject alone or in combination with another therapeutic drug, effective to prevent or ameliorate the symptoms of one or more diseases or conditions or the development of the diseases or conditions. The effective amount for a specific subject may vary based on a variety of factors, such as the disease to be treated, the overall health status of the patient, the method, route, and dose of administration, and the severity of side effects. The effective amount may be the maximum dose or administration regimen that avoids significant side effects or toxic effects. When an active ingredient is administered to an individual alone, the therapeutically effective amount refers to the amount of the individual ingredient. When a combination is administered, the therapeutically effective amount refers to the combined amount of the active ingredients that produces a therapeutic effect, regardless of whether they are administered in combination, sequentially, or simultaneously. The therapeutically effective amount will alleviate symptoms usually by at least 10%, usually by at least 20%, preferably by at least about 30%, more preferably by at least 40%, and most preferably by at least 50%.

The term "treat", "treating", "treated", or "treatment" means a beneficial or desired result, including, for example, reduction of transthyretin amyloid deposits. The term "treat", "treating", "treated", or "treatment" also includes, but is not limited to, the relief or amelioration of one or more symptoms of ATTR-PN, ATTR-CM, SSA, and leptomeningeal/central nervous system amyloidosis diseases.

"Treat", "treating", "treated", and "treatment" and "ameliorate", "ameliorating", "ameliorated", and "amelioration" can be used interchangeably herein. These terms refer to methods of obtaining beneficial or desired results, including but not limited to therapeutic benefits.

### siRNA and Modified siRNA

Provided herein are siRNAs for inhibiting TTR gene expression, each siRNA comprising a sense strand and an antisense strand. The sequence information of BPR-302045, BPR-302170, BPR-302212, BPR-302216, and BPR-302222, as well as their specific positions on TTR mRNA (NM_000371.4), are shown in Table 1. The sense strand (SS) of the siRNA has 21 nucleotides, and the antisense strand (AS) has 23 nucleotides; the 3' end of the antisense strand (AS) has a 2-base overhang. The antisense strand and the sense strand have at least 18 consecutive nucleotides in reverse complementary pairing.

**Table 1**

| siRNA No. | Strand type | SEQ ID NO | Region of position in TTR mRNA (NM_000371.4) |
|---|---|---|---|
| BPR-302045 | SS | 89 | 120-140 |
| | AS | 90 | 118-140 |
| BPR-302170 | SS | 339 | 413-433 |
| | AS | 340 | 411-433 |
| BPR-302212 | SS | 423 | 542-562 |
| | AS | 424 | 540-562 |
| BPR-302216 | SS | 431 | 546-566 |
| | AS | 432 | 544-566 |
| BPR-302222 | SS | 443 | 583-603 |
| | AS | 444 | 581-603 |
| BPR-302236 | SS | 431 | 546-566 |
| | AS | 914 | 544-566 |
| BPR-302237 | SS | 431 | 546-566 |
| | AS | 915 | 544-566 |

In order to increase the specificity, stability, and effectiveness of the double-stranded ribonucleic acid, each nucleotide of the sense strand (SS) and the antisense strand (AS) of the siRNA is independently subjected to a nucleotide modification, including a ribosyl group modification, a backbone modification (e.g., a phosphate group modification), a base modification, and the like.

The modified nucleotide is selected from: a 2'-O-methyl-modified nucleotide, a 2'-fluoro-modified nucleotide, a 2'-O-methoxyethyl-modified nucleotide, an L-2'-fluoro-modified nucleotide, a 2'-deoxyribonucleotide, a 2'-amino-modified nucleotide, a 2'-alkoxy-modified nucleotide, a 2',3'-seco-nucleotide mimic, an L-2'-O-methyl-modified nucleotide, an L-2'-deoxyribonucleotide, a locked nucleic acid, a threose nucleic acid, a 4'-modified threose nucleic acid, a reverse nucleotide, a reverse 2'-O-methyl-modified nucleotide, a reverse 2'-deoxyribonucleotide, a glycol nucleotide, and a 5'-vinylphosphonate nucleotide,
wherein the 2'-O-methyl-modified nucleotide refers to a nucleotide formed by substituting the hydroxyl group at position 2' of the ribosyl group of the nucleotide with a methoxy group, the structure of which is represented by A1;
the 2'-fluoro-modified nucleotide refers to a nucleotide formed by substituting the hydroxyl group at position 2' of the ribosyl group of the nucleotide with fluorine, the structure of which is represented by A2;
the 2'-O-methoxyethyl-modified nucleotide refers to a nucleotide formed by substituting hydrogen of the hydroxyl group at position 2' of the ribosyl group of the nucleotide with a methoxyethyl group, the structure of which is represented by A3;
the L-2'-fluoro-modified nucleotide refers to a nucleotide in which the chirality of positions 1', 2', 3', and 4' of the ribosyl group of the nucleotide is completely opposite to that of a natural nucleotide, and the hydroxyl group at position 2' is substituted with fluorine, the structure of which is represented by A4;
the 2'-deoxyribonucleotide (DNA) refers to a nucleotide formed by substituting the hydroxyl group at position 2' of the ribosyl group of the nucleotide with hydrogen, the structure of which is represented by A5;
the 2'-amino-modified nucleotide refers to a nucleotide formed by substituting the hydroxyl group at position 2' of the ribosyl group of the nucleotide with an amino group, the structure of which is represented by A6;
the 2'-alkoxy-modified nucleotide refers to a nucleotide formed by substituting hydrogen of the hydroxyl group at position 2' of the ribosyl group of the nucleotide with an alkyl group, the structure of which is represented by A7;
the 2',3'-seco-nucleotide mimic refers to a nucleotide formed by breaking the carbon-carbon bond between positions 2' and 3' of the ribosyl group of the nucleotide, the structure of which is represented by A8;
the L-2'-O-methyl-modified nucleotide refers to a nucleotide in which the chirality of positions 1', 2', 3', and 4' of the ribosyl group of the nucleotide is completely opposite to that of a natural nucleotide, and the hydroxyl group at position 2' is substituted with a methoxy group, the structure of which is represented by A9;
the L-2'-deoxyribonucleotide refers to a nucleotide in which the chirality of positions 1', 3', and 4' of the ribosyl group of the nucleotide is completely opposite to that of a natural nucleotide, and the hydroxyl group at position 2' is substituted with hydrogen, the structure of which is represented by A10;
the locked nucleic acid (LNA) refers to a nucleotide in which an oxymethylene bridge is formed between position 2'-O and position 4'-C of the ribosyl group of the nucleotide, the structure of which is represented by A11;
the threose nucleic acid (TNA) refers to a nucleotide having a threose structure, the structure of which is represented by A12;
the structure of the 4'-modified threose nucleic acid is represented by A13, wherein R represents a C10 to C30 alkyl group; preferably, R represents a C10 to C30 linear alkyl group;
the reverse nucleotide refers to a nucleotide in which position 3' of the ribosyl group of the nucleotide is substituted with phosphoric acid and linked to the 3' end of a non-reverse nucleotide, and position 5' is a hydroxyl group and linked to the 5' end of a non-reverse nucleotide, the structure of which is represented by A14; herein, unless otherwise stated, "reverse nucleotide" does not include any substitution of groups at other positions (e.g., the hydroxyl group at position 2');
the reverse 2'-O-methyl-modified nucleotide refers to a nucleotide formed by substituting position 3' of the ribosyl group of the nucleotide with phosphoric acid, position 5' being a hydroxyl group and the hydroxyl group at position 2' being substituted with a methoxy group, the structure of which is represented by A15;
the reverse 2'-deoxyribonucleotide refers to a nucleotide formed by substituting position 3' of the ribosyl group of the nucleotide with phosphoric acid, position 5' being a hydroxyl group and the hydroxyl group at position 2' being substituted with hydrogen, the structure of which is represented by A16;
the glycol nucleotide refers to a nucleotide analog having a glycol structure, the structure of which is represented by A17, wherein R represents H, OH, or an alkoxy group;
the 5'-vinylphosphonate nucleotide refers to a nucleotide formed by substituting position 5' of the ribosyl group of the nucleotide with vinylphosphonate, and the hydroxyl group at position 2' being substituted with hydrogen, the structure of which is represented by A18.

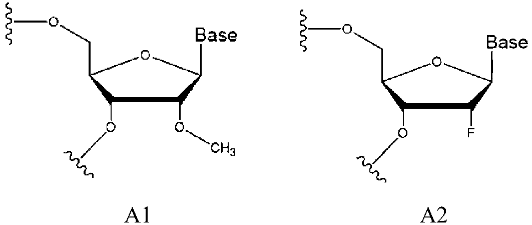

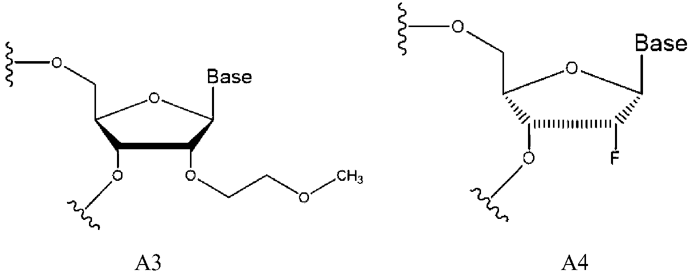

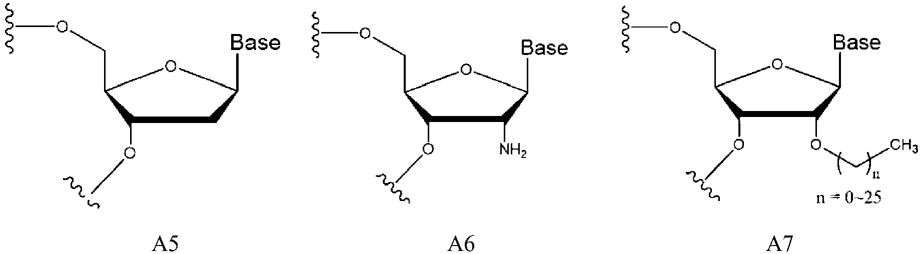

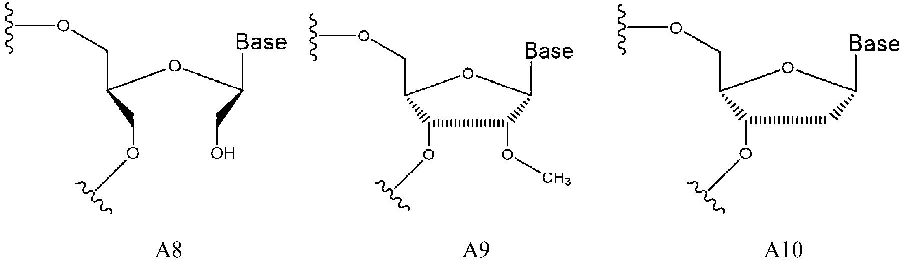

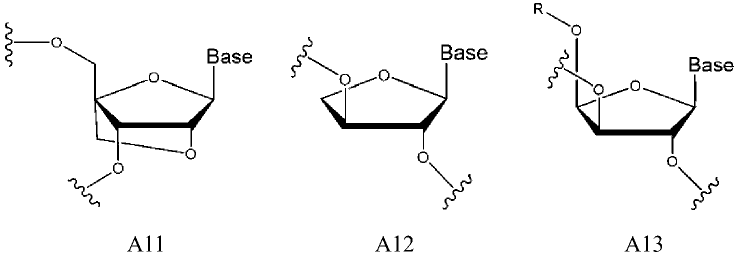

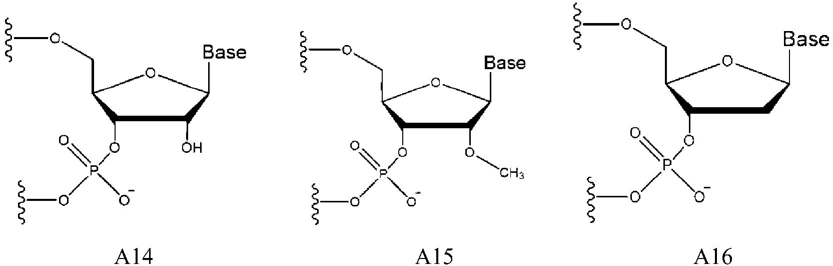

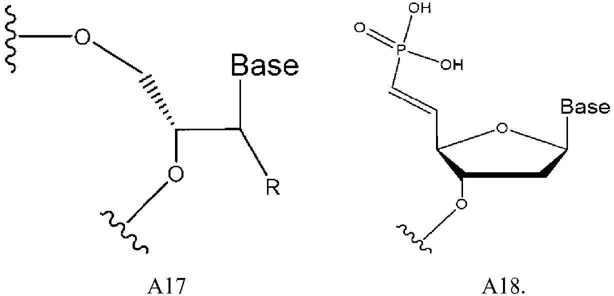

At least one of the phosphoester groups in the phosphate-sugar backbone of at least one single strand of the sense strand and the antisense strand is a phosphoester group having a modification group. The phosphoester group having a modification group is a phosphorothioate group formed by substituting at least one oxygen atom in the phosphodiester bond of the phosphoester group with a sulfur atom, for example, substituting a non-bridging oxygen atom in the phosphodiester bond with one sulfur atom, replacing the phosphodiester bond with a phosphorothioate diester bond, i.e., the linkage between two nucleotides is a phosphorothioate group linkage. This modification can stabilize the structure of the siRNA and maintain high specificity and high affinity of base pairing.

The structural formula of the phosphorothioate group mentioned herein is represented by A19:

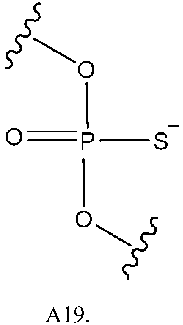

Details of the modified sense strand are shown in Table 7, and details of the modified antisense strand are shown in Table 6.

Those skilled in the art are aware that the siRNAs described herein can be obtained by conventional siRNA preparation methods in the art (e.g., methods of solid-phase synthesis and liquid-phase synthesis). Among them, the solid-phase synthesis has commercialized customized services. Modified nucleotides can be introduced into the siRNA described herein by using nucleotide monomers having corresponding modifications; the methods for preparing nucleotide monomers having corresponding modifications and the methods for introducing modified nucleotides into siRNA are also well known to those skilled in the art.

### GalNAc-siRNA Conjugate

The conjugate includes the siRNA described above disclosed herein and a conjugated group linked to the siRNA. Generally, the conjugated group comprises at least one pharmaceutically acceptable targeting group (i.e., a targeting ligand) and optionally a linker, and the siRNA, the linker, and the targeting ligand are linked in sequence.

In some embodiments, the conjugated group can be linked to an end of a strand of the siRNA, such as the 5' end and/or the 3' end of the sense strand, and/or the 5' end and/or the 3' end of the antisense strand. When the conjugated group is linked to the end of a strand of the siRNA, the conjugated group is usually linked to a phosphate group of a nucleotide.

The present disclosure uses N-acetylgalactosamine (GalNAc) as a delivery vehicle for siRNA drugs to specifically deliver the drugs to hepatic parenchymal cells to reduce or inhibit TTR gene expression, and thereby reduce the TTR protein level in a subject (e.g., a human or animal subject), so as to achieve the purpose of preventing and/or treating an ATTR-related symptom and disease, wherein the symptom and disease include, but are not limited to, ATTR-PN (transthyretin amyloid polyneuropathy), ATTR-CM (transthyretin amyloid cardiomyopathy), senile systemic amyloidosis diseases, and leptomeningeal/CNS (central nervous system) amyloidosis diseases.

In some embodiments, the 3' end of the sense strand of the siRNA disclosed herein is conjugated to three N-acetylgalactosamine (GalNAc) molecules (i.e., L96) to obtain a GalNAc-siRNA conjugate, the structural formula of which is represented by formula (B-III): 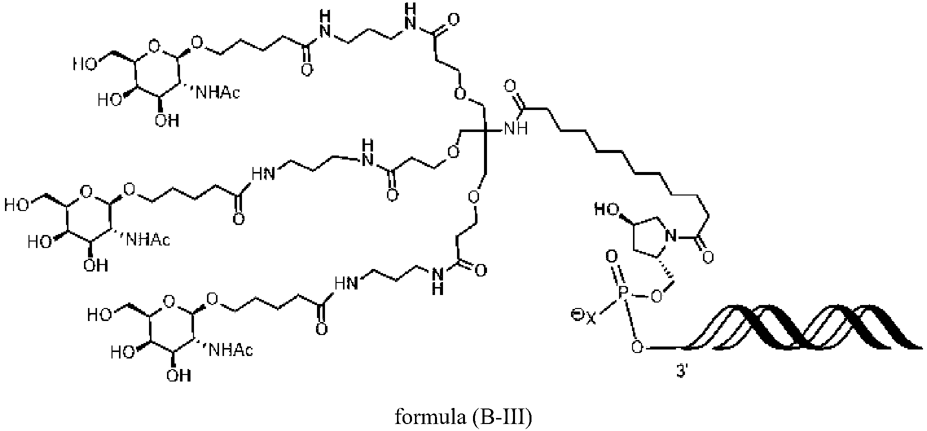 wherein 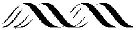 represents a double-stranded oligonucleotide of the RNAi, 3' represents the 3' end of the sense strand, and X is O or S.

In some embodiments, specific information of the conjugates is shown in Table 5.

The conjugates described above can be synthesized by methods already described in detail in the prior art.

### Pharmaceutical Composition

The present disclosure provides a pharmaceutical composition comprising the specifically modified siRNA or GalNAc-siRNA of the present disclosure as an active ingredient and a pharmaceutically acceptable diluent, carrier, and/or excipient (e.g., a PBS buffer, normal saline, or water). The purpose of the pharmaceutical composition is to facilitate administration to an organism and favor the absorption of the active ingredient to exert biological activity.

The pharmaceutically acceptable diluent, carrier, and/or excipient used in the present disclosure includes any suitable pharmaceutically acceptable diluent, carrier, and/or excipient well known in the art.

### Application

The present disclosure provides the disclosed specifically modified siRNA, GalNAc-siRNA, and pharmaceutical composition for use in treating and/or preventing a TTR gene expression-mediated (e.g., transthyretin amyloidosis-related) disease or condition.

The present disclosure provides an siRNA, a GalNAc-siRNA, or a pharmaceutical composition for use in inhibiting TTR gene expression in a cell *in vivo* or *in vitro,* which promotes degradation of mRNA of the TTR gene via an RNAi mechanism, thereby reducing TTR gene expression in cells. In some embodiments, the TTR gene expression is reduced or inhibited by at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or about 100%.

In some embodiments of the treatment and prevention provided by the present disclosure, the subject is a mammal, such as a primate, a rodent, or a human. Administration of the GalNAc-siRNA or the pharmaceutical composition of the present disclosure leads to a reduction of the serum TTR protein in a subject. Currently, vutrisiran and patisiran have demonstrated that reducing the content of the TTR protein in serum can effectively treat and prevent a TTR gene expression-mediated (e.g., transthyretin amyloidosis-related) disease or condition, including ATTR-PN (transthyretin amyloid polyneuropathy), ATTR-CM (transthyretin amyloid cardiomyopathy), senile systemic amyloidosis diseases, and the like.

Administration to a patient can be performed through any suitable route known in the art, the route including, but not limited to: subcutaneous, intravenous, intramuscular, intrabronchial, intrapleural, intraperitoneal, intraarterial, lymphatic, and/or cerebrospinal.

The dosage of the RNAi reagent and the composition of the present disclosure can be determined according to the body weight, age, sex, and severity of the disease of the patient, etc. Based on the amount of siRNA contained therein, the administered dose of the RNAi reagent and the composition of the present disclosure is about 1-300 mg/kg body weight.

The administration frequency may be once or more times every day, every week, every two weeks, every three weeks, every month, every 2 months, every 3 months, every 4 months, every 5 months, every 6 months, every 7 months, every 8 months, every 9 months, every 10 months, every 11 months, or every year.

The administration cycle is once or more times every day, every week, every two weeks, every three weeks, every month, every 2 months, every 3 months, every 4 months, every 5 months, every 6 months, every 7 months, every 8 months, every 9 months, every 10 months, every 11 months, or every year. The total number of administrations of the RNAi reagent or composition may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50. For example, the RNAi reagent or composition may be administered about 1, 2, 3, or 4 times.

In some embodiments, after being introduced into a cell expressing the transthyretin (TTR) gene, the RNAi reagent and the composition disclosed in the present disclosure can inhibit the expression of transthyretin (TTR) mRNA. In some embodiments, the cell is a liver cancer cell or a primary hepatocyte. In some embodiments, the cell is a human liver cancer cell or a primary hepatocyte. In some embodiments, the human liver cancer cell is HepG2. In some embodiments, the human liver cancer cell is Hep3B. In some embodiments, the primary human hepatocyte is PHH. In some embodiments, the primary cynomogus hepatocyte is PCH.

Various drug delivery systems are known and can be used for the RNAi reagent and the composition of the present disclosure, such as encapsulation in liposomes, microparticles, microcapsules, recombinant cells capable of expressing the compound, receptor-mediated cellular endocytosis, and construction of the nucleic acid as part of a retroviral or another vector. In some embodiments, the receptor-mediated cellular endocytosis is cellular endocytosis mediated by the binding of GalNAc to the asialoglycoprotein receptor (ASGPR) on the cell surface. In some embodiments, the drug delivery system is cellular endocytosis mediated by the binding of GalNAc₃ to the cell surface receptor ASGPR.

In some embodiments, the RNAi reagent and the composition of the present disclosure can be packaged in a kit. The RNAi reagent and the pharmaceutically acceptable diluent, carrier, or excipient in the kit are provided in a liquid form or a dry form. In some embodiments, the kit comprises instructions to explain how to mix the RNAi reagent with the pharmaceutically acceptable diluent, carrier, or excipient, or another ingredient.

In some embodiments, on day 7 after administration, BPR-30213022, BPR-30221223, BPR-30221686, BPR-30221617, BPR-30221618, BRP-30222201, and BPR-30222218 can all knock down the serum TTR protein content in mice, with a maximum knockdown rate of 96%. On day 42 after administration, the maximum knockdown rate of the serum TTR protein in mice is 90%. Details are shown in FIG. 1.

In some embodiments, the activity of GalNAc-siRNAs is evaluated in cynomolgus monkeys. BPR-30221223, BPR-30221228, BPR-30221686, BPR-30221617, BPR-30221618, BPR-30221620, BRP-30222201, and BPR-3022218 of the present disclosure can all well knock down the serum TTR protein content in cynomolgus monkeys after administration.

The present disclosure confirms that the RNAi reagent and the composition disclosed in the present disclosure both reduce the production of transthyretin by inhibiting the expression level of transthyretin messenger RNA (mRNA), thereby ultimately reducing or eliminating the deposition of the pathogenic transthyretin amyloid *in vivo*.

Description of the abbreviated names of the materials used in the present disclosure:

| English names or abbreviations of materials or compounds used | Names of materials or compounds used |
|---|---|
| TBDPSCl | tert-Butyldiphenylchlorosilane |
| Imidazole | Imidazole |
| DCM | Dichloromethane |
| BnBr | Benzyl bromide |
| NaH | Sodium hydride |
| DMF | N,N-Dimethylformamide |
| TBAF | Tetrabutylammonium fluoride |
| Bz | Benzoyl |
| iBu | Isobutyryl |
| THF | Tetrahydrofuran |
| C₁₂H₂₅Br | Bromododecane |
| AcOH | Acetic acid |
| Ac₂O | Acetic anhydride |
| H₂SO₄ | Sulfuric acid |
| Uracil | Uracil |
| BSA | N,O-Bistrimethylsilylacetamide |
| TMSOTf | Trimethylsilyl trifluoromethanesulfonate |
| ACN | Acetonitrile |
| BCl₃ | Boron trichloride |
| DMTrCl | 4,4'-Dimethoxytrityl chloride |
| AgNO₃ | Silver nitrate |
| 2,4,6-collidine | 2,4,6-Trimethylpyridine |
| DCE | 1,2-Dichloroethane |
| CEP-Cl | 2-Cyanoethyl-N,N-diisopropylchlorophosphoramidite |
| DIPEA | N,N-Diisopropylethylamine |
| DMAP | 4-Dimethylaminopyridine |
| PE | Petroleum ether |
| EA | Ethyl acetate |
| TBSCl | tert-Butyldimethylsilyl chloride |
| TPSCl | 2,4,6-Triisopropylbenzenesulfonyl chloride |
| TEA | Triethylamine |
| BzCl | Benzoyl chloride |
| pyridine | Pyridine |
| TEA-3HF | Triethylamine trihydrofluoride |
| ABz | N6-Benzoyladenine |
| CH₃ONa | Sodium methoxide |
| GiBu | N2-Isobutyrylguanine |
| CH₃I | Iodomethane |
| C₁₄H₂₉Br | Bromotetradecane |
| C₁₆H₃₃Br | Bromohexadecane |
| DMP | Dess-Martin periodinane |
| PPh₃MeBr | Triphenylphosphine hydrobromide |
| t-BuOK | Potassium tert-butoxide |
| 9-BBN | 9-Borabicyclo[3.3.1]nonane |
| hexane | n-Hexane |
| NaBO₃-4H₂O | Sodium perborate tetrahydrate |
| KF | Potassium fluoride |
| TEMPO | (2,2,6,6-Tetramethylpiperidin-1-yl)oxyl |
| DIAB | (Diacetoxyiodo)benzene |
| EDCI | 1-Ethyl-(3-dimethylaminopropyl)carbodiimide hydrochloride |
| CH₃MgBr | Methylmagnesium bromide |
| m-CPBA | m-Chloroperoxybenzoic acid |
| NaHCO₃ | Sodium bicarbonate |
| PDC | Pyridinium dichromate |
| MsCl | Methanesulfonyl chloride |
| Pd/C | Palladium on carbon |
| HATU | O-(7-Azabenzotriazol-1-yl)-N,N,N',N'-tetramethyluronium hexafluorophosphate |
| TsOH | p-Toluenesulfonic acid |
| H₂O₂ | Hydrogen peroxide |
| LAH | Lithium aluminum hydride |
| K₂OsO₄ | Potassium osmate |
| NMO | N-Methylmorpholine oxide |
| dioxane | 1,4-Dioxane |
| KNO₂ | Potassium nitrite |
| 18-crown-6 | 18-Crown-6 |
| RuO₂ | Ruthenium dioxide |
| NaIO₄ | Sodium periodate |
| LiBEt₃H | Lithium triethylborohydride |
| NaBH₄ | Sodium borohydride |
| DCA | Dichloroacetic acid |
| PMBCl | 4-Methoxybenzyl chloride |
| DBU | 1,8-Diazabicyclo[5.4.0]undec-7-ene |
| AcSK | Potassium thioacetate |
| DDQ | 2,3-Dichloro-5,6-dicyano-1,4-benzoquinone |
| PPh₃ | Triphenylphosphine |
| Pb₂CO₃ | Lead carbonate |
| I₂ | Iodine |
| PADS | Diphenylacetyl disulfide |
| ETT | 5-(Ethylthio)-1H-tetrazole |
| TEAA | Triethylammonium acetate |
| M | mol/L |
| nM | nmol/L |

Unless otherwise stated, for the following *in vivo*/*in vitro* experimental data analysis, GraphPad Prism statistical analysis software is used, the independent sample T-test is used for comparison between two groups of samples, and the one-way ANOVA test is used for multi-group comparison, with Dunnett as the post-hoc test.

### EXAMPLES

### Example 1: Preparation of hTTR siRNAs

### 1. Human transthyretin (hTTR) mRNA sequence

NM_000371.4 (Gene ID: 7276) as shown in SEQ ID NO: 884 from the NCBI RefSeq database.

ACAGAAGUCCACUCAUUCUUGGCAGGAUGGCUUCUCAUCGUCUGCUCCUCCUCUGCC
UUGCUGGACUGGUAUUUGUGUCUGAGGCUGGCCCUACGGGCACCGGUGAAUCCAAGUGUC
CUCUGAUGGUCAAAGUUCUAGAUGCUGUCCGAGGCAGUCCUGCCAUCAAUGUGGCCGUGC
AUGUGUUCAGAAAGGCUGCUGAUGACACCUGGGAGCCAUUUGCCUCUGGGAAAACCAGUG
AGUCUGGAGAGCUGCAUGGGCUCACAACUGAGGAGGAAUUUGUAGAAGGGAUAUACAAA

GUGGAAAUAGACACCAAAUCUUACUGGAAGGCACUUGGCAUCUCCCCAUUCCAUGAGCAU
GCAGAGGUGGUAUUCACAGCCAACGACUCCGGCCCCCGCCGCUACACCAUUGCCGCCCUGC
UGAGCCCCUACUCCUAUUCCACCACGGCUGUCGUCACCAAUCCCAAGGAAUGAGGGACUU
CUCCUCCAGUGGACCUGAAGGACGAGGGAUGGGAUUUCAUGUAACCAAGAGUAUUCCAUU
UUUACUAAAGCAGUGUUUUCACCUCAUAUGCUAUGUUAGAAGUCCAGGCAGAGACAAUAA
AACAUUCCUGUGAAAGGCA (SEQ ID NO: 884)

235 pairs of hTTR siRNAs were designed according to the hTTR mRNA. See Table 2 for details.

**Table 2. hTTR siRNAs**

| SEQ ID NO | siRNA No. | Sequence (5'-3') | |
|---|---|---|---|
| 1 | BPR-302001 | SS | AUUCUUGGCAGGAUGGCUUCU |
| 2 | | AS | AGAAGCCAUCCUGCCAAGAAUGA |
| 3 | BPR-302002 | SS | UUCUUGGCAGGAUGGCUUCUA |
| 4 | | AS | UAGAAGCCAUCCUGCCAAGAAUG |
| 5 | BPR-302003 | SS | UCUUGGCAGGAUGGCUUCUCA |
| 6 | | AS | UGAGAAGCCAUCCUGCCAAGAAU |
| 7 | BPR-302004 | SS | CUUGGCAGGAUGGCUUCUCAU |
| 8 | | AS | AUGAGAAGCCAUCCUGCCAAGAA |
| 9 | BPR-302005 | SS | UUGGCAGGAUGGCUUCUCAUA |
| 10 | | AS | UAUGAGAAGCCAUCCUGCCAAGA |
| 11 | BPR-302006 | SS | UGGCAGGAUGGCUUCUCAUCA |
| 12 | | AS | UGAUGAGAAGCCAUCCUGCCAAG |
| 13 | BPR-302007 | SS | GGCAGGAUGGCUUCUCAUCGU |
| 14 | | AS | ACGAUGAGAAGCCAUCCUGCCAA |
| 15 | BPR-302008 | SS | GCAGGAUGGCUUCUCAUCGUA |
| 16 | | AS | UACGAUGAGAAGCCAUCCUGCCA |
| 17 | BPR-302009 | SS | CAGGAUGGCUUCUCAUCGUCU |
| 18 | | AS | AGACGAUGAGAAGCCAUCCUGCC |
| 19 | BPR-302010 | SS | AGGAUGGCUUCUCAUCGUCUA |
| 20 | | AS | UAGACGAUGAGAAGCCAUCCUGC |
| 21 | BPR-302011 | SS | GGAUGGCUUCUCAUCGUCUGA |
| 22 | | AS | UCAGACGAUGAGAAGCCAUCCUG |
| 23 | BPR-302012 | SS | AUGGCUUCUCAUCGUCUGCUA |
| 24 | | AS | UAGCAGACGAUGAGAAGCCAUCC |
| 25 | BPR-302013 | SS | UGGCUUCUCAUCGUCUGCUCA |
| 26 | | AS | UGAGCAGACGAUGAGAAGCCAUC |
| 27 | BPR-302014 | SS | GCUUCUCAUCGUCUGCUCCUA |
| 28 | | AS | UAGGAGCAGACGAUGAGAAGCCA |
| 29 | BPR-302015 | SS | UCUCAUCGUCUGCUCCUCCUA |
| 30 | | AS | UAGGAGGAGCAGACGAUGAGAAG |
| 31 | BPR-302016 | SS | UCAUCGUCUGCUCCUCCUCUA |
| 32 | | AS | UAGAGGAGGAGCAGACGAUGAGA |
| 33 | BPR-302017 | SS | CAUCGUCUGCUCCUCCUCUGA |
| 34 | | AS | UCAGAGGAGGAGCAGACGAUGAG |
| 35 | BPR-302018 | SS | CGUCUGCUCCUCCUCUGCCUU |
| 36 | | AS | AAGGCAGAGGAGGAGCAGACGAU |
| 37 | BPR-302019 | SS | UGCUGGACUGGUAUUUGUGUA |
| 38 | | AS | UACACAAAUACCAGUCCAGCAAG |
| 39 | BPR-302020 | SS | GCUGGACUGGUAUUUGUGUCU |
| 40 | | AS | AGACACAAAUACCAGUCCAGCAA |
| 41 | BPR-302021 | SS | CUGGACUGGUAUUUGUGUCUA |
| 42 | | AS | UAGACACAAAUACCAGUCCAGCA |
| 43 | BPR-302022 | SS | UGGACUGGUAUUUGUGUCUGA |
| 44 | | AS | UCAGACACAAAUACCAGUCCAGC |
| 45 | BPR-302023 | SS | GGACUGGUAUUUGUGUCUGAA |
| 46 | | AS | UUCAGACACAAAUACCAGUCCAG |
| 47 | BPR-302024 | SS | GACUGGUAUUUGUGUCUGAGA |
| 48 | | AS | UCUCAGACACAAAUACCAGUCCA |
| 49 | BPR-302025 | SS | ACUGGUAUUUGUGUCUGAGGA |
| 50 | | AS | UCCUCAGACACAAAUACCAGUCC |
| 51 | BPR-302026 | SS | UGGUAUUUGUGUCUGAGGCUA |
| 52 | | AS | UAGCCUCAGACACAAAUACCAGU |
| 53 | BPR-302027 | SS | GUAUUUGUGUCUGAGGCUGGA |
| 54 | | AS | UCCAGCCUCAGACACAAAUACCA |
| 55 | BPR-302028 | SS | GUGAAUCCAAGUGUCCUCUGA |
| 56 | | AS | UCAGAGGACACUUGGAUUCACCG |
| 57 | BPR-302029 | SS | UGAAUCCAAGUGUCCUCUGAU |
| 58 | | AS | AUCAGAGGACACUUGGAUUCACC |
| 59 | BPR-302030 | SS | GAAUCCAAGUGUCCUCUGAUA |
| 60 | | AS | UAUCAGAGGACACUUGGAUUCAC |
| 61 | BPR-302031 | SS | AAUCCAAGUGUCCUCUGAUGA |
| 62 | | AS | UCAUCAGAGGACACUUGGAUUCA |
| 63 | BPR-302032 | SS | AUCCAAGUGUCCUCUGAUGGU |
| 64 | | AS | ACCAUCAGAGGACACUUGGAUUC |
| 65 | BPR-302033 | SS | UCCAAGUGUCCUCUGAUGGUA |
| 66 | | AS | UACCAUCAGAGGACACUUGGAUU |
| 67 | BPR-302034 | SS | CCAAGUGUCCUCUGAUGGUCA |
| 68 | | AS | UGACCAUCAGAGGACACUUGGAU |
| 69 | BPR-302035 | SS | CAAGUGUCCUCUGAUGGUCAA |
| 70 | | AS | UUGACCAUCAGAGGACACUUGGA |
| 71 | BPR-302036 | SS | AAGUGUCCUCUGAUGGUCAAA |
| 72 | | AS | UUUGACCAUCAGAGGACACUUGG |
| 73 | BPR-302037 | SS | AGUGUCCUCUGAUGGUCAAAA |
| 74 | | AS | UUUUGACCAUCAGAGGACACUUG |
| 75 | BPR-302038 | SS | GUGUCCUCUGAUGGUCAAAGU |
| 76 | | AS | ACUUUGACCAUCAGAGGACACUU |
| 77 | BPR-302039 | SS | UGUCCUCUGAUGGUCAAAGUU |
| 78 | | AS | AACUUUGACCAUCAGAGGACACU |
| 79 | BPR-302040 | SS | GUCCUCUGAUGGUCAAAGUUA |
| 80 | | AS | UAACUUUGACCAUCAGAGGACAC |
| 81 | BPR-302041 | SS | UCCUCUGAUGGUCAAAGUUCU |
| 82 | | AS | AGAACUUUGACCAUCAGAGGACA |
| 83 | BPR-302042 | SS | CCUCUGAUGGUCAAAGUUCUA |
| 84 | | AS | UAGAACUUUGACCAUCAGAGGAC |
| 85 | BPR-302043 | SS | CUCUGAUGGUCAAAGUUCUAA |
| 86 | | AS | UUAGAACUUUGACCAUCAGAGGA |
| 87 | BPR-302044 | SS | UCUGAUGGUCAAAGUUCUAGA |
| 88 | | AS | UCUAGAACUUUGACCAUCAGAGG |
| 89 | BPR-302045 | SS | CUGAUGGUCAAAGUUCUAGAU |
| 90 | | AS | AUCUAGAACUUUGACCAUCAGAG |
| 91 | BPR-302046 | SS | UGAUGGUCAAAGUUCUAGAUA |
| 92 | | AS | UAUCUAGAACUUUGACCAUCAGA |
| 93 | BPR-302047 | SS | GAUGGUCAAAGUUCUAGAUGA |
| 94 | | AS | UCAUCUAGAACUUUGACCAUCAG |
| 95 | BPR-302048 | SS | AUGGUCAAAGUUCUAGAUGCU |
| 96 | | AS | AGCAUCUAGAACUUUGACCAUCA |
| 97 | BPR-302049 | SS | AUCAAUGUGGCCGUGCAUGUA |
| 98 | | AS | UACAUGCACGGCCACAUUGAUGG |
| 99 | BPR-302050 | SS | AGCCAUUUGCCUCUGGGAAAA |
| 100 | | AS | UUUUCCCAGAGGCAAAUGGCUCC |
| 101 | BPR-302051 | SS | GCCAUUUGCCUCUGGGAAAAA |
| 102 | | AS | UUUUUCCCAGAGGCAAAUGGCUC |
| 103 | BPR-302052 | SS | CCAUUUGCCUCUGGGAAAACA |
| 104 | | AS | UGUUUUCCCAGAGGCAAAUGGCU |
| 105 | BPR-302053 | SS | CAUUUGCCUCUGGGAAAACCA |
| 106 | | AS | UGGUUUUCCCAGAGGCAAAUGGC |
| 107 | BPR-302054 | SS | AUUUGCCUCUGGGAAAACCAA |
| 108 | | AS | UUGGUUUUCCCAGAGGCAAAUGG |
| 109 | BPR-302055 | SS | UUUGCCUCUGGGAAAACCAGU |
| 110 | | AS | ACUGGUUUUCCCAGAGGCAAAUG |
| 111 | BPR-302056 | SS | UUGCCUCUGGGAAAACCAGUA |
| 112 | | AS | UACUGGUUUUCCCAGAGGCAAAU |
| 113 | BPR-302057 | SS | UGCCUCUGGGAAAACCAGUGA |
| 114 | | AS | UCACUGGUUUUCCCAGAGGCAAA |
| 115 | BPR-302058 | SS | GCCUCUGGGAAAACCAGUGAA |
| 116 | | AS | UUCACUGGUUUUCCCAGAGGCAA |
| 117 | BPR-302059 | SS | CCUCUGGGAAAACCAGUGAGU |
| 118 | | AS | ACUCACUGGUUUUCCCAGAGGCA |
| 119 | BPR-302060 | SS | CUCUGGGAAAACCAGUGAGUA |
| 120 | | AS | UACUCACUGGUUUUCCCAGAGGC |
| 121 | BPR-302061 | SS | UCUGGGAAAACCAGUGAGUCU |
| 122 | | AS | AGACUCACUGGUUUUCCCAGAGG |
| 123 | BPR-302062 | SS | CUGGGAAAACCAGUGAGUCUA |
| 124 | | AS | UAGACUCACUGGUUUUCCCAGAG |
| 125 | BPR-302063 | SS | UGGGAAAACCAGUGAGUCUGA |
| 126 | | AS | UCAGACUCACUGGUUUUCCCAGA |
| 127 | BPR-302064 | SS | GGGAAAACCAGUGAGUCUGGA |
| 128 | | AS | UCCAGACUCACUGGUUUUCCCAG |
| 129 | BPR-302065 | SS | GGAAAACCAGUGAGUCUGGAA |
| 130 | | AS | UUCCAGACUCACUGGUUUUCCCA |
| 131 | BPR-302066 | SS | GAAAACCAGUGAGUCUGGAGA |
| 132 | | AS | UCUCCAGACUCACUGGUUUUCCC |
| 133 | BPR-302067 | SS | AAAACCAGUGAGUCUGGAGAA |
| 134 | | AS | UUCUCCAGACUCACUGGUUUUCC |
| 135 | BPR-302068 | SS | AAACCAGUGAGUCUGGAGAGA |
| 136 | | AS | UCUCUCCAGACUCACUGGUUUUC |
| 137 | BPR-302069 | SS | ACCAGUGAGUCUGGAGAGCUA |
| 138 | | AS | UAGCUCUCCAGACUCACUGGUUU |
| 139 | BPR-302070 | SS | CCAGUGAGUCUGGAGAGCUGA |
| 140 | | AS | UCAGCUCUCCAGACUCACUGGUU |
| 141 | BPR-302071 | SS | AGUGAGUCUGGAGAGCUGCAU |
| 142 | | AS | AUGCAGCUCUCCAGACUCACUGG |
| 143 | BPR-302072 | SS | GUGAGUCUGGAGAGCUGCAUA |
| 144 | | AS | UAUGCAGCUCUCCAGACUCACUG |
| 145 | BPR-302073 | SS | UGAGUCUGGAGAGCUGCAUGA |
| 146 | | AS | UCAUGCAGCUCUCCAGACUCACU |
| 147 | BPR-302074 | SS | GAGUCUGGAGAGCUGCAUGGA |
| 148 | | AS | UCCAUGCAGCUCUCCAGACUCAC |
| 149 | BPR-302075 | SS | UCUGGAGAGCUGCAUGGGCUA |
| 150 | | AS | UAGCCCAUGCAGCUCUCCAGACU |
| 151 | BPR-302076 | SS | GAGAGCUGCAUGGGCUCACAA |
| 152 | | AS | UUGUGAGCCCAUGCAGCUCUCCA |
| 153 | BPR-302077 | SS | AGAGCUGCAUGGGCUCACAAA |
| 154 | | AS | UUUGUGAGCCCAUGCAGCUCUCC |
| 155 | BPR-302078 | SS | GAGCUGCAUGGGCUCACAACU |
| 156 | | AS | AGUUGUGAGCCCAUGCAGCUCUC |
| 157 | BPR-302079 | SS | AGCUGCAUGGGCUCACAACUA |
| 158 | | AS | UAGUUGUGAGCCCAUGCAGCUCU |
| 159 | BPR-302080 | SS | GCUGCAUGGGCUCACAACUGA |
| 160 | | AS | UCAGUUGUGAGCCCAUGCAGCUC |
| 161 | BPR-302081 | SS | CUGCAUGGGCUCACAACUGAA |
| 162 | | AS | UUCAGUUGUGAGCCCAUGCAGCU |
| 163 | BPR-302082 | SS | UGCAUGGGCUCACAACUGAGA |
| 164 | | AS | UCUCAGUUGUGAGCCCAUGCAGC |
| 165 | BPR-302083 | SS | GCAUGGGCUCACAACUGAGGA |
| 166 | | AS | UCCUCAGUUGUGAGCCCAUGCAG |
| 167 | BPR-302084 | SS | CAUGGGCUCACAACUGAGGAA |
| 168 | | AS | UUCCUCAGUUGUGAGCCCAUGCA |
| 169 | BPR-302085 | SS | AUGGGCUCACAACUGAGGAGA |
| 170 | | AS | UCUCCUCAGUUGUGAGCCCAUGC |
| 171 | BPR-302086 | SS | GGGCUCACAACUGAGGAGGAA |
| 172 | | AS | UUCCUCCUCAGUUGUGAGCCCAU |
| 173 | BPR-302087 | SS | GGCUCACAACUGAGGAGGAAU |
| 174 | | AS | AUUCCUCCUCAGUUGUGAGCCCA |
| 175 | BPR-302088 | SS | GCUCACAACUGAGGAGGAAUU |
| 176 | | AS | AAUUCCUCCUCAGUUGUGAGCCC |
| 177 | BPR-302089 | SS | CUCACAACUGAGGAGGAAUUU |
| 178 | | AS | AAAUUCCUCCUCAGUUGUGAGCC |
| 179 | BPR-302090 | SS | UCACAACUGAGGAGGAAUUUA |
| 180 | | AS | UAAAUUCCUCCUCAGUUGUGAGC |
| 181 | BPR-302091 | SS | CACAACUGAGGAGGAAUUUGU |
| 182 | | AS | ACAAAUUCCUCCUCAGUUGUGAG |
| 183 | BPR-302092 | SS | ACAACUGAGGAGGAAUUUGUA |
| 184 | | AS | UACAAAUUCCUCCUCAGUUGUGA |
| 185 | BPR-302093 | SS | CAACUGAGGAGGAAUUUGUAA |
| 186 | | AS | UUACAAAUUCCUCCUCAGUUGUG |
| 187 | BPR-302094 | SS | AACUGAGGAGGAAUUUGUAGA |
| 188 | | AS | UCUACAAAUUCCUCCUCAGUUGU |
| 189 | BPR-302095 | SS | ACUGAGGAGGAAUUUGUAGAA |
| 190 | | AS | UUCUACAAAUUCCUCCUCAGUUG |
| 191 | BPR-302096 | SS | CUGAGGAGGAAUUUGUAGAAA |
| 192 | | AS | UUUCUACAAAUUCCUCCUCAGUU |
| 193 | BPR-302097 | SS | UGAGGAGGAAUUUGUAGAAGA |
| 194 | | AS | UCUUCUACAAAUUCCUCCUCAGU |
| 195 | BPR-302098 | SS | GAGGAGGAAUUUGUAGAAGGA |
| 196 | | AS | UCCUUCUACAAAUUCCUCCUCAG |
| 197 | BPR-302099 | SS | AGGAGGAAUUUGUAGAAGGGA |
| 198 | | AS | UCCCUUCUACAAAUUCCUCCUCA |
| 199 | BPR-302100 | SS | GGAGGAAUUUGUAGAAGGGAU |
| 200 | | AS | AUCCCUUCUACAAAUUCCUCCUC |
| 201 | BPR-302101 | SS | GAGGAAUUUGUAGAAGGGAUA |
| 202 | | AS | UAUCCCUUCUACAAAUUCCUCCU |
| 203 | BPR-302102 | SS | AGGAAUUUGUAGAAGGGAUAU |
| 204 | | AS | AUAUCCCUUCUACAAAUUCCUCC |
| 205 | BPR-302103 | SS | GGAAUUUGUAGAAGGGAUAUA |
| 206 | | AS | UAUAUCCCUUCUACAAAUUCCUC |
| 207 | BPR-302104 | SS | GAAUUUGUAGAAGGGAUAUAA |
| 208 | | AS | UUAUAUCCCUUCUACAAAUUCCU |
| 209 | BPR-302105 | SS | AAUUUGUAGAAGGGAUAUACA |
| 210 | | AS | UGUAUAUCCCUUCUACAAAUUCC |
| 211 | BPR-302106 | SS | AUUUGUAGAAGGGAUAUACAA |
| 212 | | AS | UUGUAUAUCCCUUCUACAAAUUC |
| 213 | BPR-302107 | SS | UUUGUAGAAGGGAUAUACAAA |
| 214 | | AS | UUUGUAUAUCCCUUCUACAAAUU |
| 215 | BPR-302108 | SS | UUGUAGAAGGGAUAUACAAAA |
| 216 | | AS | UUUUGUAUAUCCCUUCUACAAAU |
| 217 | BPR-302109 | SS | UGUAGAAGGGAUAUACAAAGU |
| 218 | | AS | ACUUUGUAUAUCCCUUCUACAAA |
| 219 | BPR-302110 | SS | GUAGAAGGGAUAUACAAAGUA |
| 220 | | AS | UACUUUGUAUAUCCCUUCUACAA |
| 221 | BPR-302111 | SS | UAGAAGGGAUAUACAAAGUGA |
| 222 | | AS | UCACUUUGUAUAUCCCUUCUACA |
| 223 | BPR-302112 | SS | AGAAGGGAUAUACAAAGUGGA |
| 224 | | AS | UCCACUUUGUAUAUCCCUUCUAC |
| 225 | BPR-302113 | SS | GAAGGGAUAUACAAAGUGGAA |
| 226 | | AS | UUCCACUUUGUAUAUCCCUUCUA |
| 227 | BPR-302114 | SS | AAGGGAUAUACAAAGUGGAAA |
| 228 | | AS | UUUCCACUUUGUAUAUCCCUUCU |
| 229 | BPR-302115 | SS | AGGGAUAUACAAAGUGGAAAU |
| 230 | | AS | AUUUCCACUUUGUAUAUCCCUUC |
| 231 | BPR-302116 | SS | GGGAUAUACAAAGUGGAAAUA |
| 232 | | AS | UAUUUCCACUUUGUAUAUCCCUU |
| 233 | BPR-302117 | SS | GGAUAUACAAAGUGGAAAUAA |
| 234 | | AS | UUAUUUCCACUUUGUAUAUCCCU |
| 235 | BPR-302118 | SS | GAUAUACAAAGUGGAAAUAGA |
| 236 | | AS | UCUAUUUCCACUUUGUAUAUCCC |
| 237 | BPR-302119 | SS | AUAUACAAAGUGGAAAUAGAA |
| 238 | | AS | UUCUAUUUCCACUUUGUAUAUCC |
| 239 | BPR-302120 | SS | UAUACAAAGUGGAAAUAGACA |
| 240 | | AS | UGUCUAUUUCCACUUUGUAUAUC |
| 241 | BPR-302121 | SS | AUACAAAGUGGAAAUAGACAA |
| 242 | | AS | UUGUCUAUUUCCACUUUGUAUAU |
| 243 | BPR-302122 | SS | UACAAAGUGGAAAUAGACACA |
| 244 | | AS | UGUGUCUAUUUCCACUUUGUAUA |
| 245 | BPR-302123 | SS | ACAAAGUGGAAAUAGACACCA |
| 246 | | AS | UGGUGUCUAUUUCCACUUUGUAU |
| 247 | BPR-302124 | SS | CAAAGUGGAAAUAGACACCAA |
| 248 | | AS | UUGGUGUCUAUUUCCACUUUGUA |
| 249 | BPR-302125 | SS | AAAGUGGAAAUAGACACCAAA |
| 250 | | AS | UUUGGUGUCUAUUUCCACUUUGU |
| 251 | BPR-302126 | SS | AAGUGGAAAUAGACACCAAAU |
| 252 | | AS | AUUUGGUGUCUAUUUCCACUUUG |
| 253 | BPR-302127 | SS | AGUGGAAAUAGACACCAAAUA |
| 254 | | AS | UAUUUGGUGUCUAUUUCCACUUU |
| 255 | BPR-302128 | SS | GUGGAAAUAGACACCAAAUCU |
| 256 | | AS | AGAUUUGGUGUCUAUUUCCACUU |
| 257 | BPR-302129 | SS | UGGAAAUAGACACCAAAUCUU |
| 258 | | AS | AAGAUUUGGUGUCUAUUUCCACU |
| 259 | BPR-302130 | SS | GGAAAUAGACACCAAAUCUUA |
| 260 | | AS | UAAGAUUUGGUGUCUAUUUCCAC |
| 261 | BPR-302131 | SS | GAAAUAGACACCAAAUCUUAA |
| 262 | | AS | UUAAGAUUUGGUGUCUAUUUCCA |
| 263 | BPR-302132 | SS | AAAUAGACACCAAAUCUUACU |
| 264 | | AS | AGUAAGAUUUGGUGUCUAUUUCC |
| 265 | BPR-302133 | SS | AAUAGACACCAAAUCUUACUA |
| 266 | | AS | UAGUAAGAUUUGGUGUCUAUUUC |
| 267 | BPR-302134 | SS | AUAGACACCAAAUCUUACUGA |
| 268 | | AS | UCAGUAAGAUUUGGUGUCUAUUU |
| 269 | BPR-302135 | SS | UAGACACCAAAUCUUACUGGA |
| 270 | | AS | UCCAGUAAGAUUUGGUGUCUAUU |
| 271 | BPR-302136 | SS | AGACACCAAAUCUUACUGGAA |
| 272 | | AS | UUCCAGUAAGAUUUGGUGUCUAU |
| 273 | BPR-302137 | SS | GACACCAAAUCUUACUGGAAA |
| 274 | | AS | UUUCCAGUAAGAUUUGGUGUCUA |
| 275 | BPR-302138 | SS | GCACUUGGCAUCUCCCCAUUA |
| 276 | | AS | UAAUGGGGAGAUGCCAAGUGCCU |
| 277 | BPR-302139 | SS | CACUUGGCAUCUCCCCAUUCA |
| 278 | | AS | UGAAUGGGGAGAUGCCAAGUGCC |
| 279 | BPR-302140 | SS | ACUUGGCAUCUCCCCAUUCCA |
| 280 | | AS | UGGAAUGGGGAGAUGCCAAGUGC |
| 281 | BPR-302141 | SS | CUUGGCAUCUCCCCAUUCCAU |
| 282 | | AS | AUGGAAUGGGGAGAUGCCAAGUG |
| 283 | BPR-302142 | SS | UUGGCAUCUCCCCAUUCCAUA |
| 284 | | AS | UAUGGAAUGGGGAGAUGCCAAGU |
| 285 | BPR-302143 | SS | UGGCAUCUCCCCAUUCCAUGA |
| 286 | | AS | UCAUGGAAUGGGGAGAUGCCAAG |
| 287 | BPR-302144 | SS | GGCAUCUCCCCAUUCCAUGAA |
| 288 | | AS | UUCAUGGAAUGGGGAGAUGCCAA |
| 289 | BPR-302145 | SS | GCAUCUCCCCAUUCCAUGAGA |
| 290 | | AS | UCUCAUGGAAUGGGGAGAUGCCA |
| 291 | BPR-302146 | SS | CAUCUCCCCAUUCCAUGAGCA |
| 292 | | AS | UGCUCAUGGAAUGGGGAGAUGCC |
| 293 | BPR-302147 | SS | AUCUCCCCAUUCCAUGAGCAU |
| 294 | | AS | AUGCUCAUGGAAUGGGGAGAUGC |
| 295 | BPR-302148 | SS | UCUCCCCAUUCCAUGAGCAUA |
| 296 | | AS | UAUGCUCAUGGAAUGGGGAGAUG |
| 297 | BPR-302149 | SS | CUCCCCAUUCCAUGAGCAUGA |
| 298 | | AS | UCAUGCUCAUGGAAUGGGGAGAU |
| 299 | BPR-302150 | SS | UCCCCAUUCCAUGAGCAUGCA |
| 300 | | AS | UGCAUGCUCAUGGAAUGGGGAGA |
| 301 | BPR-302151 | SS | CCCCAUUCCAUGAGCAUGCAA |
| 302 | | AS | UUGCAUGCUCAUGGAAUGGGGAG |
| 303 | BPR-302152 | SS | CCCAUUCCAUGAGCAUGCAGA |
| 304 | | AS | UCUGCAUGCUCAUGGAAUGGGGA |
| 305 | BPR-302153 | SS | CCAUUCCAUGAGCAUGCAGAA |
| 306 | | AS | UUCUGCAUGCUCAUGGAAUGGGG |
| 307 | BPR-302154 | SS | CAUUCCAUGAGCAUGCAGAGA |
| 308 | | AS | UCUCUGCAUGCUCAUGGAAUGGG |
| 309 | BPR-302155 | SS | UUCCAUGAGCAUGCAGAGGUA |
| 310 | | AS | UACCUCUGCAUGCUCAUGGAAUG |
| 311 | BPR-302156 | SS | UCCAUGAGCAUGCAGAGGUGA |
| 312 | | AS | UCACCUCUGCAUGCUCAUGGAAU |
| 313 | BPR-302157 | SS | CAUGAGCAUGCAGAGGUGGUA |
| 314 | | AS | UACCACCUCUGCAUGCUCAUGGA |
| 315 | BPR-302158 | SS | AUGAGCAUGCAGAGGUGGUAU |
| 316 | | AS | AUACCACCUCUGCAUGCUCAUGG |
| 317 | BPR-302159 | SS | UGAGCAUGCAGAGGUGGUAUU |
| 318 | | AS | AAUACCACCUCUGCAUGCUCAUG |
| 319 | BPR-302160 | SS | GAGCAUGCAGAGGUGGUAUUA |
| 320 | | AS | UAAUACCACCUCUGCAUGCUCAU |
| 321 | BPR-302161 | SS | AGCAUGCAGAGGUGGUAUUCA |
| 322 | | AS | UGAAUACCACCUCUGCAUGCUCA |
| 323 | BPR-302162 | SS | GCAUGCAGAGGUGGUAUUCAA |
| 324 | | AS | UUGAAUACCACCUCUGCAUGCUC |
| 325 | BPR-302163 | SS | CAUGCAGAGGUGGUAUUCACA |
| 326 | | AS | UGUGAAUACCACCUCUGCAUGCU |
| 327 | BPR-302164 | SS | AUGCAGAGGUGGUAUUCACAA |
| 328 | | AS | UUGUGAAUACCACCUCUGCAUGC |
| 329 | BPR-302165 | SS | UGCAGAGGUGGUAUUCACAGA |
| 330 | | AS | UCUGUGAAUACCACCUCUGCAUG |
| 331 | BPR-302166 | SS | GCAGAGGUGGUAUUCACAGCA |
| 332 | | AS | UGCUGUGAAUACCACCUCUGCAU |
| 333 | BPR-302167 | SS | AGAGGUGGUAUUCACAGCCAA |
| 334 | | AS | UUGGCUGUGAAUACCACCUCUGC |
| 335 | BPR-302168 | SS | GAGGUGGUAUUCACAGCCAAA |
| 336 | | AS | UUUGGCUGUGAAUACCACCUCUG |
| 337 | BPR-302169 | SS | AGGUGGUAUUCACAGCCAACA |
| 338 | | AS | UGUUGGCUGUGAAUACCACCUCU |
| 339 | BPR-302170 | SS | CCUGCUGAGCCCCUACUCCUA |
| 340 | | AS | UAGGAGUAGGGGCUCAGCAGGGC |
| 341 | BPR-302171 | SS | CUGCUGAGCCCCUACUCCUAU |
| 342 | | AS | AUAGGAGUAGGGGCUCAGCAGGG |
| 343 | BPR-302172 | SS | UGCUGAGCCCCUACUCCUAUU |
| 344 | | AS | AAUAGGAGUAGGGGCUCAGCAGG |
| 345 | BPR-302173 | SS | GCUGAGCCCCUACUCCUAUUA |
| 346 | | AS | UAAUAGGAGUAGGGGCUCAGCAG |
| 347 | BPR-302174 | SS | CUGAGCCCCUACUCCUAUUCA |
| 348 | | AS | UGAAUAGGAGUAGGGGCUCAGCA |
| 349 | BPR-302175 | SS | UGAGCCCCUACUCCUAUUCCA |
| 350 | | AS | UGGAAUAGGAGUAGGGGCUCAGC |
| 351 | BPR-302176 | SS | GAGCCCCUACUCCUAUUCCAA |
| 352 | | AS | UUGGAAUAGGAGUAGGGGCUCAG |
| 353 | BPR-302177 | SS | AGCCCCUACUCCUAUUCCACA |
| 354 | | AS | UGUGGAAUAGGAGUAGGGGCUCA |
| 355 | BPR-302178 | SS | GCCCCUACUCCUAUUCCACCA |
| 356 | | AS | UGGUGGAAUAGGAGUAGGGGCUC |
| 357 | BPR-302179 | SS | CCCCUACUCCUAUUCCACCAA |
| 358 | | AS | UUGGUGGAAUAGGAGUAGGGGCU |
| 359 | BPR-302180 | SS | UAUUCCACCACGGCUGUCGUA |
| 360 | | AS | UACGACAGCCGUGGUGGAAUAGG |
| 361 | BPR-302181 | SS | AUUCCACCACGGCUGUCGUCA |
| 362 | | AS | UGACGACAGCCGUGGUGGAAUAG |
| 363 | BPR-302182 | SS | UUCCACCACGGCUGUCGUCAA |
| 364 | | AS | UUGACGACAGCCGUGGUGGAAUA |
| 365 | BPR-302183 | SS | CACCACGGCUGUCGUCACCAA |
| 366 | | AS | UUGGUGACGACAGCCGUGGUGGA |
| 367 | BPR-302184 | SS | ACCACGGCUGUCGUCACCAAU |
| 368 | | AS | AUUGGUGACGACAGCCGUGGUGG |
| 369 | BPR-302185 | SS | CCACGGCUGUCGUCACCAAUA |
| 370 | | AS | UAUUGGUGACGACAGCCGUGGUG |
| 371 | BPR-302186 | SS | CACGGCUGUCGUCACCAAUCA |
| 372 | | AS | UGAUUGGUGACGACAGCCGUGGU |
| 373 | BPR-302187 | SS | ACGGCUGUCGUCACCAAUCCA |
| 374 | | AS | UGGAUUGGUGACGACAGCCGUGG |
| 375 | BPR-302188 | SS | CGGCUGUCGUCACCAAUCCCA |
| 376 | | AS | UGGGAUUGGUGACGACAGCCGUG |
| 377 | BPR-302189 | SS | GGCUGUCGUCACCAAUCCCAA |
| 378 | | AS | UUGGGAUUGGUGACGACAGCCGU |
| 379 | BPR-302190 | SS | GCUGUCGUCACCAAUCCCAAA |
| 380 | | AS | UUUGGGAUUGGUGACGACAGCCG |
| 381 | BPR-302191 | SS | CUGUCGUCACCAAUCCCAAGA |
| 382 | | AS | UCUUGGGAUUGGUGACGACAGCC |
| 383 | BPR-302192 | SS | GUCGUCACCAAUCCCAAGGAA |
| 384 | | AS | UUCCUUGGGAUUGGUGACGACAG |
| 385 | BPR-302193 | SS | UCGUCACCAAUCCCAAGGAAU |
| 386 | | AS | AUUCCUUGGGAUUGGUGACGACA |
| 387 | BPR-302194 | SS | CGUCACCAAUCCCAAGGAAUA |
| 388 | | AS | UAUUCCUUGGGAUUGGUGACGAC |
| 389 | BPR-302195 | SS | GUCACCAAUCCCAAGGAAUGA |
| 390 | | AS | UCAUUCCUUGGGAUUGGUGACGA |
| 391 | BPR-302196 | SS | UCACCAAUCCCAAGGAAUGAA |
| 392 | | AS | UUCAUUCCUUGGGAUUGGUGACG |
| 393 | BPR-302197 | SS | CACCAAUCCCAAGGAAUGAGA |
| 394 | | AS | UCUCAUUCCUUGGGAUUGGUGAC |
| 395 | BPR-302198 | SS | ACCAAUCCCAAGGAAUGAGGA |
| 396 | | AS | UCCUCAUUCCUUGGGAUUGGUGA |
| 397 | BPR-302199 | SS | CCAAUCCCAAGGAAUGAGGGA |
| 398 | | AS | UCCCUCAUUCCUUGGGAUUGGUG |
| 399 | BPR-302200 | SS | CAAUCCCAAGGAAUGAGGGAA |
| 400 | | AS | UUCCCUCAUUCCUUGGGAUUGGU |
| 401 | BPR-302201 | SS | AUCCCAAGGAAUGAGGGACUU |
| 402 | | AS | AAGUCCCUCAUUCCUUGGGAUUG |
| 403 | BPR-302202 | SS | UCCCAAGGAAUGAGGGACUUA |
| 404 | | AS | UAAGUCCCUCAUUCCUUGGGAUU |
| 405 | BPR-302203 | SS | CCCAAGGAAUGAGGGACUUCU |
| 406 | | AS | AGAAGUCCCUCAUUCCUUGGGAU |
| 407 | BPR-302204 | SS | CCAAGGAAUGAGGGACUUCUA |
| 408 | | AS | UAGAAGUCCCUCAUUCCUUGGGA |
| 409 | BPR-302205 | SS | CAAGGAAUGAGGGACUUCUCA |
| 410 | | AS | UGAGAAGUCCCUCAUUCCUUGGG |
| 411 | BPR-302206 | SS | AAGGAAUGAGGGACUUCUCCU |
| 412 | | AS | AGGAGAAGUCCCUCAUUCCUUGG |
| 413 | BPR-302207 | SS | UGGGAUUUCAUGUAACCAAGA |
| 414 | | AS | UCUUGGUUACAUGAAAUCCCAUC |
| 415 | BPR-302208 | SS | AUGUAACCAAGAGUAUUCCAU |
| 416 | | AS | AUGGAAUACUCUUGGUUACAUGA |
| 417 | BPR-302209 | SS | UUACUAAAGCAGUGUUUUCAA |
| 418 | | AS | UUGAAAACACUGCUUUAGUAAAA |
| 419 | BPR-302210 | SS | UACUAAAGCAGUGUUUUCACA |
| 420 | | AS | UGUGAAAACACUGCUUUAGUAAA |
| 421 | BPR-302211 | SS | ACUAAAGCAGUGUUUUCACCU |
| 422 | | AS | AGGUGAAAACACUGCUUUAGUAA |
| 423 | BPR-302212 | SS | CUAAAGCAGUGUUUUCACCUA |
| 424 | | AS | UAGGUGAAAACACUGCUUUAGGG |
| 425 | BPR-302213 | SS | UAAAGCAGUGUUUUCACCUCA |
| 426 | | AS | UGAGGUGAAAACACUGCUUUAGU |
| 427 | BPR-302214 | SS | AAAGCAGUGUUUUCACCUCAU |
| 428 | | AS | AUGAGGUGAAAACACUGCUUUAG |
| 429 | BPR-302215 | SS | AAGCAGUGUUUUCACCUCAUA |
| 430 | | AS | UAUGAGGUGAAAACACUGCUUUA |
| 431 | BPR-302216 | SS | AGCAGUGUUUUCACCUCAUAU |
| 432 | | AS | AUAUGAGGUGAAAACACUGCUGG |
| 433 | BPR-302217 | SS | AAGUCCAGGCAGAGACAAUAA |
| 434 | | AS | UUAUUGUCUCUGCCUGGACUUCU |
| 435 | BPR-302218 | SS | AGUCCAGGCAGAGACAAUAAA |
| 436 | | AS | UUUAUUGUCUCUGCCUGGACUUC |
| 437 | BPR-302219 | SS | GUCCAGGCAGAGACAAUAAAA |
| 438 | | AS | UUUUAUUGUCUCUGCCUGGACUU |
| 439 | BPR-302220 | SS | CCAGGCAGAGACAAUAAAACA |
| 440 | | AS | UGUUUUAUUGUCUCUGCCUGGAC |
| 441 | BPR-302221 | SS | CAGGCAGAGACAAUAAAACAU |
| 442 | | AS | AUGUUUUAUUGUCUCUGCCUGGA |
| 443 | BPR-302222 | SS | AGGCAGAGACAAUAAAACAUU |
| 444 | | AS | AAUGUUUUAUUGUCUCUGCCUGG |
| 445 | BPR-302223 | SS | GGCAGAGACAAUAAAACAUUA |
| 446 | | AS | UAAUGUUUUAUUGUCUCUGCCUG |
| 447 | BPR-302224 | SS | GCAGAGACAAUAAAACAUUCA |
| 448 | | AS | UGAAUGUUUUAUUGUCUCUGCCU |
| 449 | BPR-302225 | SS | CAGAGACAAUAAAACAUUCCU |
| 450 | | AS | AGGAAUGUUUUAUUGUCUCUGCC |
| 451 | BPR-302226 | SS | AGAGACAAUAAAACAUUCCUA |
| 452 | | AS | UAGGAAUGUUUUAUUGUCUCUGC |
| 453 | BPR-302227 | SS | GAGACAAUAAAACAUUCCUGU |
| 454 | | AS | ACAGGAAUGUUUUAUUGUCUCUG |
| 455 | BPR-302228 | SS | AGACAAUAAAACAUUCCUGUA |
| 456 | | AS | UACAGGAAUGUUUUAUUGUCUCU |
| 457 | BPR-302229 | SS | GACAAUAAAACAUUCCUGUGA |
| 458 | | AS | UCACAGGAAUGUUUUAUUGUCUC |
| 459 | BPR-302230 | SS | ACAAUAAAACAUUCCUGUGAA |
| 460 | | AS | UUCACAGGAAUGUUUUAUUGUCU |
| 461 | BPR-302231 | SS | CAAUAAAACAUUCCUGUGAAA |
| 462 | | AS | UUUCACAGGAAUGUUUUAUUGUC |
| 463 | BPR-302232 | SS | AAUAAAACAUUCCUGUGAAAA |
| 464 | | AS | UUUUCACAGGAAUGUUUUAUUGU |
| 465 | BPR-302233 | SS | AUAAAACAUUCCUGUGAAAGA |
| 466 | | AS | UCUUUCACAGGAAUGUUUUAUUG |
| 467 | BPR-302234 | SS | UAAAACAUUCCUGUGAAAGGA |
| 468 | | AS | UCCUUUCACAGGAAUGUUUUAUU |
| 469 | BPR-302235 | SS | AAAACAUUCCUGUGAAAGGCA |
| 470 | | AS | UGCCUUUCACAGGAAUGUUUUAU |
| 431 | BPR-302236 | SS | AGCAGUGUUUUCACCUCAUAU |
| 914 | | AS | AUAUGAGGUGAAAACACUGCUUU |
| 431 | BPR-302237 | SS | AGCAGUGUUUUCACCUCAUAU |
| 915 | | AS | AUAUGAGGUGAAAACACUGCUAU |

In Table 2, SS represents the sense strand, and AS represents the antisense strand; the uppercase letters "G", "C", "A", and "U" in the AS and SS strands represent ribonucleotides containing guanine, cytosine, adenine, and uracil as bases, respectively.

### 2. Preparation of modified siRNAs

The modified hTTR siRNAs in Table 2 were synthesized using the oligonucleotide solid-phase synthesis process. The specific modifications were as follows: For the SS strand, counted from the 5' end to the 3' end, positions 1, 2, 3, 4, 5, 6, 8, 12, 13, 14, 15, 16, 17, 18, 19, 20, and 21 were a 2'-O-methyl-modified nucleotide, and positions 7, 9, 10, and 11 were a 2'-fluoro-modified nucleotide. For the AS strand, counted from the 5' end to the 3' end, positions 1, 3, 4, 5, 7, 8, 9, 10, 11, 12, 13, 15, 17, 18, 19, 20, 21, 22, and 23 were a 2'-O-methyl-modified nucleotide, and positions 2, 6, 14, and 16 were a 2'-fluoro-modified nucleotide.

With a universal solid-phase synthesis support (UnyLinker^{TM} loaded HL Solid Supports, Kinovate Life Sciences) as the support and phosphoramidite nucleoside monomers as the starting material, the nucleoside monomers were linked one by one in the 3'-5' direction according to the oligonucleotide sequence arrangement by the phosphoramidite solid-phase synthesis method. Then, the crude product of the oligonucleotide was obtained by an ammonolysis reaction. After purification, ultrafiltration, and lyophilization of the crude product, two single-stranded oligonucleotides constituting the siRNA were obtained. Finally, the two single-stranded oligonucleotides are annealed to form a double-stranded siRNA with reverse complementary pairing of bases.

The solid-phase synthesis process mainly involved the following 4 reaction steps (a, b, c, and d), which constituted one cycle. The cycle was repeated to sequentially add different types of required nucleoside phosphoramidite monomers according to the oligonucleotide sequence. The 5'-DMTr group at the end of the oligonucleotide was cleaved, and cyanoethyl-protected phosphoester was removed using a solution of diethylamine in acetonitrile (20% (v/v)). After the solid-phase reaction was completed, an oligonucleotide single strand linked to the solid-phase support was obtained.
a. De-dimethoxytritylation reaction: Under room temperature conditions (20-25°C), the protecting group DMTr (dimethoxytrityl) on the support/nucleotide was removed with dichloroacetic acid to obtain an active hydroxyl group capable of undergoing a coupling reaction. The deprotecting reagent was a solution of dichloroacetic acid in toluene (3% (v/v)) or a solution of dichloroacetic acid in dichloromethane (3% (v/v)).
b. Coupling reaction: The nucleotide phosphoramidite monomer and an activator were simultaneously injected for solid-phase synthesis. The phosphoramidite group was activated and underwent a coupling condensation reaction with the active hydroxyl group to generate phosphite triester. The activator was a 0.6 M solution of 5-ethylthio-1H-tetrazole (ETT) in acetonitrile.
c. Oxidation reaction: Under the action of iodine-water as an oxidant, the phosphite triester generated in the previous coupling condensation reaction was converted into stable phosphate triester. The oxidant was an iodine/water/pyridine solution at a concentration of 0.04 M, with v(water):v(pyridine) = 1:9.
d. Capping reaction: The active hydroxyl groups that did not react completely during the coupling reaction were capped, so that they did not participate in subsequent reactions. The capping reagents were CapA (acetic anhydride/acetonitrile) and CapB (NMI:Py:acetonitrile = 2:3:5).

An ammonolysis reaction was performed on the solid-phase support linked to the oligonucleotide described above using an ammonolysis reagent (25% to 28% concentrated aqueous ammonia solution). While the oligonucleotide was cleaved and separated from the support, various protecting groups on the nucleoside bases were removed. The resulting solution was concentrated to obtain a crude product of the oligonucleotide. The crude product was sent for a test, and the target molecular weight was measured by high-resolution liquid chromatography-mass spectrometry (LC-MS).

The crude product of the oligonucleotide was purified by anion exchange chromatography and desalted using a gel column (Cytiva, HiTrap^{TM}Desalting). The desalted sense strand and antisense strand were mixed at an equimolar ratio, heated to 65 °C, maintained for 30 min, and then naturally cooled to room temperature. The two single strands formed a double-stranded structure, i.e., siRNA, through hydrogen bonding. The molecular weight measurement was performed using ion-pair reversed-phase chromatography (IPRP-HPLC) and high-resolution liquid chromatography-mass spectrometry (LC-MS) to confirm that the modified siRNAs were prepared from the motif siRNAs shown in Table 2.

### Example 2: Assay of hTTR siRNA Activity in Liver Cancer Cells

### 1. Cell culture and transfection

Under a culture environment of 37 °C and 5% CO₂, liver cancer cell lines HepG2 (ATCC, HB-8065) and Hep3B (ATCC, HB-8064) were cultured in an EMEM culture medium (ATCC, 30-2003) until approaching a confluent state, and then the cells were released from the plate by the action of trypsin digestion. 5 µL of siRNA and 5 µL of mix (4.9 µL of Opti-MEM and 0.1 µL of Lipofectamine RNAiMax (Invitrogen, Cat. No. 13778-150)) were added to a 96-well plate and incubated at room temperature for 15-20 min. Then, 90 µL of a complete growth culture medium without antibiotics but containing 4000 liver cancer cells was added to each well of the 96-well plate for reverse transfection; meanwhile, a negative control group transfected without the siRNA compound (i.e., only the solvent PBS) was set.

### 2. RT-qPCR

After incubation for 48 h under a culture environment of 37 °C and 5% CO₂, cell lysis, RNA extraction, and RT-qPCR were performed on the transfected cells. RNA extraction was performed using the Fast Pure Universal Plant Total RNA Isolation Kit (VAZYME, Cat. No.: RC411-01), and reverse transcription was performed using the HiScriptIII 1st Strand cDNA Synthesis Kit (+gDNA wiper) (VAZYME, Cat. No.: R312-01), both of which were performed according to the instructions. A PCR mixture (10 µL of 2XSYBR Mix, 0.4 µL of Primer mix (0.2 µL of a 10 µM forward primer and 0.2 µL of a 10 µM reverse primer)) was added to a 96-well plate (Applied Biosystems, Cat. No.: 4326659). Subsequently, 2 µL of the corresponding cDNA was added to each well, followed by centrifugation to mix thoroughly. The expression levels of the target gene TTR and the internal reference gene GAPDH mRNA were measured by qPCR. The sequences of the primers were as follows:
TTR forward primer: 5'-CCCCTACTCCTATTCCACC-3' (SEQ ID NO: 885);
TTR reverse primer: 5'-GAAATCCCATCCCTCGTC-3' (SEQ ID NO: 886);
GAPDH forward primer: 5'-CCCTACTCCTATTCCACCAC-3' (SEQ ID NO: 887);
GAPDH reverse primer: 5'-GAAATCCCATCCCTCGTC-3' (SEQ ID NO: 888).

The relative expression level of TTR mRNA was represented by 2^{-ΔΔCT}, and the calculation formulas were as follows:$Relative expression level of TTR mRNA = {2}^{- ΔΔCT}$ 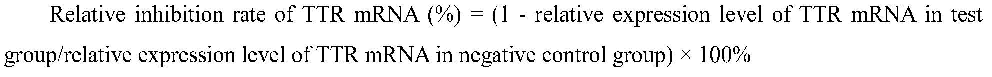 Relative inhibition rate of TTR mRNA (%) = (1 - relative expression level of TTR mRNA in test group/relative expression level of TTR mRNA in negative control group) × 100%

### 3. In vitro screening results of liver cancer cell activity

The TTR inhibition rates of the single-concentration siRNAs in HepG2 were about 10%, 20%, 30%, 40%, 50%, 60%, 70%, 80%, and 90%, and the maximum inhibition rate was 98.60%; the TTR inhibition rates of the single-concentration siRNAs in Hep3B were about 10%, 20%, 30%, 40%, 50%, 60%, 70%, 80%, and 90%, and the maximum inhibition rate was 95.85%. The results are shown in Table 3.

**Table 3**

| siRNA No. | HepG2 single-concentration screening | | Hep3B single-concentration screening | |
|---|---|---|---|---|
| | siRNA screening concentration (nM) | Relative inhibition rate of TTR mRNA (%) | siRNA screening concentration (nM) | Relative inhibition rate of TTR mRNA (%) |
| BPR-302001 | 0.125 | 58.40 | ND | ND |
| BPR-302002 | 0.125 | 4.21 | ND | ND |
| BPR-302003 | 0.125 | 74.09 | ND | ND |
| BPR-302004 | 0.125 | 94.68 | 0.01 | 23.41 |
| BPR-302005 | 0.125 | 51.20 | ND | ND |
| BPR-302006 | 0.125 | 91.72 | 0.01 | 22.57 |
| BPR-302007 | 0.125 | 63.83 | ND | ND |
| BPR-302008 | 0.125 | 76.68 | ND | ND |
| BPR-302009 | 0.125 | 43.62 | ND | ND |
| BPR-302010 | 0.125 | 51.68 | ND | ND |
| BPR-302011 | 0.125 | 58.24 | ND | ND |
| BPR-302012 | 0.125 | 64.05 | ND | ND |
| BPR-302013 | 0.125 | 45.24 | ND | ND |
| BPR-302014 | 0.125 | 72.77 | 0.01 | 38.84 |
| BPR-302015 | 0.125 | 18.10 | ND | ND |
| BPR-302016 | 0.125 | 18.91 | ND | ND |
| BPR-302017 | 0.125 | 90.27 | ND | ND |
| BPR-302018 | 0.125 | 98.09 | 0.01 | -9.56 |
| BPR-302019 | 0.125 | 22.08 | ND | ND |
| BPR-302020 | 0.125 | 91.81 | ND | ND |
| BPR-302021 | 0.125 | 93.46 | ND | ND |
| BPR-302022 | 0.125 | 52.81 | ND | ND |
| BPR-302023 | 0.125 | 96.88 | 0.01 | 26.47 |
| BPR-302024 | 0.125 | 97.27 | 0.01 | 16.73 |
| BPR-302025 | 0.125 | 98.34 | 0.01 | 15.55 |
| BPR-302026 | 0.125 | 82.25 | ND | ND |
| BPR-302027 | 0.125 | 98.60 | 0.01 | 37.96 |
| BPR-302028 | 0.125 | 97.97 | 0.01 | 34.70 |
| BPR-302029 | 0.125 | 71.16 | ND | ND |
| BPR-302030 | 0.125 | 68.19 | ND | ND |
| BPR-302031 | 0.125 | 89.08 | ND | ND |
| BPR-302032 | 0.125 | 58.32 | 0.01 | 26.22 |
| BPR-302033 | 0.125 | 63.46 | ND | ND |
| BPR-302034 | 0.125 | 34.70 | ND | ND |
| BPR-302035 | 0.125 | 48.45 | ND | ND |
| BPR-302036 | 0.125 | 49.29 | ND | ND |
| BPR-302037 | 0.125 | 13.07 | ND | ND |
| BPR-302038 | 0.125 | 58.95 | ND | ND |
| BPR-302039 | 0.125 | -42.80 | ND | ND |
| BPR-302040 | 0.125 | -8.65 | ND | ND |
| BPR-302041 | 0.125 | 48.82 | 0.01 | 42.84 |
| BPR-302042 | 0.125 | 48.73 | 0.01 | 47.68 |
| BPR-302043 | 0.125 | 22.25 | ND | ND |
| BPR-302044 | 0.125 | -40.42 | 0.01 | 47.71 |
| BPR-302045 | 0.125 | 47.24 | 0.01 | 58.73 |
| BPR-302046 | 0.125 | 13.78 | ND | ND |
| BPR-302047 | 0.125 | 35.07 | 0.01 | 53.39 |
| BPR-302048 | 0.125 | 26.11 | ND | ND |
| BPR-302049 | 0.125 | 46.85 | 0.01 | 26.02 |
| BPR-302050 | 0.125 | 57.63 | 0.01 | 15.05 |
| BPR-302051 | 0.125 | 38.11 | ND | ND |
| BPR-302052 | 0.125 | 21.83 | ND | ND |
| BPR-302053 | 0.125 | -48.11 | ND | ND |
| BPR-302054 | 0.125 | 61.43 | 0.01 | 2.05 |
| BPR-302055 | 0.125 | -18.51 | ND | ND |
| BPR-302056 | 0.125 | 93.91 | 0.12 | 52.30 |
| BPR-302057 | 0.125 | 27.25 | ND | ND |
| BPR-302058 | 0.125 | 39.87 | ND | ND |
| BPR-302059 | 0.125 | 53.80 | ND | ND |
| BPR-302060 | 0.125 | -74.91 | 0.12 | 88.17 |
| BPR-302061 | 0.125 | 81.18 | ND | ND |
| BPR-302062 | 0.125 | 69.03 | ND | ND |
| BPR-302063 | 0.125 | 88.82 | ND | ND |
| BPR-302064 | 0.125 | 98.43 | ND | ND |
| BPR-302065 | 0.125 | 0.00 | ND | ND |
| BPR-302066 | 0.125 | 57.70 | 0.12 | 6.44 |
| BPR-302067 | 0.125 | -101.45 | 0.12 | -0.79 |
| BPR-302068 | 0.125 | 41.75 | 0.12 | 39.65 |
| BPR-302069 | 0.125 | 40.10 | 0.12 | 91.44 |
| BPR-302070 | 0.125 | 30.40 | 0.12 | 68.75 |
| BPR-302071 | 0.125 | 0.00 | ND | ND |
| BPR-302072 | 0.125 | 52.33 | 0.12 | 54.92 |
| BPR-302073 | 0.125 | 0.00 | ND | ND |
| BPR-302074 | 0.125 | 0.00 | ND | ND |
| BPR-302075 | 0.125 | 0.00 | ND | ND |
| BPR-302076 | 0.125 | 0.00 | ND | ND |
| BPR-302077 | 0.125 | -12.14 | ND | ND |
| BPR-302078 | 0.125 | ND | ND | ND |
| BPR-302079 | 0.125 | -104.11 | ND | ND |
| BPR-302080 | 0.125 | 65.75 | 0.12 | 58.28 |
| BPR-302081 | 0.125 | 0.00 | ND | ND |
| BPR-302082 | 0.125 | 95.62 | 0.12 | 61.48 |
| BPR-302083 | ND | ND | 0.12 | 44.59 |
| BPR-302084 | ND | ND | 0.12 | 16.34 |
| BPR-302085 | ND | ND | 0.12 | 54.29 |
| BPR-302086 | ND | ND | 0.12 | 34.28 |
| BPR-302087 | ND | ND | 0.12 | 72.98 |
| BPR-302088 | ND | ND | 0.12 | 72.07 |
| BPR-302089 | ND | ND | 0.12 | 54.58 |
| BPR-302090 | ND | ND | 0.12 | 60.81 |
| BPR-302091 | ND | ND | 0.12 | 89.68 |
| BPR-302092 | ND | ND | 0.12 | 95.85 |
| BPR-302093 | ND | ND | 0.12 | 57.77 |
| BPR-302094 | ND | ND | 0.12 | 85.49 |
| BPR-302095 | ND | ND | 0.12 | 92.92 |
| BPR-302096 | ND | ND | 0.12 | 82.08 |
| BPR-302097 | ND | ND | 0.12 | 83.25 |
| BPR-302098 | ND | ND | 0.12 | 85.81 |
| BPR-302099 | ND | ND | 0.12 | 60.69 |
| BPR-302100 | ND | ND | 0.12 | 79.42 |
| BPR-302101 | ND | ND | 0.12 | 72.85 |
| BPR-302102 | ND | ND | 0.12 | 70.38 |
| BPR-302103 | ND | ND | 0.12 | 64.88 |
| BPR-302104 | ND | ND | 0.12 | 95.28 |
| BPR-302105 | ND | ND | 0.12 | 87.43 |
| BPR-302106 | ND | ND | 0.12 | 8.16 |
| BPR-302107 | ND | ND | 0.03 | 13.61 |
| BPR-302108 | ND | ND | 0.03 | 34.95 |
| BPR-302109 | ND | ND | 0.03 | 5.58 |
| BPR-302110 | ND | ND | 0.03 | 13.94 |
| BPR-302111 | ND | ND | 0.03 | 26.71 |
| BPR-302112 | ND | ND | 0.03 | 22.78 |
| BPR-302113 | ND | ND | 0.03 | -2.78 |
| BPR-302114 | ND | ND | 0.03 | 14.69 |
| BPR-302115 | ND | ND | 0.03 | 21.24 |
| BPR-302116 | ND | ND | 0.03 | 8.50 |
| BPR-302117 | ND | ND | 0.03 | 20.30 |
| BPR-302118 | ND | ND | 0.03 | 48.18 |
| BPR-302119 | ND | ND | ND | ND |
| BPR-302120 | 0.125 | 20.21 | ND | ND |
| BPR-302121 | 0.125 | 24.39 | ND | ND |
| BPR-302122 | 0.125 | -119.16 | ND | ND |
| BPR-302123 | 0.125 | -152.75 | ND | ND |
| BPR-302124 | 0.125 | -267.19 | ND | ND |
| BPR-302125 | ND | ND | 0.03 | 29.50 |
| BPR-302126 | ND | ND | 0.03 | 13.70 |
| BPR-302127 | ND | ND | 0.03 | 14.68 |
| BPR-302128 | ND | ND | 0.03 | 32.40 |
| BPR-302129 | ND | ND | 0.03 | 49.78 |
| BPR-302130 | ND | ND | 0.03 | 71.50 |
| BPR-302131 | 0.125 | -57.51 | ND | ND |
| BPR-302132 | 0.125 | -136.99 | ND | ND |
| BPR-302133 | 0.125 | 26.62 | ND | ND |
| BPR-302134 | 0.125 | -78.26 | ND | ND |
| BPR-302135 | 0.125 | -221.60 | ND | ND |
| BPR-302136 | 0.125 | -42.76 | ND | ND |
| BPR-302137 | 0.125 | 45.51 | ND | ND |
| BPR-302138 | 0.125 | 43.50 | ND | ND |
| BPR-302139 | 0.125 | 57.03 | ND | ND |
| BPR-302140 | 0.125 | 5.36 | ND | ND |
| BPR-302141 | 0.125 | 40.69 | ND | ND |
| BPR-302142 | 0.125 | 32.10 | ND | ND |
| BPR-302143 | 0.125 | -251.95 | ND | ND |
| BPR-302144 | 0.125 | -5.19 | ND | ND |
| BPR-302145 | 0.125 | -22.90 | ND | ND |
| BPR-302146 | 0.125 | 33.03 | ND | ND |
| BPR-302147 | 0.125 | -119.22 | ND | ND |
| BPR-302148 | 0.125 | -55.62 | ND | ND |
| BPR-302149 | 0.125 | 2.31 | ND | ND |
| BPR-302150 | 0.125 | -38.83 | ND | ND |
| BPR-302151 | 0.125 | -66.93 | ND | ND |
| BPR-302152 | 0.125 | -83.04 | ND | ND |
| BPR-302153 | 0.125 | -20.42 | ND | ND |
| BPR-302154 | 0.125 | -73.06 | ND | ND |
| BPR-302155 | ND | ND | 0.03 | 22.98 |
| BPR-302156 | ND | ND | 0.03 | 13.24 |
| BPR-302157 | ND | ND | 0.03 | 22.21 |
| BPR-302158 | ND | ND | 0.03 | 20.48 |
| BPR-302159 | ND | ND | 0.03 | 23.86 |
| BPR-302160 | ND | ND | 0.03 | -1.56 |
| BPR-302161 | 0.125 | 67.48 | ND | ND |
| BPR-302162 | 0.125 | 81.54 | ND | ND |
| BPR-302163 | 0.125 | 72.35 | ND | ND |
| BPR-302164 | 0.125 | 74.99 | ND | ND |
| BPR-302165 | 0.125 | 42.80 | ND | ND |
| BPR-302166 | 0.125 | 40.92 | ND | ND |
| BPR-302167 | 0.125 | 54.29 | ND | ND |
| BPR-302168 | 0.125 | -2.16 | ND | ND |
| BPR-302169 | 0.125 | 38.01 | ND | ND |
| BPR-302170 | 0.125 | 69.63 | ND | ND |
| BPR-302171 | 0.125 | 55.26 | 0.03 | 4.53 |
| BPR-302172 | 0.125 | -18.93 | ND | ND |
| BPR-302173 | 0.125 | 12.43 | ND | ND |
| BPR-302174 | 0.125 | -43.61 | ND | ND |
| BPR-302175 | 0.125 | -1.17 | ND | ND |
| BPR-302176 | 0.125 | -0.57 | ND | ND |
| BPR-302177 | 0.125 | 62.72 | 0.03 | 10.52 |
| BPR-302178 | 0.125 | -17.95 | ND | ND |
| BPR-302179 | 0.125 | 24.28 | ND | ND |
| BPR-302180 | 0.125 | 0.54 | ND | ND |
| BPR-302181 | 0.125 | -21.49 | ND | ND |
| BPR-302182 | 0.125 | 51.23 | 0.03 | 11.18 |
| BPR-302183 | 0.125 | 34.60 | ND | ND |
| BPR-302184 | 0.125 | 0.93 | ND | ND |
| BPR-302185 | 0.125 | 30.87 | ND | ND |
| BPR-302186 | 0.125 | 7.30 | ND | ND |
| BPR-302187 | 0.125 | 25.92 | ND | ND |
| BPR-302188 | 0.125 | 4.20 | ND | ND |
| BPR-302189 | 0.125 | 14.96 | ND | ND |
| BPR-302190 | 0.125 | -31.01 | ND | ND |
| BPR-302191 | 0.125 | -65.48 | ND | ND |
| BPR-302192 | 0.125 | -35.69 | ND | ND |
| BPR-302193 | 0.125 | -10.15 | ND | ND |
| BPR-302194 | 0.125 | -63.55 | ND | ND |
| BPR-302195 | 0.125 | -65.95 | ND | ND |
| BPR-302196 | 0.125 | -74.86 | ND | ND |
| BPR-302197 | 0.125 | -38.83 | ND | ND |
| BPR-302198 | 0.125 | -58.06 | ND | ND |
| BPR-302199 | 0.125 | 0.15 | ND | ND |
| BPR-302200 | 0.125 | -15.95 | ND | ND |
| BPR-302201 | 0.125 | 38.95 | 0.03 | 10.77 |
| BPR-302202 | 0.125 | 13.11 | ND | ND |
| BPR-302203 | 0.125 | 2.78 | ND | ND |
| BPR-302204 | 0.125 | -36.22 | ND | ND |
| BPR-302205 | 0.125 | 37.50 | 0.03 | 15.66 |
| BPR-302206 | 0.125 | 15.47 | ND | ND |
| BPR-302207 | ND | ND | ND | ND |
| BPR-302208 | 0.125 | 83.65 | ND | ND |
| BPR-302209 | 0.125 | 82.90 | ND | ND |
| BPR-302210 | 0.125 | 72.77 | ND | ND |
| BPR-302211 | 0.125 | 50.72 | ND | ND |
| BPR-302212 | 0.125 | 72.38 | ND | ND |
| BPR-302213 | 0.125 | 52.32 | ND | ND |
| BPR-302214 | 0.125 | 77.14 | ND | ND |
| BPR-302215 | 0.125 | 64.87 | ND | ND |
| BPR-302216 | 0.125 | 82.30 | ND | ND |
| BPR-302217 | 0.125 | 45.57 | ND | ND |
| BPR-302218 | 0.125 | 56.41 | 0.03 | 13.18 |
| BPR-302219 | 0.125 | 68.84 | ND | ND |
| BPR-302220 | 0.125 | 54.02 | 0.03 | 23.46 |
| BPR-302221 | 0.125 | 60.65 | ND | ND |
| BPR-302222 | 0.125 | 52.96 | ND | ND |
| BPR-302223 | 0.125 | 53.74 | ND | ND |
| BPR-302224 | 0.125 | 47.40 | ND | ND |
| BPR-302225 | 0.125 | 61.05 | ND | ND |
| BPR-302226 | 0.125 | 64.21 | ND | ND |
| BPR-302227 | 0.125 | 64.43 | ND | ND |
| BPR-302228 | 0.125 | 71.41 | ND | ND |
| BPR-302229 | 0.125 | 56.29 | ND | ND |
| BPR-302230 | 0.125 | 61.18 | ND | ND |
| BPR-302231 | 0.125 | 91.69 | ND | ND |
| BPR-302232 | 0.125 | 68.08 | ND | ND |
| BPR-302233 | 0.125 | 73.62 | ND | ND |
| BPR-302234 | 0.125 | 58.23 | ND | ND |
| BPR-302235 | 0.125 | 45.99 | ND | ND |

| | | | | |
|---|---|---|---|---|
| ND indicates that the sample was not detected. | | | | |

**Table 4. Hep3B multi-concentration screening**

| siRNA | sample location(NM 000371.4) | 0.08 nM relative inhibition rate | 0.04 nM relative inhibition rate | 0.02 nM relative inhibition rate | 0.08 nM relative inhibition rate SD | 0.04 nM relative inhibition rate SD | 0.02 nM relative inhibition rate SD |
|---|---|---|---|---|---|---|---|
| BPR-302033 | 108 | 72.03% | 51.43% | 49.35% | 0.06 | 0.02 | 0.04 |
| BPR-302164 | 355 | 83.35% | 67.86% | 35.51% | 0.00 | 0.03 | 0.04 |
| BPR-302170 | 413 | 79.79% | 60.81% | 31.59% | 0.07 | 0.08 | 0.10 |
| BPR-302209 | 539 | 92.92% | 81.12% | 52.29% | 0.01 | 0.02 | 0.07 |
| BPR-302210 | 540 | 93.16% | 90.46% | 86.47% | 0.00 | 0.00 | 0.03 |
| BPR-302212 | 542 | 92.70% | 86.40% | 80.18% | 0.02 | 0.01 | 0.06 |
| BPR-302214 | 544 | 94.13% | 87.18% | 69.96% | 0.02 | 0.01 | 0.06 |
| BPR-302215 | 545 | 92.06% | 85.45% | 64.81% | 0.01 | 0.02 | 0.02 |
| BPR-302216 | 546 | 90.76% | 76.67% | 50.76% | 0.00 | 0.01 | 0.04 |
| BPR-302219 | 579 | 90.50% | 86.53% | 69.63% | 0.01 | 0.03 | 0.05 |
| BPR-302221 | 582 | 94.05% | 90.62% | 80.10% | 0.00 | 0.01 | 0.03 |
| BPR-302222 | 583 | 93.90% | 91.75% | 83.38% | 0.01 | 0.00 | 0.02 |
| BPR-302223 | 584 | 90.26% | 81.60% | 69.91% | 0.01 | 0.01 | 0.01 |
| BPR-302224 | 585 | 86.96% | 70.88% | 53.94% | 0.03 | 0.02 | 0.01 |
| BPR-302225 | 586 | 90.52% | 82.64% | 65.30% | 0.01 | 0.02 | 0.02 |
| BPR-302226 | 587 | 93.10% | 88.45% | 79.45% | 0.00 | 0.01 | 0.02 |
| BPR-302227 | 588 | 84.22% | 70.37% | 50.68% | 0.03 | 0.04 | 0.02 |
| BPR-302228 | 589 | 75.12% | 70.39% | 53.64% | 0.05 | 0.02 | 0.05 |
| BPR-302229 | 590 | 66.35% | 50.59% | 23.96% | 0.05 | 0.07 | 0.02 |
| BPR-302230 | 591 | 46.63% | 31.65% | 1.98% | 0.06 | 0.08 | 0.03 |
| BPR-302231 | 592 | 57.65% | 47.19% | 24.24% | 0.07 | 0.09 | 0.08 |
| BPR-302232 | 593 | 91.02% | 84.47% | 71.39% | 0.01 | 0.04 | 0.08 |
| BPR-302233 | 594 | 46.13% | 41.39% | 23.72% | 0.08 | 0.14 | 0.03 |
| BPR-302234 | 595 | 58.63% | 35.06% | 15.81% | 0.04 | 0.06 | 0.07 |

### Example 3: Preparation of 4'-Modified Threose Nucleosides

### 1. Synthesis of compound 11

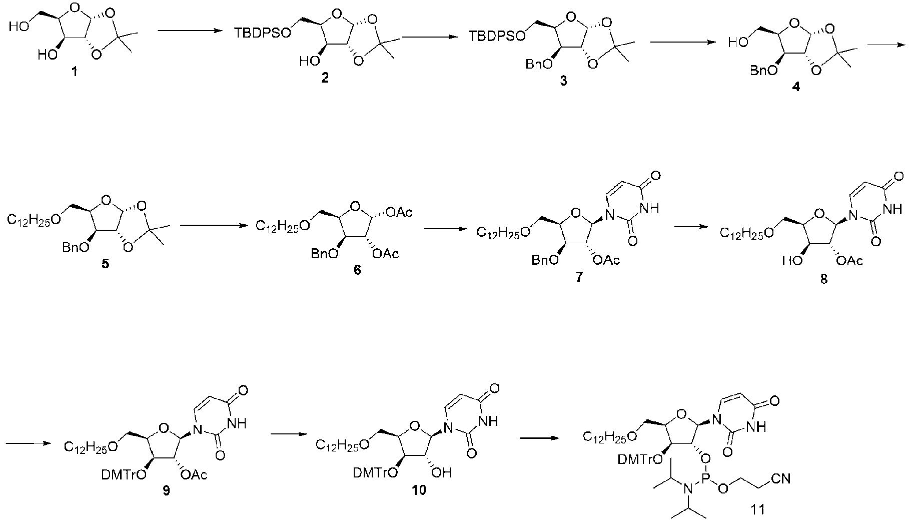

### 1) Synthesis of compound 2:

Dry compound 1 (20.0 g, 105.15 mmol, 1.0 eq.) was dissolved in DCM (200 mL). Imidazole (17.9 g, 262.88 mmol, 2.5 eq.) was added to the reaction system. The reaction system was cooled to 0 °C and successively stirred for 30 min. tert-Butyldiphenylchlorosilane (31.79 g, 115.67 mmol, 1.1 eq.) was then slowly added dropwise to the reaction system. After the addition was completed, the ice bath was removed, and the reaction was gradually warmed to room temperature. The reaction was performed at room temperature overnight. The reaction of compound 1 was completed as determined by TLC. Water was added to the reaction system, and then the mixture was extracted twice with ethyl acetate. The organic phases were combined and washed with water and saturated brine. The organic phase was dried over anhydrous sodium sulfate and concentrated under reduced pressure to obtain a crude product. The crude product was subjected to column chromatography (PE/EA = 100/15) to obtain compound 2 (35.24 g, 82.29 mmol, yield: 78.3%). ESI-MS: m/z 451.2 [M+Na]⁺

### 2) Synthesis of compound 3:

Compound 2 (38.2 g, 89.13 mmol, 1.0 eq.) was dissolved in DMF (300 mL), and the mixture was stirred until complete dissolution. The reaction system was cooled to about 0 °C. NaH (5.35 g, 133.70 mmol, 1.5 eq.) was slowly added in batches to the reaction, and after the addition was completed, the reaction system was stirred at 0 °C for 30 min. Benzyl bromide (22.87 g, 133.70 mmol, 1.5 eq.) was slowly added dropwise to the reaction system, and the temperature of the reaction system was maintained at about 0 °C. After the addition was completed, the reaction system was warmed to room temperature and reacted at room temperature overnight. The reaction of compound 2 was completed as determined by TLC. The reaction system was slowly poured into a saturated aqueous ammonium chloride solution at about 0 °C, and the mixed system was extracted twice with ethyl acetate. The organic phases were combined. The organic phase was washed with water and saturated brine, dried over anhydrous sodium sulfate, and concentrated under reduced pressure to obtain a crude product of compound 3 (68.2 g), which was directly used in the next step. ESI-MS: m/z 519.3 [M+H]⁺

### 3) Synthesis of compound 4:

The crude product of compound 3 (68.2 g) was dissolved in THF (400 mL), and the mixture was stirred until complete dissolution. TBAF (1 M in THF, 100 mL, 100 mmol, 1.12 eq.) was added to the reaction, and the mixture was stirred at room temperature overnight until the reaction of compound 3 was completed. The reaction liquid was concentrated under reduced pressure to remove the solvent, and the resulting crude product was subjected to column chromatography (PE/EA = 10/3) to obtain compound 4 (22.07 g, 78.73 mmol, two-step yield: 88.3%).

### 4) Synthesis of compound 5:

Compound 4 (22.07 g, 78.73 mmol, 1.0 eq.) was dissolved in DMF (200 mL), and the mixture was stirred homogeneously. The reaction was cooled to 0 °C and stirred for 30 min. Compound bromododecane (23.55 g, 94.48 mmol, 1.2 eq.) was slowly added dropwise to the reaction system. After the addition was completed, the reaction was warmed to room temperature, and the reaction was performed at room temperature overnight. The reaction of compound 4 was completed as determined by TLC. The reaction system was slowly poured into ice water until quenching was completed. The mixed system was extracted twice with ethyl acetate, and the organic phases were combined. The organic phase was washed with water and saturated brine, dried over anhydrous sodium sulfate, and concentrated under reduced pressure to remove the solvent to obtain a crude compound. The crude product was subjected to column chromatography (PE/EA = 100/9) to obtain compound 5 (32.0 g, 71.37 mmol, yield: 90.6%).

### 5) Synthesis of compound 6:

Compound 5 (35.32 g, 78.73 mmol, 1.0 eq.) was added to a 500 mL round-bottom three-necked flask, and acetic acid (120 mL) and acetic anhydride (40.19 g, 393.65 mmol, 5.0 eq.) were added to the reaction system. The mixture was stirred until complete dissolution. The reaction was cooled to 0 °C and stirred at this temperature for 30 min. Concentrated sulfuric acid (5 mL) was slowly added dropwise to the reaction system with the temperature maintained at 0-5 °C at all times. After the addition was completed, the reaction system was warmed to room temperature and stirred for another 8 h until the reaction of compound 5 was completed. The reaction system was cooled to about -5 °C and neutralized to about pH 7 with ammonia water. Water was added to the reaction system, the reaction was extracted twice with ethyl acetate, and the organic phases were combined. The organic phase was washed with water and saturated brine, dried over anhydrous sodium sulfate, and concentrated under reduced pressure to obtain crude product 6. Crude product 6 was subjected to column chromatography (PE/EA = 10/2) to obtain compound 6 (19.0 g, 38.59 mmol, yield: 49%).

### 6) Synthesis of compound 7:

Compound 6 (5.0 g, 10.15 mmol, 1.00 eq.) and uracil (2.28 g, 20.30 mmol, 2.0 eq.) were added to a 500 mL three-necked round-bottom flask, and extra dry acetonitrile (60 mL) was added to the reaction flask. The mixture was stirred for dissolution, and then BSA (6.19 g, 30.45 mmol, 3.0 eq.) was added. The reaction system was placed in an oil bath, heated to 80 °C, and stirred at this temperature for 1 h. The entire reaction was performed under a nitrogen atmosphere. After the reaction was completed, the reaction system was placed in an ice-water bath at 0 °C and stirred for 30 min, and TMSOTf (2.26 g, 10.15 mmol, 1.0 eq.) was slowly added dropwise to the reaction system. After the addition was completed, the reaction was placed in an oil bath and slowly heated to 80 °C, and the reaction was performed at this temperature overnight. The reaction of compound 6 was completed as determined by TLC and LCMS. The reaction was removed from the oil bath and cooled to room temperature. A saturated aqueous sodium bicarbonate solution was added to the reaction system to quench the reaction, the system was extracted with ethyl acetate, and the organic phases were combined. The organic phase was washed with water and saturated brine, dried over anhydrous sodium sulfate, and concentrated under reduced pressure to remove the solvent to obtain crude product 7. The crude product was subjected to column chromatography (PE/EA = 5/2) to obtain compound 7 (4.71 g, 8.65 mmol, yield: 85.2%).

### 7) Synthesis of compound 8:

Compound 7 (4.71 g, 8.65 mmol) was added to a 250 mL three-necked flask, and extra dry dichloromethane (50 mL) was added to the reaction flask. The reaction system was cooled to -20 °C and stirred at this temperature for 30 min. Boron trichloride (1 M in toluene, 26 mL, 25.95 mmol, 3.0 eq.) was slowly added dropwise to the reaction system, and the reaction was performed for another 5 h until the reaction of compound 7 was completed. The reaction was quenched with triethylamine and methanol at - 20 °C. The reaction was warmed to room temperature, and water was added. The mixed system was extracted with ethyl acetate, and the organic phases were combined. The organic phase was washed with water and saturated brine, dried over anhydrous sodium sulfate, and concentrated under reduced pressure to remove the solvent to obtain crude product 8. The crude compound was subjected to column chromatography (PE/EA = 2/3) to obtain compound 8 (2.0 g, 4.4 mmol, yield: 50.9%). ESI-MS: m/z 455.3 [M+H]⁺

### 8) Synthesis of compound 9:

Compound 8 (2.0 g, 4.40 mmol, 1.0 eq.) was added to a 250 mL round-bottom flask, and extra dry DCE (30 mL) was added to the reaction flask. The mixture was stirred until complete dissolution. DMTrCl (7.45 g, 22.00 mmol, 5.0 eq.), silver nitrate (747 mg, 4.40 mmol, 1.0 eq.), and 2,4,6-trimethylpyridine (5.33 g, 44 mmol, 10.0 eq.) were added to the reaction at room temperature, and the mixture was stirred homogeneously at room temperature. The reaction was placed in an oil bath and heated to 80 °C, and the reaction was performed overnight. The reaction of compound 8 was completed as determined by TLC and LCMS. The reaction was cooled to room temperature and quenched with the addition of methanol. Ethyl acetate was added to the reaction for dilution, and the mixture was filtered through celite to obtain a filtrate. The filter cake was washed twice with ethyl acetate. The filtrates were combined and concentrated under reduced pressure to obtain crude compound 9. The crude product was subjected to column chromatography (PE/EA = 100/35) to obtain compound 9 (3.2 g, 4.23 mmol, yield: 96%). ESI-MS: m/z 757.4 [M+H]⁺

### 9) Synthesis of compound 10:

Compound 9 (3.2 g, 4.23 mmol, 1.0 eq.) was added to a 100 mL round-bottom flask, and then a 7 M aqueous ammonia solution (50 mL) was added to the reaction at room temperature. The reaction was stirred at room temperature for 3 h until the reaction of compound 9 was completed. After the reaction was completed, the reaction system was concentrated under reduced pressure at 40 °C to obtain crude product 10. The crude product was subjected to column chromatography (PE/EA = 1/1) to obtain compound 10 (2.9 g, 4.06 mmol, yield: 96%).

### 10) Synthesis of compound 11:

Dry compound 10 (2.9 g, 4.06 mmol, 1.0 eq.) was added to a 100 mL round-bottom flask, and extra dry dichloromethane (30 mL) was added to the flask. The mixture was stirred until complete dissolution. DIPEA (1.05 g, 8.12 mmol, 2.0 eq.) and DMAP (99 mg, 0.81 mmol, 0.2 eq.) were added to the reaction, and the mixture was stirred at room temperature for 15 min. The reaction system was purged with nitrogen and performed under a nitrogen atmosphere. Compound CEP-Cl (1.44 g, 6.09 mmol, 1.5 eq.) was added dropwise to the reaction at room temperature. The reaction was performed at room temperature for 30-60 min until the reaction of compound 10 was completed. The reaction system was quenched by adding a saturated aqueous sodium bicarbonate solution and extracted twice with dichloromethane. The organic phases were combined, washed with water and saturated brine, dried over anhydrous sodium sulfate, and concentrated under reduced pressure to remove the solvent to obtain crude product 11. The crude product was purified by column chromatography (PE/EA = 1/1) to obtain compound 11 (3.16 g, 3.46 mmol, yield: 85.2%). ¹H NMR (400 MHz, DMSO-*d*₆) δ 11.35 (dd, *J* = 14.6, 2.2 Hz, 1H), 7.93 (d, *J* = 8.1 Hz, 1H), 7.47 - 7.36 (m, 2H), 7.33 - 7.23 (m, 7H), 6.89 - 6.83 (m, 4H), 5.71 - 5.51 (m, 2H), 4.34 (dt, *J* = 8.8, 4.7 Hz, 1H), 4.11 - 3.90 (m, 1H), 3.84 - 3.76 (m, 1H), 3.75 (s, 6H), 3.69 - 3.36 (m, 6H), 2.71 (t, *J* = 6.0 Hz, 1H), 2.60 - 2.39 (m, 1H), 1.62 - 1.52 (m, 2H), 1.42 - 1.20 (m, 20H), 1.10 (t, *J* = 6.6 Hz, 6H), 1.02 (d, *J* = 6.7 Hz, 6H), 0.89 - 0.81 (m, 3H). ³¹P NMR (162 MHz, DMSO-*d*₆) δ 151.30, 148.65., ESI-MS: m/z 915.6 [M+H]⁺

### 2. Synthesis of compound 16

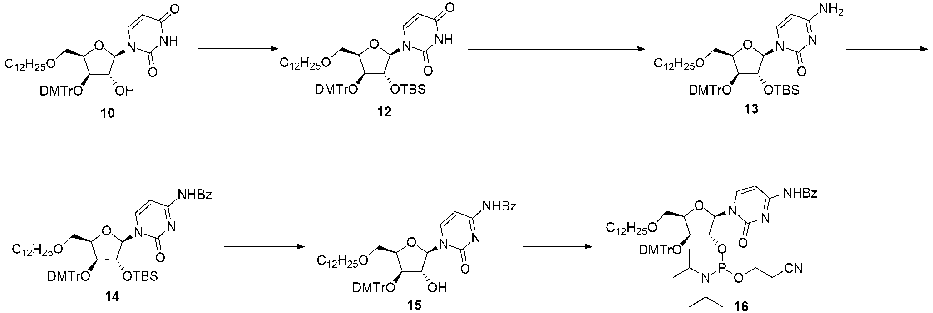

### 1) Synthesis of compound 12:

Compound 10 (2.6 g, 3.64 mmol, 1.0 eq.) was added to a 100 mL round-bottom flask, and extra dry DMF (30 mL) was added to the flask. The mixture was stirred for dissolution. Imidazole (991 mg, 14.56 mmol, 4.0 eq.) was added to the flask at room temperature, and the mixture was stirred for 10 min. Then, TBSCl (1.10 g, 7.28 mmol, 2.0 eq.) was slowly added in batches to the reaction system, and the mixture was reacted at room temperature overnight under a nitrogen atmosphere. The reaction of compound 10 was completed as determined by TLC and LCMS, and water was added to the mixture to quench the reaction. The mixed system was extracted twice with ethyl acetate, and the organic phases were combined. The organic phase was washed with water and saturated brine, dried over anhydrous sodium sulfate, and concentrated under reduced pressure to remove the solvent to obtain a crude product. The crude product was subjected to column chromatography (PE/EA = 2/1) to obtain compound 12 (2.75 g, 3.32 mmol, yield: 91.2%). ESI-MS: m/z 829.5 [M+H]⁺

### 2) Synthesis of compound 13:

Compound 12 (2.75 g, 3.32 mmol, 1.0 eq.) was added to a 250 mL round-bottom flask, and extra dry acetonitrile (30 mL) was added to the flask. The mixture was stirred until complete dissolution. Triethylamine (672 mg, 6.64 mmol, 2.0 eq.) and DMAP (811 mg, 6.64 mmol, 2.0 eq.) were added to the reaction system, and the mixture was stirred homogeneously. The reaction was cooled to 0-5 °C, and compound TPSCl (2.01 g, 6.64 mmol, 2.0 eq.) was slowly added in batches. After the addition was completed, the ice bath was removed, and the mixture was warmed to room temperature. The reaction was stirred at room temperature overnight. The reaction of compound 12 was completed as determined by TLC. Ammonia water (20 mL) was added to the reaction at room temperature, and the mixture was stirred for about 12 h until the reaction of the intermediate was completed. Saturated brine was added to the reaction, the mixed system was extracted twice with ethyl acetate, and the organic phases were combined. The organic phase was washed with water and saturated brine, dried over anhydrous sodium sulfate, and concentrated under reduced pressure to obtain crude product 13 (8.3 g, calculated as 100% yield). ESI-MS: m/z 828.6 [M+H]⁺

### 3) Synthesis of compound 14:

Crude compound 13 (8.3 g, 1.0 eq.) was added to a 100 mL round-bottom flask, and pyridine (50 mL) was added to the flask. The mixture was stirred until crude product 13 was completely dissolved. The reaction system was cooled to 0 °C, and BzCl (933 mg, 6.64 mmol, 2.0 eq.) was slowly added dropwise to the reaction system. The mixture was stirred at 0 °C for 1 h until the reaction of compound 13 was completed. The whole reaction was performed under a nitrogen atmosphere. The reaction system was warmed to room temperature, and methanol and water were added to quench the reaction. The mixed system was extracted twice with ethyl acetate, and the organic phases were combined. The organic phase was washed with water and saturated brine, dried over anhydrous sodium sulfate, and concentrated under reduced pressure to remove the solvent to obtain a crude compound. The crude product was subjected to column chromatography (PE/EA = 1/1) to obtain compound 14 (2.35 g, 2.52 mmol, overall yield over 2 steps: 76%). ESI-MS: m/z 931.5 [M+H]⁺

### 4) Synthesis of compound 15:

Compound 14 (2.35 g, 2.52 mmol, 1.0 eq.) was added to a 100 mL round-bottom flask, and THF (30 mL) was added. The mixture was stirred until complete dissolution. Triethylamine trihydrofluoride (5.0 mL) was neutralized to alkalinity with triethylamine (17 mL), and then the mixture was added to the reaction system described above. The reaction was placed in an oil bath at 40 °C and stirred overnight under a nitrogen atmosphere. The reaction of compound 14 was completed as determined by TLC and LCMS. Water was added to the reaction, the mixed system was extracted twice with ethyl acetate, and the organic phases were combined. The organic phase was washed with water, a saturated aqueous sodium bicarbonate solution, and saturated brine, dried over anhydrous sodium sulfate, and concentrated under reduced pressure to remove the solvent to obtain a crude product. The crude product was subjected to column chromatography (PE/EA = 1/1) to obtain compound 15 (1.57 g, 1.92 mmol, yield: 76.2%). ESI-MS: m/z 818.5 [M+H]⁺

### 5) Synthesis of compound 16:

Dry compound 15 (1.57 g, 1.92 mmol) was added to a 100 mL round-bottom flask, and extra dry dichloromethane (20 mL) was added to the flask. The mixture was stirred for dissolution. DIPEA (496 mg, 3.84 mmol, 2.0 eq.) and DMAP (47 mg, 0.38 mmol, 0.2 eq.) were added to the reaction, and the reaction was purged with nitrogen. The reaction was cooled to about 0 °C, and CEP-Cl (682 mg, 2.93 mmol, 1.5 eq.) was slowly added dropwise. The reaction was performed at this temperature for 1 h under a nitrogen atmosphere until the reaction of compound 15 was completed. After the reaction was completed, the reaction was quenched by adding a saturated aqueous sodium bicarbonate solution, and the mixed system was extracted twice with dichloromethane. The organic phases were combined. The organic phase was washed with water and saturated brine, dried over anhydrous sodium sulfate, and concentrated under reduced pressure to remove the solvent to obtain a crude product. The crude product was purified by column chromatography (PE/EA = 1/1) to obtain compound 16 (1.7 g, 1.67 mmol, yield: 87%). ¹H NMR (400 MHz, DMSO-*d*₆) δ 11.27 (d, *J =* 17.5 Hz, 1H), 8.46 (dd, *J =* 30.3, 7.5 Hz, 1H), 8.01 (d, *J* = 7.6 Hz, 2H), 7.62 (t, *J* = 7.3 Hz, 1H), 7.55 - 7.44 (m, 2H), 7.39 - 7.13 (m, 9H), 6.87 - 6.82 (m, 4H), 5.71 (d, *J* = 25.0 Hz, 1H), 4.23 - 3.90 (m, 3H), 3.78 - 3.38 (m, 13H), 2.74 (dd, *J* = 10.7, 5.6 Hz, 1H), 2.61 - 2.32 (m, 1H), 1.61 - 1.50 (m, 2H), 1.38 - 1.19 (m, 20H), 1.12 - 1.04 (m, 9H), 0.96 (d, *J* = 6.7 Hz, 3H), 0.83 (t, *J* = 6.6 Hz, 3H), ³¹P NMR (162 MHz, DMSO-*d*₆) δ 149.87, 149.34, ESI-MS: m/z 1018.6 [M+H]⁺

### 3. Synthesis of compound 21

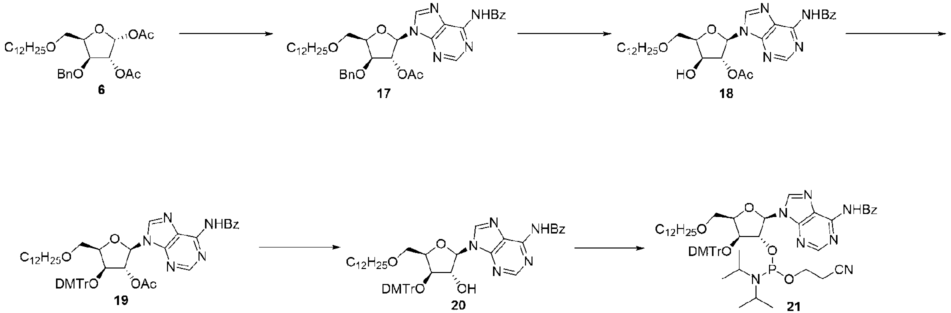

### 1) Synthesis of compound 17:

Compound 6 (27 g, 54.81 mmol, 1.00 eq.) was added to a 500 mL three-necked round-bottom flask, and extra dry acetonitrile (250 mL) was added to the reaction flask. The mixture was stirred for dissolution, and then BSA (33.45 g, 164.43 mmol, 3.0 eq.) and ABz (26.22 g, 109.62 mmol, 2.0 eq.) were added. The reaction system was placed in an oil bath, heated to 80 °C, and stirred at this temperature for 1 h. The entire reaction was performed under a nitrogen atmosphere. After the reaction was completed, the reaction system was placed in an ice-water bath at 0 °C and stirred for 30 min, and TMSOTf (12.18 g, 54.81 mmol, 1.0 eq.) was slowly added dropwise to the reaction system. After the addition was completed, the reaction was placed in an oil bath and slowly heated to 80 °C, and the reaction was performed at this temperature overnight. The reaction of compound 6 was completed as determined by TLC and LCMS. The reaction was removed from the oil bath and cooled to room temperature. A saturated aqueous sodium bicarbonate solution was added to the reaction system to quench the reaction, the system was extracted with ethyl acetate, and the organic phases were combined. The organic phase was washed with water and saturated brine, dried over anhydrous sodium sulfate, and concentrated under reduced pressure to remove the solvent to obtain crude product 17. The crude product was subjected to column chromatography (PE/EA = 1/1) to obtain compound 17 (25 g, 37.23 mmol, yield: 67.9%).

### 2) Synthesis of compound 18:

Compound 17 (3.0 g, 4.47 mmol, 1.0 eq.) was added to a 250 mL three-necked flask, and solvent extra dry dichloromethane (30 mL) was added to the reaction flask. The reaction system was cooled to - 10 °C and stirred at this temperature for 30 min. A 1.0 mol/L solution of boron trichloride in dichloromethane (13.4 mL, 3.0 eq) was slowly added dropwise to the reaction system, and the reaction was performed for another 5 h until the reaction of compound 17 was completed. The reaction was quenched with triethylamine and methanol at -20 °C. The reaction was warmed to room temperature, and water was added. The mixed system was extracted with ethyl acetate, and the organic phases were combined. The organic phase was washed with water and saturated brine, dried over anhydrous sodium sulfate, and concentrated under reduced pressure to remove the solvent to obtain crude product 18. The crude compound was subjected to column chromatography (PE/EA = 1/2) to obtain compound 18 (1.0 g, 1.72 mmol, yield: 38.5%).

### 3) Synthesis of compound 19:

Compound 18 (1.0 g, 1.72 mmol, 1.0 eq.) was added to a 100 mL round-bottom flask, and extra dry DCE (15 mL) was added to the reaction flask. The mixture was stirred until complete dissolution. DMTrCl (2.9 g, 8.6 mmol, 5.0 eq.), silver nitrate (0.29 g, 1.72 mmol, 1.0 eq.), and 2,4,6-trimethylpyridine (2.08 g, 17.2 mmol, 10.0 eq.) were added to the reaction at room temperature, and the mixture was stirred homogeneously at room temperature. The reaction was placed in an oil bath and heated to 80 °C, and the reaction was performed overnight. The reaction of compound 18 was completed as determined by TLC and LCMS. The reaction was cooled to room temperature and quenched with the addition of methanol. Ethyl acetate was added to the reaction for dilution, and the mixture was filtered through celite to obtain a filtrate. The filter cake was washed twice with ethyl acetate. The filtrates were combined and concentrated under reduced pressure to obtain crude compound 19. The crude product was subjected to column chromatography (PE/EA = 2/3) to obtain compound 19 (1.2 g, 1.36 mmol, yield: 72%). ESI-MS: m/z 884.5 [M+H]⁺

### 4) Synthesis of compound 20:

Compound 19 (1.2 g, 1.36 mmol, 1.0 eq.) was added to a 100 mL round-bottom flask, and THF (10 mL) was added to the reaction. The reaction was stirred until complete dissolution. A 5.4 mol/L sodium methoxide solution (0.76 mL, 4.08 mmol, 3.0 eq.) was added to the reaction. The reaction was stirred at room temperature for 3 h until the reaction of compound 19 was completed. After the reaction was completed, the reaction system was concentrated under reduced pressure at 40 °C to obtain crude product 20. The crude product was subjected to column chromatography to obtain compound 20 (0.82 g, 0.97 mmol, yield: 73%). ESI-MS: m/z 842.4 [M+H]⁺

### 5) Synthesis of compound 21:

Dry compound 20 (0.82 g, 0.97 mmol, 1.0 eq.) was added to a 50 mL round-bottom flask, and extra dry dichloromethane (10 mL) was added to the flask. The mixture was stirred until complete dissolution. DIPEA (0.25 g, 1.94 mmol, 2.0 eq.) and DMAP (23.8 mg, 0.194 mmol, 0.2 eq.) were added to the reaction, and the mixture was stirred at room temperature for 15 min. The reaction system was purged with nitrogen and performed under a nitrogen atmosphere. Compound CEP-Cl (0.35 g, 1.46 mmol, 1.5 eq.) was added dropwise to the reaction at room temperature. The reaction was performed at room temperature for 30-60 min until the reaction of compound 20 was completed. The reaction system was quenched by adding a saturated aqueous sodium bicarbonate solution and extracted twice with dichloromethane. The organic phases were combined, washed with water and saturated brine, dried over anhydrous sodium sulfate, and concentrated under reduced pressure to remove the solvent to obtain crude product 21. The crude product was purified by column chromatography to obtain compound 21 (0.74 g, 0.71 mmol, yield: 73.2%). ¹H NMR (400 MHz, DMSO-*d*₆) δ 11.21 (d, *J* = 4.0 Hz, 1H), 8.71 (d, *J* = 5.9 Hz, 1H), 8.60 (d, *J* = 29.9 Hz, 1H), 8.04 (d, *J* = 7.6 Hz, 2H), 7.65 (t, *J* = 7.3 Hz, 1H), 7.57 (t, *J* = 7.6 Hz, 2H), 7.35 (t, *J* = 8.6 Hz, 2H), 7.21 - 7.12 (m, 7H), 6.83 - 6.71 (m, 4H), 6.02 (dd, *J* = 36.3, 3.4 Hz, 1H), 4.78 - 4.31 (m, 2H), 3.81 - 3.36 (m, 15H), 2.67 - 2.38 (m, 2H), 1.62 - 1.48 (m, 2H), 1.42 - 1.21 (m, 18H), 1.12 - 0.99 (m, 9H), 0.93 - 0.78 (m, 6H). ESI-MS: m/z 1042.6 [M+H]⁺

### 4. Synthesis of compound 26

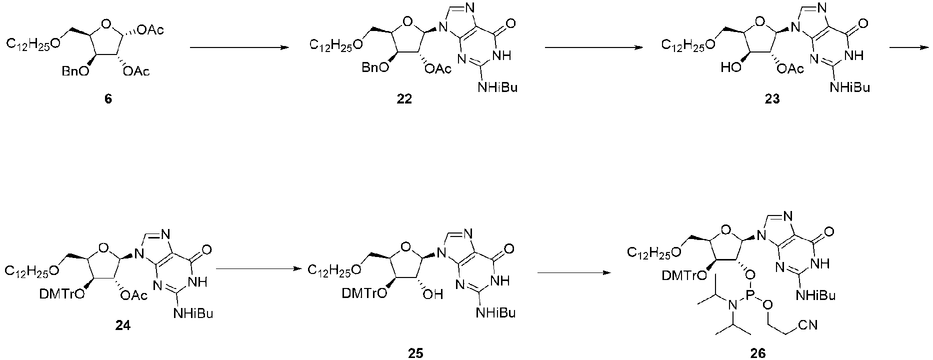

### 1) Synthesis of compound 22:

Compound 6 (20 g, 40.60 mmol, 1.00 eq.) was added to a 500 mL three-necked round-bottom flask, and extra dry acetonitrile (200 mL) was added to the reaction flask. The mixture was stirred for dissolution, and then BSA (24.78 g, 121.8 mmol, 3.0 eq.) and GiBu (17.96 g, 81.2 mmol, 2.0 eq.) were added. The reaction system was placed in an oil bath, heated to 80 °C, and stirred at this temperature for 1 h. The entire reaction was performed under a nitrogen atmosphere. After the reaction was completed, the reaction system was placed in an ice-water bath at 0 °C and stirred for 30 min, and TMSOTf (9.02 g, 40.6 mmol, 1.0 eq.) was slowly added dropwise to the reaction system. After the addition was completed, the reaction was placed in an oil bath and slowly heated to 80 °C, and the reaction was performed at this temperature overnight. The reaction of compound 6 was completed as determined by TLC and LCMS. The reaction was removed from the oil bath and cooled to room temperature. A saturated aqueous sodium bicarbonate solution was added to the reaction system to quench the reaction, the system was extracted with ethyl acetate, and the organic phases were combined. The organic phase was washed with water and saturated brine, dried over anhydrous sodium sulfate, and concentrated under reduced pressure to remove the solvent to obtain crude product 22. The crude product was subjected to column chromatography (PE/EA = 1/1) to obtain compound 22 (18 g, 27.53 mmol, yield: 67.8%). ESI-MS: m/z 654.5 [M+H]⁺

### 2) Synthesis of compound 23:

Compound 22 (5.0 g, 7.64 mmol, 1.0 eq.) was added to a 250 mL three-necked flask, and solvent extra dry dichloromethane (50 mL) was added to the reaction flask. The reaction system was cooled to - 20 °C and stirred at this temperature for 30 min. A 1.0 mol/L solution of boron trichloride in dichloromethane (22.92 mL, 3.0 eq) was slowly added dropwise to the reaction system, and the reaction was performed for another 5 h until the reaction of compound 22 was completed. The reaction was quenched with triethylamine and methanol at -20 °C. The reaction was warmed to room temperature, and water was added. The mixed system was extracted with ethyl acetate, and the organic phases were combined. The organic phase was washed with water and saturated brine, dried over anhydrous sodium sulfate, and concentrated under reduced pressure to remove the solvent to obtain crude product 23. The crude compound was subjected to column chromatography (PE/EA = 1/2) to obtain compound 23 (2.6 g, 4.61 mmol, yield: 60.3%). ESI-MS: m/z 564.3 [M+H]⁺

### 3) Synthesis of compound 24:

Compound 23 (2.6 g, 4.61 mmol, 1.0 eq.) was added to a 250 mL round-bottom flask, and extra dry DCE (35 mL) was added to the reaction flask. The mixture was stirred until complete dissolution. DMTrCl (7.8 g, 23.05 mmol, 5.0 eq.), silver nitrate (0.8 g, 4.61 mmol, 1.0 eq.), and 2,4,6-trimethylpyridine (5.59 g, 46.1 mmol, 10.0 eq.) were added to the reaction at room temperature, and the mixture was stirred homogeneously at room temperature. The reaction was placed in an oil bath and heated to 80 °C, and the reaction was performed overnight. The reaction of compound 23 was completed as determined by TLC and LCMS. The reaction was cooled to room temperature and quenched with the addition of methanol. Ethyl acetate was added to the reaction for dilution, and the mixture was filtered through celite to obtain a filtrate. The filter cake was washed twice with ethyl acetate. The filtrates were combined and concentrated under reduced pressure to obtain crude compound 24. The crude product was subjected to column chromatography (PE/EA = 2/3) to obtain compound 24 (3.18 g, 3.67 mmol, yield: 79.6%). ESI-MS: m/z 866.6 [M+H]⁺

### 4) Synthesis of compound 25:

Compound 24 (3.18 g, 3.67 mmol, 1.0 eq.) was added to a 100 mL round-bottom flask, and THF (30 mL) was added to the reaction. The reaction was stirred until complete dissolution. A 5.4 mol/L sodium methoxide solution (2.04 mL, 11.01 mmol, 3.0 eq.) was added to the reaction. The reaction was stirred at room temperature for 3 h until the reaction of compound 24 was completed. After the reaction was completed, the reaction system was concentrated under reduced pressure at 40 °C to obtain crude product 25. The crude product was subjected to column chromatography to obtain compound 25 (2.48 g, 3.01 mmol, yield: 82.0%). ESI-MS: m/z 824.5 [M+H]+

### 5) Synthesis of compound 26:

Dry compound 25 (2.0 g, 2.43 mmol, 1.0 eq.) was added to a 50 mL round-bottom flask, and extra dry dichloromethane (30 mL) was added to the flask. The mixture was stirred until complete dissolution. DIPEA (0.63 g, 4.86 mmol, 2.0 eq.) and DMAP (59.4 mg, 0.486 mmol, 0.2 eq.) were added to the reaction, and the mixture was stirred at room temperature for 15 min. The reaction system was purged with nitrogen and performed under a nitrogen atmosphere. Compound CEP-Cl (0.86 g, 3.65 mmol, 1.5 eq.) was added dropwise to the reaction at room temperature. The reaction was performed at room temperature for 30-60 min until the reaction of compound 25 was completed. The reaction system was quenched by adding a saturated aqueous sodium bicarbonate solution and extracted twice with dichloromethane. The organic phases were combined, washed with water and saturated brine, dried over anhydrous sodium sulfate, and concentrated under reduced pressure to remove the solvent to obtain crude product 26. The crude product was purified by column chromatography to obtain compound 26 (2.08 g, 2.06 mmol, yield: 83.5%). ¹H NMR (400 MHz, DMSO-*d*₆) *δ* 12.08 (s, 1H), 11.48 (s, 1H), 8.18 (d, *J* = 29.6 Hz, 1H), 7.52 - 7.11 (m, 9H), 6.93 - 6.84 (m, 4H), 5.72 (dd, *J* = 38.6, 4.8 Hz, 1H), 5.21 - 5.04 (m, 1H), 4.53 (t, *J* = 5.5 Hz, 1H), 3.81 - 3.69 (m, 8H), 3.61 - 3.34 (m, 3H), 3.30 - 3.23 (m, 2H), 3.11 (d, *J* = 8.4 Hz, 1H), 2.79 - 2.64 (m, 1H), 2.59 - 2.48 (m, 2H), 2.46 - 2.35 (m, 1H), 1.51 (d, *J* = 6.6 Hz, 2H), 1.38 - 1.18 (m, 18H), 1.13 - 0.98 (m, 13H), 0.88 - 0.74 (m, 8H).³¹P NMR (162 MHz, DMSO-*d*₆) δ 151.94, 148.86, ESI-MS: m/z 1024.6 [M+H]⁺

### 5. Synthesis of compound 27

Nucleoside compound 27 is a TNA structural compound with a 2'-phosphoramidite group, 3'-ODMTr, Base = U, and R = C₁₄H₂₉. The preparation route referred to that of compound 11, except that in the step wherein intermediate compound 4 was used to synthesize compound 5, starting material C₁₂H₂₅Br (bromododecane) was replaced with C₁₄H₂₉Br. The remaining steps were similar to those for compound 11. The structure and nuclear magnetic resonance data of the obtained nucleoside compound 27 are shown below:

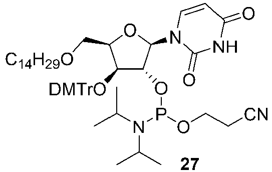

¹H NMR (400 MHz, DMSO-*d*₆) δ 11.35 (dd, *J* = 14.6, 2.2 Hz, 1H), 7.93 (d, *J* = 8.1 Hz, 1H), 7.47 - 7.36 (m, 2H), 7.33 - 7.23 (m, 7H), 6.89 - 6.83 (m, 4H), 5.71 - 5.51 (m, 2H), 4.34 (dt, *J* = 8.8, 4.7 Hz, 1H), 4.11 - 3.90 (m, 1H), 3.84 - 3.76 (m, 1H), 3.75 (s, 6H), 3.69 - 3.36 (m, 6H), 2.71 (t, *J* = 6.0 Hz, 1H), 2.60 - 2.39 (m, 1H), 1.62 - 1.52 (m, 2H), 1.42 - 1.20 (m, 24H), 1.10 (t, *J* = 6.6 Hz, 6H), 1.02 (d, *J* = 6.7 Hz, 6H), 0.89 - 0.81 (m, 3H). ³¹P NMR (162 MHz, DMSO-*d*₆) δ 151.32, 148.63., ESI-MS: m/z 943.3 [M+H]⁺

### 6. Synthesis of compound 28

Nucleoside compound 28 is a TNA structural compound with a 2'-phosphoramidite group, 3'-ODMTr, Nu = U, and R = C₁₆H₃₃. The preparation route referred to that of compound 11, except that in the step wherein intermediate compound 4 was used to synthesize compound 5, starting material C₁₂H₂₅Br was replaced with C₁₆H₃₃Br. The remaining steps were similar to those for compound 11. The structure and nuclear magnetic resonance data of the obtained nucleoside compound 28 are shown below:

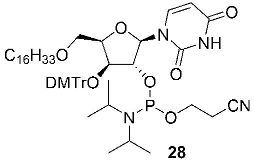

¹H NMR (400 MHz, DMSO-*d*₆) δ 11.35 (dd, *J* = 14.6, 2.2 Hz, 1H), 7.93 (d, *J* = 8.1 Hz, 1H), 7.47 - 7.36 (m, 2H), 7.33 - 7.23 (m, 7H), 6.89 - 6.83 (m, 4H), 5.71 - 5.51 (m, 2H), 4.34 (dt, *J* = 8.8, 4.7 Hz, 1H), 4.11 - 3.90 (m, 1H), 3.84 - 3.76 (m, 1H), 3.75 (s, 6H), 3.69 - 3.36 (m, 6H), 2.71 (t, *J* = 6.0 Hz, 1H), 2.60 - 2.39 (m, 1H), 1.62 - 1.52 (m, 2H), 1.42 - 1.20 (m, 28H), 1.10 (t, *J* = 6.6 Hz, 6H), 1.02 (d, *J* = 6.7 Hz, 6H), 0.89 - 0.81 (m, 3H). ³¹P NMR (162 MHz, DMSO-*d*₆) δ 151.35, 148.66., ESI-MS: m/z 971.5 [M+H]⁺

### 7. Synthesis of compound 29

Nucleoside compound 29 is a TNA structural compound with a 2'-phosphoramidite group, 3'-ODMTr, Nu = C, and R = C₁₄H₂₉. The preparation route referred to that of compound 16, except that R = C₁₂H₂₅ in the starting compound 10 was replaced with C₁₄H₂₉. The remaining steps were similar to those for compound 16. The structure and nuclear magnetic resonance data of the obtained nucleoside compound 29 are shown below:

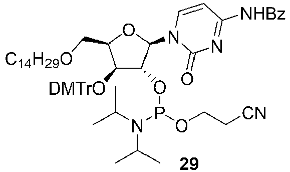

¹H NMR (400 MHz, DMSO-*d₆*) δ 11.27 (d, J = 17.5 Hz, 1H), 8.46 (dd, J = 30.3, 7.5 Hz, 1H), 8.01 (d, J = 7.6 Hz, 2H), 7.62 (t, J = 7.3 Hz, 1H), 7.55 - 7.44 (m, 2H), 7.39 - 7.13 (m, 9H), 6.87 - 6.82 (m, 4H), 5.71 (d, J = 25.0 Hz, 1H), 4.23 - 3.90 (m, 3H), 3.78 - 3.38 (m, 13H), 2.74 (dd, J = 10.7, 5.6 Hz, 1H), 2.61 - 2.32 (m, 1H), 1.61 - 1.50 (m, 2H), 1.38 - 1.19 (m, 24H), 1.12 - 1.04 (m, 9H), 0.96 (d, J = 6.7 Hz, 3H), 0.83 (t, J = 6.6 Hz, 3H), ³¹P NMR (162 MHz, DMSO-d6) δ 149.82, 149.30, ESI-MS: m/z 1045.6 [M+H]⁺

### 8. Synthesis of compound 30

Nucleoside compound 30 is a TNA structural compound with a 2'-phosphoramidite group, 3'-ODMTr, Nu = C, and R = C₁₆H₃₃. The preparation route referred to that of compound 16, except that R = C₁₂H₃₃ in the starting compound 10 was replaced with C₁₆H₂₉. The remaining steps were similar to those for compound 16. The structure and nuclear magnetic resonance data of the obtained nucleoside compound 30 are shown below:

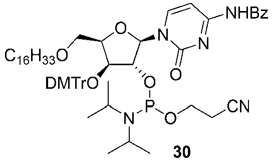

¹H NMR (400 MHz, DMSO-*d₆*) δ 11.27 (d, J = 17.5 Hz, 1H), 8.46 (dd, J = 30.3, 7.5 Hz, 1H), 8.01 (d, J = 7.6 Hz, 2H), 7.62 (t, J = 7.3 Hz, 1H), 7.55 - 7.44 (m, 2H), 7.39 - 7.13 (m, 9H), 6.87 - 6.82 (m, 4H), 5.71 (d, J = 25.0 Hz, 1H), 4.23 - 3.90 (m, 3H), 3.78 - 3.38 (m, 13H), 2.74 (dd, J = 10.7, 5.6 Hz, 1H), 2.61 - 2.32 (m, 1H), 1.61 - 1.50 (m, 2H), 1.38 - 1.19 (m, 28H), 1.12 - 1.04 (m, 9H), 0.96 (d, J = 6.7 Hz, 3H), 0.83 (t, J = 6.6 Hz, 3H), ³¹P NMR (162 MHz, DMSO-*d*₆) δ 149.79, 149.31, ESI-MS: m/z 1073.6 [M+H]⁺

### 9. Synthesis of compound 31

Nucleoside compound 31 is a TNA structural compound with a 2'-phosphoramidite group, 3'-ODMTr, Nu = A, and R = C₁₄H₃₃. The preparation route referred to that of compound 21, except that R = C₁₂H₃₃ in the starting compound 6 was replaced with C₁₄H₂₉. The remaining steps were similar to those for compound 21. The structure and nuclear magnetic resonance data of the obtained nucleoside compound 31 are shown below:

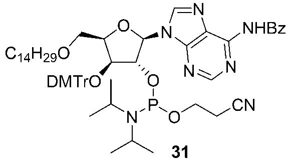

¹H NMR (400 MHz, DMSO-*d*₆) *δ* 11.26 (d, *J =* 4.0 Hz, 1H), 8.74 (d, *J =* 5.9 Hz, 1H), 8.56 (d, *J =* 29.9 Hz, 1H), 8.24 (d, J= 7.6 Hz, 2H), 7.65 (t, J= 7.3 Hz, 1H), 7.53 (t, J= 7.6 Hz, 2H), 7.36 (t, J= 8.6 Hz, 2H), 7.24 - 7.12 (m, 7H), 6.80 - 6.61 (m, 4H), 6.03 (dd, *J =* 36.3, 3.4 Hz, 1H), 4.74 - 4.32 (m, 2H), 3.82 - 3.39 (m, 15H), 2.67 - 2.35 (m, 2H), 1.61 - 1.49 (m, 2H), 1.41 - 1.20 (m, 22H), 1.03 - 0.96 (m, 9H), 0.90 - 0.76 (m, 6H). ³¹P NMR (162 MHz, DMSO-*d*₆) δ 149.66, 148.93. ESI-MS: m/z 1070.5 [M+H]⁺

### 10. Synthesis of compound 32

Nucleoside compound 32 is a TNA structural compound with a 2'-phosphoramidite group, 3'-ODMTr, Nu = A, and R = C₁₆H₃₃. The preparation route referred to that of compound 21, except that R = C₁₂H₃₃ in the starting compound 6 was replaced with C₁₆H₂₉. The remaining steps were similar to those for compound 21. The structure and nuclear magnetic resonance data of the obtained nucleoside compound 32 are shown below:

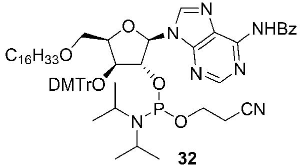

¹H NMR (400 MHz, DMSO-*d*₆) *δ* 11.19 (d, *J =* 4.0 Hz, 1H), 8.71 (d, *J =* 5.9 Hz, 1H), 8.49 (d, *J* = 29.9 Hz, 1H), 8.27 (d, *J* = 7.6 Hz, 2H), 7.35 (t, *J* = 7.3 Hz, 1H), 7.55 (t, *J* = 7.6 Hz, 2H), 7.37 (t, *J* = 8.6 Hz, 2H), 7.25 - 7.17 (m, 7H), 6.81 - 6.60 (m, 4H), 6.05 (dd, *J* = 36.3, 3.4 Hz, 1H), 4.75 - 4.33 (m, 2H), 3.89 - 3.37 (m, 15H), 2.69 - 2.32 (m, 2H), 1.62 - 1.45 (m, 2H), 1.43 - 1.22 (m, 26H), 1.13 - 0.79 (m, 9H), 0.91 - 0.77 (m, 6H). ³¹P NMR (162 MHz, DMSO-*d*₆) δ 149.29, 148.76. ESI-MS: m/z 1098.4 [M+H]⁺

### 11. Synthesis of compound 33

Nucleoside compound 33 is a TNA structural compound with a 2'-phosphoramidite group, 3'-ODMTr, Nu = G, and R = C₁₄H₃₃. The preparation route referred to that of compound 21, except that R = C₁₂H₃₃ in the starting compound 6 was replaced with C₁₄H₂₉. The remaining steps were similar to those for compound 21. The structure and nuclear magnetic resonance data of the obtained nucleoside compound 33 are shown below:

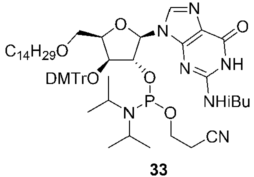

¹H NMR (400 MHz, DMSO-*d₆*) δ 12.08 (s, 1H), 11.48 (s, 1H), 8.18 (d, J = 29.6 Hz, 1H), 7.52 - 7.11 (m, 9H), 6.93 - 6.84 (m, 4H), 5.72 (dd, J = 38.6, 4.8 Hz, 1H), 5.21 - 5.04 (m, 1H), 4.53 (t, J = 5.5 Hz, 1H), 3.81 - 3.69 (m, 8H), 3.61 - 3.34 (m, 3H), 3.30 - 3.23 (m, 2H), 3.11 (d, J = 8.4 Hz, 1H), 2.79 - 2.64 (m, 1H), 2.59 - 2.48 (m, 2H), 2.46 - 2.35 (m, 1H), 1.51 (d, J = 6.6 Hz, 2H), 1.38 - 1.18 (m, 22H), 1.13 - 0.98 (m, 13H), 0.88 - 0.74 (m, 8H).³¹P NMR (162 MHz, DMSO-*d₆*) δ 151.92, 148.83, ESI-MS: m/z 1038.3 [M+H]⁺

### 12. Synthesis of compound 34

Nucleoside compound 34 is a TNA structural compound with a 2'-phosphoramidite group, 3'-ODMTr, Nu = G, and R = C₁₆H₃₃. The preparation route referred to that of compound 21, except that R = C₁₂H₃₃ in the starting compound 6 was replaced with C₁₆H₂₉. The remaining steps were similar to those for compound 21. The structure and nuclear magnetic resonance data of the obtained nucleoside compound 34 are shown below:

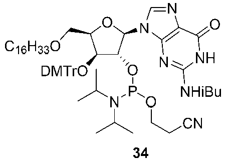

¹H NMR (400 MHz, DMSO-*d₆*) δ 12.08 (s, 1H), 11.48 (s, 1H), 8.18 (d, J = 29.6 Hz, 1H), 7.52 - 7.11 (m, 9H), 6.93 - 6.84 (m, 4H), 5.72 (dd, J = 38.6, 4.8 Hz, 1H), 5.21 - 5.04 (m, 1H), 4.53 (t, J = 5.5 Hz, 1H), 3.81 - 3.69 (m, 8H), 3.61 - 3.34 (m, 3H), 3.30 - 3.23 (m, 2H), 3.11 (d, J = 8.4 Hz, 1H), 2.79 - 2.64 (m, 1H), 2.59 - 2.48 (m, 2H), 2.46 - 2.35 (m, 1H), 1.51 (d, J = 6.6 Hz, 2H), 1.38 - 1.18 (m, 22H), 1.13 - 0.98 (m, 13H), 0.88 - 0.74 (m, 8H).³¹P NMR (162 MHz, DMSO-*d₆*) δ 151.96, 148.87, ESI-MS: m/z 1066.5 [M+H]⁺

### Example 4: Preparation of GalNAc-siRNA Conjugates

1. The synthesis process of the GalNAc-siRNA conjugate was as follows: 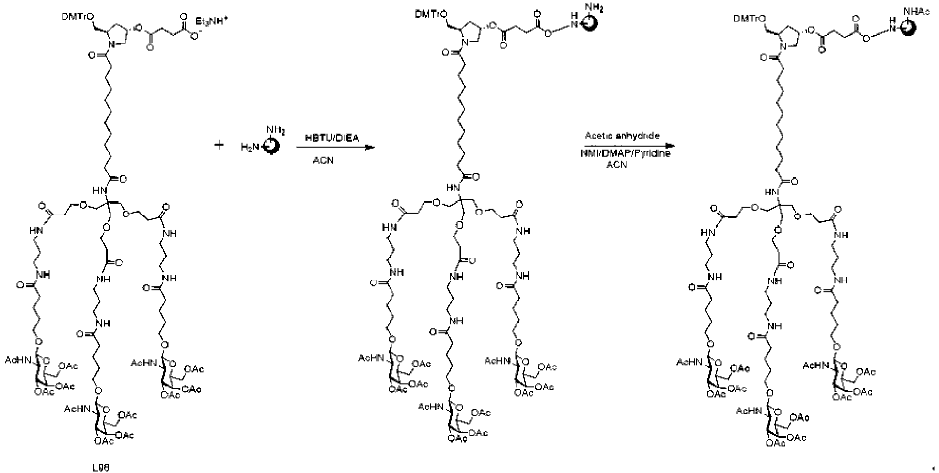 According to the process steps described above, L96 (GalNAc₃) and an amino support (polystyrene resin or CPG powder) were subjected to a coupling reaction and a capping reaction to prepare a solid-phase synthesis support preloaded with L96. The support loading was determined by analysis.
2. The solid-phase synthesis support described above was loaded into a solid-phase reaction synthesis column, and a nucleic acid synthesizer was loaded. According to the standard oligonucleotide solid-phase synthesis method, the modified nucleotide monomers were used to synthesize the oligonucleotide sequence coupled to L96 (modified SS strand) by four cycles of the de-dimethoxytritylation reaction, coupling reaction, oxidation reaction, and capping reaction as in Example 1. If the target product had a phosphorothioate modification, a thiation reaction was used instead of the oxidation reaction; that is, the oxidation reaction in c in Example 1 was replaced with the thiation reaction: under the action of a thiation reagent PADS/ADTT, the phosphite triester generated by the coupling and condensation in the previous step was converted into stable phosphorothioate triester. The desired sequence was synthesized, and the cyanoethyl protecting group was removed with 20% diethylamine. The solid-phase support was blow-dried with argon and then subjected to ammonolysis and deprotection in concentrated ammonia water at 55 °C for 5-16 h. After the ammonolysis and deprotection were completed, the support was removed by filtration; the ammonolysis solution was concentrated to remove ammonia water, and the remaining concentrated sample was sent for LC-MS analysis. It was determined that the molecular weight of the sample was consistent with the theoretical molecular weight.
3. The L96-coupled oligonucleotide sample (with L96 located at the 3' end of the modified SS strand) was purified on AKTA Pure 150 by an ion exchange method, and desalted with a gel column to prepare a single-stranded sample that met the requirements.
4. The antisense strand (modified AS strand) was synthesized using a universal solid-phase synthesis support Nitto Phase HLUny Linker according to a standard solid-phase synthesis method.
5. The modified SS strand and the modified AS strand were added to an annealing vessel in an equimolar ratio, heated to 55 °C, held for about 30 min, and then naturally cooled to room temperature.
6. The annealed double-stranded sample was aliquoted into lyophilization containers according to the required amount and lyophilized to obtain the GalNAc-siRNA conjugate.

The structure of the GalNAc-siRNA conjugate is shown in formula (B-III), and in each GalNAc-siRNA, the 3' end of the sense strand is coupled with L96: 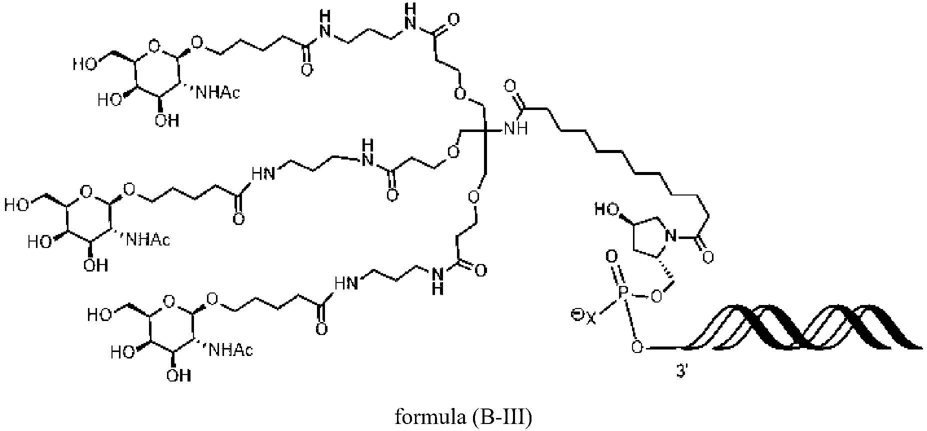 wherein 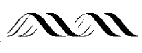 represents a double-stranded oligonucleotide of the RNAi, 3' represents the 3' end of the sense strand, and X is O or S.

The sense strands and antisense strands contained in the GalNAc-siRNA conjugates are shown in Table 5. Each conjugate was analyzed by LC-MS to confirm identity and quantified by UV (260 nm), and the purity was determined by the HPLC analysis.

**Table 5. Sense strands and antisense strands contained in conjugates**

| Conjugate No. | Sense strand (SEQ ID NO) | Coupling of 3' end of sense strand to L96 | Antisense strand (SEQ ID NO) |
|---|---|---|---|
| BPR-30203301 | 471 | Yes | 581 |
| BPR-30203302 | 472 | Yes | 581 |
| BPR-30203303 | 471 | Yes | 594 |
| BPR-30203304 | 471 | Yes | 596 |
| BPR-30203305 | 471 | Yes | 595 |
| BPR-30203306 | 471 | Yes | 588 |
| BPR-30203307 | 471 | Yes | 585 |
| BPR-30203308 | 471 | Yes | 593 |
| BPR-30203309 | 471 | Yes | 587 |
| BPR-30203310 | 471 | Yes | 576 |
| BPR-30203311 | 471 | Yes | 577 |
| BPR-30203312 | 473 | Yes | 581 |
| BPR-30203313 | 476 | Yes | 581 |
| BPR-30203314 | 475 | Yes | 581 |
| BPR-30203315 | 474 | Yes | 581 |
| BPR-30203316 | 473 | Yes | 578 |
| BPR-30203317 | 476 | Yes | 578 |
| BPR-30203318 | 475 | Yes | 578 |
| BPR-30203319 | 474 | Yes | 578 |
| BPR-30203320 | 473 | Yes | 580 |
| BPR-30203321 | 476 | Yes | 580 |
| BPR-30203322 | 475 | Yes | 580 |
| BPR-30203323 | 474 | Yes | 580 |
| BPR-30203324 | 473 | Yes | 579 |
| BPR-30203325 | 476 | Yes | 579 |
| BPR-30203326 | 475 | Yes | 579 |
| BPR-30203327 | 474 | Yes | 579 |
| BPR-30203328 | 473 | Yes | 588 |
| BPR-30203329 | 476 | Yes | 591 |
| BPR-30203330 | 476 | Yes | 590 |
| BPR-30203331 | 476 | Yes | 589 |
| BPR-30203332 | 475 | Yes | 591 |
| BPR-30203333 | 475 | Yes | 590 |
| BPR-30203334 | 475 | Yes | 589 |
| BPR-30203335 | 474 | Yes | 591 |
| BPR-30203336 | 474 | Yes | 590 |
| BPR-30203337 | 474 | Yes | 589 |
| BPR-30203338 | 473 | Yes | 585 |
| BPR-30203339 | 476 | Yes | 584 |
| BPR-30203340 | 476 | Yes | 583 |
| BPR-30203341 | 476 | Yes | 582 |
| BPR-30203342 | 475 | Yes | 584 |
| BPR-30203343 | 475 | Yes | 583 |
| BPR-30203344 | 475 | Yes | 582 |
| BPR-30203345 | 474 | Yes | 584 |
| BPR-30203346 | 474 | Yes | 583 |
| BPR-30203347 | 474 | Yes | 582 |
| BPR-30203348 | 473 | Yes | 593 |
| BPR-30203349 | 476 | Yes | 593 |
| BPR-30203350 | 475 | Yes | 593 |
| BPR-30203351 | 474 | Yes | 593 |
| BPR-30203352 | 473 | Yes | 575 |
| BPR-30203353 | 476 | Yes | 575 |
| BPR-30203354 | 475 | Yes | 575 |
| BPR-30203355 | 474 | Yes | 575 |
| BPR-30203356 | 473 | Yes | 587 |
| BPR-30203357 | 476 | Yes | 587 |
| BPR-30203358 | 475 | Yes | 587 |
| BPR-30203359 | 474 | Yes | 587 |
| BPR-30203360 | 478 | Yes | 581 |
| BPR-30203361 | 478 | Yes | 576 |
| BPR-30203362 | 477 | Yes | 581 |
| BPR-30203363 | 471 | Yes | 586 |
| BPR-30203364 | 471 | Yes | 592 |
| BPR-30203365 | 471 | Yes | 591 |
| BPR-30203366 | 471 | Yes | 590 |
| BPR-30203367 | 471 | Yes | 589 |
| BPR-30203368 | 471 | Yes | 584 |
| BPR-30203369 | 471 | Yes | 583 |
| BPR-30203370 | 471 | Yes | 582 |
| BPR-30204201 | 480 | Yes | 609 |
| BPR-30204202 | 481 | Yes | 609 |
| BPR-30204203 | 480 | Yes | 623 |
| BPR-30204204 | 480 | Yes | 626 |
| BPR-30204205 | 480 | Yes | 624 |
| BPR-30204206 | 480 | Yes | 615 |
| BPR-30204207 | 480 | Yes | 613 |
| BPR-30204208 | 480 | Yes | 607 |
| BPR-30204209 | 480 | Yes | 608 |
| BPR-30204210 | 480 | Yes | 598 |
| BPR-30204211 | 480 | Yes | 599 |
| BPR-30204212 | 482 | Yes | 609 |
| BPR-30204213 | 485 | Yes | 609 |
| BPR-30204214 | 484 | Yes | 609 |
| BPR-30204215 | 483 | Yes | 609 |
| BPR-30204216 | 482 | Yes | 600 |
| BPR-30204217 | 485 | Yes | 600 |
| BPR-30204218 | 484 | Yes | 600 |
| BPR-30204219 | 483 | Yes | 600 |
| BPR-30204220 | 482 | Yes | 625 |
| BPR-30204221 | 485 | Yes | 625 |
| BPR-30204222 | 484 | Yes | 625 |
| BPR-30204223 | 483 | Yes | 625 |
| BPR-30204224 | 482 | Yes | 602 |
| BPR-30204225 | 485 | Yes | 602 |
| BPR-30204226 | 484 | Yes | 602 |
| BPR-30204227 | 483 | Yes | 602 |
| BPR-30204228 | 482 | Yes | 615 |
| BPR-30204229 | 485 | Yes | 618 |
| BPR-30204230 | 485 | Yes | 619 |
| BPR-30204231 | 485 | Yes | 616 |
| BPR-30204232 | 484 | Yes | 618 |
| BPR-30204233 | 484 | Yes | 619 |
| BPR-30204234 | 484 | Yes | 616 |
| BPR-30204235 | 483 | Yes | 618 |
| BPR-30204236 | 483 | Yes | 619 |
| BPR-30204237 | 483 | Yes | 616 |
| BPR-30204238 | 482 | Yes | 613 |
| BPR-30204239 | 485 | Yes | 612 |
| BPR-30204240 | 485 | Yes | 611 |
| BPR-30204241 | 485 | Yes | 610 |
| BPR-30204242 | 484 | Yes | 612 |
| BPR-30204243 | 484 | Yes | 611 |
| BPR-30204244 | 484 | Yes | 610 |
| BPR-30204245 | 483 | Yes | 612 |
| BPR-30204246 | 483 | Yes | 611 |
| BPR-30204247 | 483 | Yes | 610 |
| BPR-30204248 | 482 | Yes | 607 |
| BPR-30204249 | 485 | Yes | 607 |
| BPR-30204250 | 484 | Yes | 607 |
| BPR-30204251 | 483 | Yes | 607 |
| BPR-30204252 | 482 | Yes | 627 |
| BPR-30204253 | 485 | Yes | 627 |
| BPR-30204254 | 484 | Yes | 627 |
| BPR-30204255 | 483 | Yes | 627 |
| BPR-30204256 | 482 | Yes | 608 |
| BPR-30204257 | 485 | Yes | 608 |
| BPR-30204258 | 484 | Yes | 608 |
| BPR-30204259 | 483 | Yes | 608 |
| BPR-30204260 | 479 | Yes | 609 |
| BPR-30204261 | 479 | Yes | 598 |
| BPR-30204262 | 486 | Yes | 609 |
| BPR-30204263 | 480 | Yes | 614 |
| BPR-30204264 | 480 | Yes | 622 |
| BPR-30204265 | 480 | Yes | 618 |
| BPR-30204266 | 480 | Yes | 619 |
| BPR-30204267 | 480 | Yes | 616 |
| BPR-30204268 | 480 | Yes | 612 |
| BPR-30204269 | 480 | Yes | 611 |
| BPR-30204270 | 480 | Yes | 610 |
| BPR-30204501 | 490 | Yes | 636 |
| BPR-30204502 | 489 | Yes | 636 |
| BPR-30204503 | 490 | Yes | 649 |
| BPR-30204504 | 490 | Yes | 650 |
| BPR-30204505 | 490 | Yes | 651 |
| BPR-30204506 | 490 | Yes | 643 |
| BPR-30204507 | 490 | Yes | 640 |
| BPR-30204508 | 490 | Yes | 634 |
| BPR-30204509 | 490 | Yes | 635 |
| BPR-30204510 | 491 | Yes | 630 |
| BPR-30204511 | 491 | Yes | 631 |
| BPR-30204512 | 492 | Yes | 636 |
| BPR-30204513 | 498 | Yes | 636 |
| BPR-30204514 | 496 | Yes | 636 |
| BPR-30204515 | 494 | Yes | 636 |
| BPR-30204516 | 492 | Yes | 632 |
| BPR-30204517 | 498 | Yes | 632 |
| BPR-30204518 | 496 | Yes | 632 |
| BPR-30204519 | 494 | Yes | 632 |
| BPR-30204520 | 492 | Yes | 628 |
| BPR-30204521 | 498 | Yes | 628 |
| BPR-30204522 | 496 | Yes | 628 |
| BPR-30204523 | 494 | Yes | 628 |
| BPR-30204524 | 492 | Yes | 633 |
| BPR-30204525 | 498 | Yes | 633 |
| BPR-30204526 | 496 | Yes | 633 |
| BPR-30204527 | 494 | Yes | 633 |
| BPR-30204528 | 492 | Yes | 643 |
| BPR-30204529 | 498 | Yes | 647 |
| BPR-30204530 | 498 | Yes | 646 |
| BPR-30204531 | 498 | Yes | 645 |
| BPR-30204532 | 496 | Yes | 647 |
| BPR-30204533 | 496 | Yes | 646 |
| BPR-30204534 | 496 | Yes | 645 |
| BPR-30204535 | 494 | Yes | 647 |
| BPR-30204536 | 494 | Yes | 646 |
| BPR-30204537 | 494 | Yes | 645 |
| BPR-30204538 | 492 | Yes | 640 |
| BPR-30204539 | 498 | Yes | 639 |
| BPR-30204540 | 498 | Yes | 638 |
| BPR-30204541 | 498 | Yes | 637 |
| BPR-30204542 | 496 | Yes | 639 |
| BPR-30204543 | 496 | Yes | 638 |
| BPR-30204544 | 496 | Yes | 637 |
| BPR-30204545 | 494 | Yes | 639 |
| BPR-30204546 | 494 | Yes | 638 |
| BPR-30204547 | 494 | Yes | 637 |
| BPR-30204548 | 492 | Yes | 634 |
| BPR-30204549 | 498 | Yes | 634 |
| BPR-30204550 | 496 | Yes | 634 |
| BPR-30204551 | 494 | Yes | 634 |
| BPR-30204552 | 493 | Yes | 629 |
| BPR-30204553 | 499 | Yes | 629 |
| BPR-30204554 | 497 | Yes | 629 |
| BPR-30204555 | 495 | Yes | 629 |
| BPR-30204556 | 492 | Yes | 635 |
| BPR-30204557 | 498 | Yes | 635 |
| BPR-30204558 | 496 | Yes | 635 |
| BPR-30204559 | 494 | Yes | 635 |
| BPR-30204560 | 487 | Yes | 636 |
| BPR-30204561 | 488 | Yes | 630 |
| BPR-30204562 | 500 | Yes | 636 |
| BPR-30204563 | 490 | Yes | 642 |
| BPR-30204564 | 490 | Yes | 648 |
| BPR-30204565 | 490 | Yes | 647 |
| BPR-30204566 | 490 | Yes | 646 |
| BPR-30204567 | 490 | Yes | 645 |
| BPR-30204568 | 490 | Yes | 639 |
| BPR-30204569 | 490 | Yes | 638 |
| BPR-30204570 | 490 | Yes | 637 |
| BPR-30211801 | 503 | Yes | 659 |
| BPR-30211802 | 502 | Yes | 659 |
| BPR-30211803 | 503 | Yes | 670 |
| BPR-30211804 | 503 | Yes | 675 |
| BPR-30211805 | 503 | Yes | 671 |
| BPR-30211806 | 503 | Yes | 665 |
| BPR-30211807 | 503 | Yes | 663 |
| BPR-30211808 | 503 | Yes | 657 |
| BPR-30211809 | 503 | Yes | 658 |
| BPR-30211810 | 503 | Yes | 652 |
| BPR-30211811 | 503 | Yes | 654 |
| BPR-30211812 | 504 | Yes | 659 |
| BPR-30211813 | 507 | Yes | 659 |
| BPR-30211814 | 506 | Yes | 659 |
| BPR-30211815 | 505 | Yes | 659 |
| BPR-30211816 | 504 | Yes | 673 |
| BPR-30211817 | 507 | Yes | 673 |
| BPR-30211818 | 506 | Yes | 673 |
| BPR-30211819 | 505 | Yes | 673 |
| BPR-30211820 | 504 | Yes | 672 |
| BPR-30211821 | 507 | Yes | 672 |
| BPR-30211822 | 506 | Yes | 672 |
| BPR-30211823 | 505 | Yes | 672 |
| BPR-30211824 | 504 | Yes | 674 |
| BPR-30211825 | 507 | Yes | 674 |
| BPR-30211826 | 506 | Yes | 674 |
| BPR-30211827 | 505 | Yes | 674 |
| BPR-30211828 | 504 | Yes | 665 |
| BPR-30211829 | 507 | Yes | 668 |
| BPR-30211830 | 507 | Yes | 667 |
| BPR-30211831 | 507 | Yes | 666 |
| BPR-30211832 | 506 | Yes | 668 |
| BPR-30211833 | 506 | Yes | 667 |
| BPR-30211834 | 506 | Yes | 666 |
| BPR-30211835 | 505 | Yes | 668 |
| BPR-30211836 | 505 | Yes | 667 |
| BPR-30211837 | 505 | Yes | 666 |
| BPR-30211838 | 504 | Yes | 663 |
| BPR-30211839 | 507 | Yes | 662 |
| BPR-30211840 | 507 | Yes | 661 |
| BPR-30211841 | 507 | Yes | 660 |
| BPR-30211842 | 506 | Yes | 662 |
| BPR-30211843 | 506 | Yes | 661 |
| BPR-30211844 | 506 | Yes | 660 |
| BPR-30211845 | 505 | Yes | 662 |
| BPR-30211846 | 505 | Yes | 661 |
| BPR-30211847 | 505 | Yes | 660 |
| BPR-30211848 | 504 | Yes | 657 |
| BPR-30211849 | 507 | Yes | 657 |
| BPR-30211850 | 506 | Yes | 657 |
| BPR-30211851 | 505 | Yes | 657 |
| BPR-30211852 | 504 | Yes | 656 |
| BPR-30211853 | 507 | Yes | 656 |
| BPR-30211854 | 506 | Yes | 656 |
| BPR-30211855 | 505 | Yes | 656 |
| BPR-30211856 | 504 | Yes | 658 |
| BPR-30211857 | 507 | Yes | 658 |
| BPR-30211858 | 506 | Yes | 658 |
| BPR-30211859 | 505 | Yes | 658 |
| BPR-30211860 | 501 | Yes | 659 |
| BPR-30211861 | 501 | Yes | 652 |
| BPR-30211862 | 508 | Yes | 659 |
| BPR-30211863 | 503 | Yes | 664 |
| BPR-30211864 | 503 | Yes | 669 |
| BPR-30211865 | 503 | Yes | 668 |
| BPR-30211866 | 503 | Yes | 667 |
| BPR-30211867 | 503 | Yes | 666 |
| BPR-30211868 | 503 | Yes | 662 |
| BPR-30211869 | 503 | Yes | 661 |
| BPR-30211870 | 503 | Yes | 660 |
| BPR-30213002 | 510 | Yes | 683 |
| BPR-30213003 | 512 | Yes | 694 |
| BPR-30213004 | 512 | Yes | 696 |
| BPR-30213005 | 512 | Yes | 695 |
| BPR-30213006 | 512 | Yes | 688 |
| BPR-30213007 | 512 | Yes | 686 |
| BPR-30213008 | 512 | Yes | 681 |
| BPR-30213009 | 512 | Yes | 682 |
| BPR-30213010 | 512 | Yes | 677 |
| BPR-30213011 | 512 | Yes | 678 |
| BPR-30213012 | 513 | Yes | 683 |
| BPR-30213013 | 516 | Yes | 683 |
| BPR-30213014 | 515 | Yes | 683 |
| BPR-30213015 | 514 | Yes | 683 |
| BPR-30213016 | 513 | Yes | 679 |
| BPR-30213017 | 516 | Yes | 679 |
| BPR-30213018 | 515 | Yes | 679 |
| BPR-30213019 | 514 | Yes | 679 |
| BPR-30213020 | 513 | Yes | 697 |
| BPR-30213021 | 516 | Yes | 697 |
| BPR-30213022 | 512 | Yes | 683 |
| BPR-30213023 | 515 | Yes | 697 |
| BPR-30213024 | 514 | Yes | 697 |
| BPR-30213025 | 513 | Yes | 680 |
| BPR-30213026 | 516 | Yes | 680 |
| BPR-30213027 | 515 | Yes | 680 |
| BPR-30213028 | 514 | Yes | 680 |
| BPR-30213029 | 513 | Yes | 688 |
| BPR-30213030 | 516 | Yes | 692 |
| BPR-30213031 | 516 | Yes | 691 |
| BPR-30213032 | 516 | Yes | 690 |
| BPR-30213033 | 515 | Yes | 692 |
| BPR-30213034 | 515 | Yes | 691 |
| BPR-30213035 | 515 | Yes | 690 |
| BPR-30213036 | 514 | Yes | 692 |
| BPR-30213037 | 514 | Yes | 691 |
| BPR-30213038 | 514 | Yes | 690 |
| BPR-30213039 | 513 | Yes | 686 |
| BPR-30213040 | 516 | Yes | 687 |
| BPR-30213041 | 516 | Yes | 685 |
| BPR-30213042 | 516 | Yes | 684 |
| BPR-30213043 | 515 | Yes | 687 |
| BPR-30213044 | 515 | Yes | 685 |
| BPR-30213045 | 515 | Yes | 684 |
| BPR-30213046 | 514 | Yes | 687 |
| BPR-30213047 | 514 | Yes | 685 |
| BPR-30213048 | 514 | Yes | 684 |
| BPR-30213049 | 513 | Yes | 681 |
| BPR-30213050 | 516 | Yes | 681 |
| BPR-30213051 | 515 | Yes | 681 |
| BPR-30213052 | 514 | Yes | 681 |
| BPR-30213053 | 513 | Yes | 676 |
| BPR-30213054 | 516 | Yes | 676 |
| BPR-30213055 | 515 | Yes | 676 |
| BPR-30213056 | 514 | Yes | 676 |
| BPR-30213057 | 513 | Yes | 682 |
| BPR-30213058 | 516 | Yes | 682 |
| BPR-30213059 | 515 | Yes | 682 |
| BPR-30213060 | 514 | Yes | 682 |
| BPR-30213061 | 509 | Yes | 683 |
| BPR-30213062 | 509 | Yes | 677 |
| BPR-30213063 | 511 | Yes | 683 |
| BPR-30213064 | 512 | Yes | 689 |
| BPR-30213065 | 512 | Yes | 693 |
| BPR-30213066 | 512 | Yes | 692 |
| BPR-30213067 | 512 | Yes | 691 |
| BPR-30213068 | 512 | Yes | 690 |
| BPR-30213069 | 512 | Yes | 687 |
| BPR-30213070 | 512 | Yes | 685 |
| BPR-30213071 | 512 | Yes | 684 |
| BPR-30216401 | 518 | Yes | 704 |
| BPR-30216402 | 519 | Yes | 704 |
| BPR-30216403 | 518 | Yes | 715 |
| BPR-30216404 | 518 | Yes | 718 |
| BPR-30216405 | 518 | Yes | 716 |
| BPR-30216406 | 518 | Yes | 708 |
| BPR-30216407 | 518 | Yes | 707 |
| BPR-30216408 | 518 | Yes | 701 |
| BPR-30216409 | 518 | Yes | 703 |
| BPR-30216410 | 518 | Yes | 699 |
| BPR-30216411 | 518 | Yes | 698 |
| BPR-30216412 | 520 | Yes | 704 |
| BPR-30216413 | 523 | Yes | 704 |
| BPR-30216414 | 522 | Yes | 704 |
| BPR-30216415 | 521 | Yes | 704 |
| BPR-30216416 | 520 | Yes | 717 |
| BPR-30216417 | 523 | Yes | 717 |
| BPR-30216418 | 522 | Yes | 717 |
| BPR-30216419 | 521 | Yes | 717 |
| BPR-30216420 | 520 | Yes | 702 |
| BPR-30216421 | 523 | Yes | 702 |
| BPR-30216422 | 522 | Yes | 702 |
| BPR-30216423 | 521 | Yes | 702 |
| BPR-30216424 | 520 | Yes | 719 |
| BPR-30216425 | 523 | Yes | 719 |
| BPR-30216426 | 522 | Yes | 719 |
| BPR-30216427 | 521 | Yes | 719 |
| BPR-30216428 | 520 | Yes | 708 |
| BPR-30216429 | 523 | Yes | 711 |
| BPR-30216430 | 523 | Yes | 712 |
| BPR-30216431 | 523 | Yes | 713 |
| BPR-30216432 | 522 | Yes | 711 |
| BPR-30216433 | 522 | Yes | 712 |
| BPR-30216434 | 522 | Yes | 713 |
| BPR-30216435 | 521 | Yes | 711 |
| BPR-30216436 | 521 | Yes | 712 |
| BPR-30216437 | 521 | Yes | 713 |
| BPR-30216438 | 520 | Yes | 707 |
| BPR-30216439 | 523 | Yes | 710 |
| BPR-30216440 | 523 | Yes | 706 |
| BPR-30216441 | 523 | Yes | 705 |
| BPR-30216442 | 522 | Yes | 710 |
| BPR-30216443 | 522 | Yes | 706 |
| BPR-30216444 | 522 | Yes | 705 |
| BPR-30216445 | 521 | Yes | 710 |
| BPR-30216446 | 521 | Yes | 706 |
| BPR-30216447 | 521 | Yes | 705 |
| BPR-30216448 | 520 | Yes | 701 |
| BPR-30216449 | 523 | Yes | 701 |
| BPR-30216450 | 522 | Yes | 701 |
| BPR-30216451 | 521 | Yes | 701 |
| BPR-30216452 | 520 | Yes | 700 |
| BPR-30216453 | 523 | Yes | 700 |
| BPR-30216454 | 522 | Yes | 700 |
| BPR-30216455 | 521 | Yes | 700 |
| BPR-30216456 | 520 | Yes | 703 |
| BPR-30216457 | 523 | Yes | 703 |
| BPR-30216458 | 522 | Yes | 703 |
| BPR-30216459 | 521 | Yes | 703 |
| BPR-30216460 | 517 | Yes | 704 |
| BPR-30216461 | 517 | Yes | 699 |
| BPR-30216462 | 524 | Yes | 704 |
| BPR-30216463 | 518 | Yes | 709 |
| BPR-30216464 | 518 | Yes | 723 |
| BPR-30216465 | 518 | Yes | 711 |
| BPR-30216466 | 518 | Yes | 712 |
| BPR-30216467 | 518 | Yes | 713 |
| BPR-30216468 | 518 | Yes | 710 |
| BPR-30216469 | 518 | Yes | 706 |
| BPR-30216470 | 518 | Yes | 705 |
| BPR-30217001 | 526 | Yes | 726 |
| BPR-30217002 | 527 | Yes | 726 |
| BPR-30217003 | 526 | Yes | 738 |
| BPR-30217004 | 526 | Yes | 741 |
| BPR-30217005 | 526 | Yes | 739 |
| BPR-30217006 | 526 | Yes | 733 |
| BPR-30217007 | 526 | Yes | 734 |
| BPR-30217008 | 526 | Yes | 727 |
| BPR-30217009 | 526 | Yes | 728 |
| BPR-30217010 | 526 | Yes | 720 |
| BPR-30217011 | 526 | Yes | 721 |
| BPR-30217012 | 528 | Yes | 726 |
| BPR-30217013 | 528 | Yes | 724 |
| BPR-30217014 | 531 | Yes | 724 |
| BPR-30217015 | 530 | Yes | 724 |
| BPR-30217016 | 529 | Yes | 724 |
| BPR-30217017 | 528 | Yes | 742 |
| BPR-30217018 | 531 | Yes | 742 |
| BPR-30217019 | 530 | Yes | 742 |
| BPR-30217020 | 529 | Yes | 742 |
| BPR-30217021 | 528 | Yes | 725 |
| BPR-30217022 | 531 | Yes | 725 |
| BPR-30217023 | 530 | Yes | 725 |
| BPR-30217024 | 529 | Yes | 725 |
| BPR-30217025 | 528 | Yes | 733 |
| BPR-30217026 | 531 | Yes | 735 |
| BPR-30217027 | 531 | Yes | 736 |
| BPR-30217028 | 531 | Yes | 737 |
| BPR-30217029 | 530 | Yes | 735 |
| BPR-30217030 | 530 | Yes | 736 |
| BPR-30217031 | 530 | Yes | 737 |
| BPR-30217032 | 529 | Yes | 735 |
| BPR-30217033 | 529 | Yes | 736 |
| BPR-30217034 | 529 | Yes | 737 |
| BPR-30217035 | 528 | Yes | 734 |
| BPR-30217036 | 531 | Yes | 729 |
| BPR-30217037 | 531 | Yes | 730 |
| BPR-30217038 | 531 | Yes | 731 |
| BPR-30217039 | 530 | Yes | 729 |
| BPR-30217040 | 530 | Yes | 730 |
| BPR-30217041 | 530 | Yes | 731 |
| BPR-30217042 | 529 | Yes | 729 |
| BPR-30217043 | 529 | Yes | 730 |
| BPR-30217044 | 529 | Yes | 731 |
| BPR-30217045 | 528 | Yes | 727 |
| BPR-30217046 | 531 | Yes | 727 |
| BPR-30217047 | 530 | Yes | 727 |
| BPR-30217048 | 529 | Yes | 727 |
| BPR-30217049 | 528 | Yes | 722 |
| BPR-30217050 | 531 | Yes | 722 |
| BPR-30217051 | 530 | Yes | 722 |
| BPR-30217052 | 529 | Yes | 722 |
| BPR-30217053 | 528 | Yes | 728 |
| BPR-30217054 | 531 | Yes | 728 |
| BPR-30217055 | 530 | Yes | 728 |
| BPR-30217056 | 529 | Yes | 728 |
| BPR-30217057 | 525 | Yes | 726 |
| BPR-30217058 | 525 | Yes | 720 |
| BPR-30217059 | 532 | Yes | 726 |
| BPR-30217060 | 526 | Yes | 732 |
| BPR-30217061 | 526 | Yes | 740 |
| BPR-30217062 | 526 | Yes | 729 |
| BPR-30217063 | 526 | Yes | 730 |
| BPR-30217064 | 526 | Yes | 731 |
| BPR-30217065 | 526 | Yes | 735 |
| BPR-30217066 | 526 | Yes | 736 |
| BPR-30217067 | 526 | Yes | 737 |
| BPR-30220701 | 876 | Yes | 877 |
| BPR-30221201 | 534 | Yes | 746 |
| BPR-30221202 | 535 | Yes | 746 |
| BPR-30221203 | 534 | Yes | 767 |
| BPR-30221204 | 534 | Yes | 768 |
| BPR-30221205 | 534 | Yes | 747 |
| BPR-30221206 | 534 | Yes | 879 |
| BPR-30221207 | 534 | Yes | 750 |
| BPR-30221208 | 534 | Yes | 597 |
| BPR-30221209 | 534 | Yes | 749 |
| BPR-30221210 | 534 | Yes | 743 |
| BPR-30221211 | 534 | Yes | 745 |
| BPR-30221212 | 601 | Yes | 746 |
| BPR-30221213 | 601 | Yes | 604 |
| BPR-30221214 | 601 | Yes | 763 |
| BPR-30221215 | 601 | Yes | 764 |
| BPR-30221216 | 601 | Yes | 879 |
| BPR-30221217 | 601 | Yes | 750 |
| BPR-30221218 | 756 | Yes | 597 |
| BPR-30221219 | 601 | Yes | 772 |
| BPR-30221220 | 601 | Yes | 749 |
| BPR-30221221 | 603 | Yes | 604 |
| BPR-30221222 | 533 | Yes | 743 |
| BPR-30221223 | 755 | Yes | 604 |
| BPR-30221224 | 536 | Yes | 604 |
| BPR-30221225 | 534 | Yes | 765 |
| BPR-30221226 | 534 | Yes | 766 |
| BPR-30221227 | 756 | Yes | 746 |
| BPR-30221228 | 756 | Yes | 604 |
| BPR-30221229 | 762 | Yes | 604 |
| BPR-30221230 | 755 | Yes | 746 |
| BPR-30221231 | 762 | Yes | 746 |
| BPR-30221232 | 756 | Yes | 763 |
| BPR-30221233 | 755 | Yes | 763 |
| BPR-30221234 | 762 | Yes | 763 |
| BPR-30221236 | 756 | Yes | 764 |
| BPR-30221237 | 755 | Yes | 764 |
| BPR-30221238 | 762 | Yes | 764 |
| BPR-30221239 | 756 | Yes | 769 |
| BPR-30221240 | 756 | Yes | 770 |
| BPR-30221241 | 756 | Yes | 771 |
| BPR-30221242 | 755 | Yes | 769 |
| BPR-30221243 | 755 | Yes | 770 |
| BPR-30221244 | 755 | Yes | 771 |
| BPR-30221245 | 762 | Yes | 769 |
| BPR-30221246 | 762 | Yes | 770 |
| BPR-30221247 | 762 | Yes | 771 |
| BPR-30221248 | 756 | Yes | 880 |
| BPR-30221249 | 756 | Yes | 773 |
| BPR-30221250 | 756 | Yes | 878 |
| BPR-30221251 | 755 | Yes | 880 |
| BPR-30221252 | 755 | Yes | 773 |
| BPR-30221253 | 755 | Yes | 878 |
| BPR-30221254 | 762 | Yes | 880 |
| BPR-30221255 | 762 | Yes | 773 |
| BPR-30221256 | 762 | Yes | 878 |
| BPR-30221257 | 601 | Yes | 597 |
| BPR-30221258 | 755 | Yes | 597 |
| BPR-30221259 | 762 | Yes | 597 |
| BPR-30221260 | 756 | Yes | 772 |
| BPR-30221261 | 755 | Yes | 772 |
| BPR-30221262 | 762 | Yes | 772 |
| BPR-30221263 | 756 | Yes | 749 |
| BPR-30221264 | 755 | Yes | 749 |
| BPR-30221265 | 762 | Yes | 749 |
| BPR-30221266 | 534 | Yes | 769 |
| BPR-30221267 | 534 | Yes | 770 |
| BPR-30221268 | 534 | Yes | 771 |
| BPR-30221269 | 534 | Yes | 880 |
| BPR-30221270 | 534 | Yes | 773 |
| BPR-30221271 | 534 | Yes | 878 |
| BPR-30221272 | 603 | Yes | 597 |
| BPR-30221273 | 756 | Yes | 896 |
| BPR-30221275 | 756 | Yes | 897 |
| BPR-30221276 | 909 | Yes | 898 |
| BPR-30221277 | 909 | Yes | 899 |
| BPR-30221278 | 910 | Yes | 900 |
| BPR-30221279 | 910 | Yes | 901 |
| BPR-30221280 | 913 | Yes | 905 |
| BPR-30221281 | 913 | Yes | 906 |
| BPR-30221601 | 744 | Yes | 621 |
| BPR-30221602 | 540 | Yes | 606 |
| BPR-30221603 | 605 | Yes | 881 |
| BPR-30221604 | 605 | Yes | 775 |
| BPR-30221605 | 605 | Yes | 776 |
| BPR-30221606 | 605 | Yes | 777 |
| BPR-30221607 | 605 | Yes | 785 |
| BPR-30221608 | 605 | Yes | 787 |
| BPR-30221609 | 605 | Yes | 783 |
| BPR-30221610 | 539 | Yes | 774 |
| BPR-30221611 | 539 | Yes | 778 |
| BPR-30221612 | 617 | Yes | 781 |
| BPR-30221613 | 617 | Yes | 777 |
| BPR-30221614 | 617 | Yes | 782 |
| BPR-30221615 | 751 | Yes | 606 |
| BPR-30221686 | 751 | Yes | 620 |
| BPR-30221617 | 754 | Yes | 621 |
| BPR-30221618 | 605 | Yes | 606 |
| BPR-30221619 | 752 | Yes | 620 |
| BPR-30221620 | 753 | Yes | 621 |
| BPR-30221621 | 537 | Yes | 606 |
| BPR-30221622 | 538 | Yes | 774 |
| BPR-30221623 | 751 | Yes | 783 |
| BPR-30221624 | 752 | Yes | 783 |
| BPR-30221625 | 617 | Yes | 779 |
| BPR-30221626 | 617 | Yes | 780 |
| BPR-30221627 | 541 | Yes | 606 |
| BPR-30221628 | 605 | Yes | 784 |
| BPR-30221629 | 605 | Yes | 786 |
| BPR-30221630 | 605 | Yes | 780 |
| BPR-30221631 | 605 | Yes | 779 |
| BPR-30221632 | 541 | Yes | 620 |
| BPR-30221633 | 537 | Yes | 620 |
| BPR-30221634 | 760 | Yes | 783 |
| BPR-30221635 | 617 | Yes | 783 |
| BPR-30221636 | 760 | Yes | 620 |
| BPR-30221637 | 761 | Yes | 621 |
| BPR-30221638 | 752 | Yes | 781 |
| BPR-30221639 | 760 | Yes | 781 |
| BPR-30221640 | 751 | Yes | 788 |
| BPR-30221641 | 751 | Yes | 789 |
| BPR-30221642 | 751 | Yes | 790 |
| BPR-30221643 | 752 | Yes | 788 |
| BPR-30221644 | 752 | Yes | 789 |
| BPR-30221645 | 752 | Yes | 790 |
| BPR-30221646 | 760 | Yes | 788 |
| BPR-30221647 | 760 | Yes | 789 |
| BPR-30221648 | 760 | Yes | 790 |
| BPR-30221649 | 751 | Yes | 782 |
| BPR-30221650 | 752 | Yes | 782 |
| BPR-30221651 | 760 | Yes | 782 |
| BPR-30221652 | 617 | Yes | 606 |
| BPR-30221653 | 752 | Yes | 606 |
| BPR-30221654 | 760 | Yes | 606 |
| BPR-30221655 | 617 | Yes | 620 |
| BPR-30221656 | 751 | Yes | 779 |
| BPR-30221657 | 752 | Yes | 779 |
| BPR-30221658 | 760 | Yes | 779 |
| BPR-30221659 | 751 | Yes | 791 |
| BPR-30221660 | 751 | Yes | 792 |
| BPR-30221661 | 751 | Yes | 793 |
| BPR-30221662 | 752 | Yes | 791 |
| BPR-30221663 | 752 | Yes | 792 |
| BPR-30221664 | 752 | Yes | 793 |
| BPR-30221665 | 760 | Yes | 791 |
| BPR-30221666 | 760 | Yes | 792 |
| BPR-30221667 | 760 | Yes | 793 |
| BPR-30221668 | 751 | Yes | 781 |
| BPR-30221669 | 605 | Yes | 788 |
| BPR-30221670 | 605 | Yes | 789 |
| BPR-30221671 | 605 | Yes | 790 |
| BPR-30221672 | 605 | Yes | 620 |
| BPR-30221673 | 605 | Yes | 792 |
| BPR-30221674 | 605 | Yes | 793 |
| BPR-30221675 | 605 | Yes | 791 |
| BPR-30221676 | 605 | Yes | 889 |
| BPR-30221677 | 751 | Yes | 890 |
| BPR-30221678 | 751 | Yes | 891 |
| BPR-30221679 | 907 | Yes | 892 |
| BPR-30221680 | 907 | Yes | 894 |
| BPR-30221681 | 908 | Yes | 893 |
| BPR-30221682 | 908 | Yes | 895 |
| BPR-30221683 | 911 | Yes | 902 |
| BPR-30221684 | 911 | Yes | 903 |
| BPR-30221685 | 912 | Yes | 904 |
| BPR-30221901 | 544 | Yes | 800 |
| BPR-30221902 | 543 | Yes | 800 |
| BPR-30221903 | 544 | Yes | 813 |
| BPR-30221904 | 544 | Yes | 882 |
| BPR-30221905 | 544 | Yes | 812 |
| BPR-30221906 | 544 | Yes | 806 |
| BPR-30221907 | 544 | Yes | 804 |
| BPR-30221908 | 544 | Yes | 811 |
| BPR-30221909 | 544 | Yes | 799 |
| BPR-30221910 | 544 | Yes | 796 |
| BPR-30221911 | 544 | Yes | 883 |
| BPR-30221912 | 545 | Yes | 800 |
| BPR-30221913 | 548 | Yes | 800 |
| BPR-30221914 | 547 | Yes | 800 |
| BPR-30221915 | 546 | Yes | 800 |
| BPR-30221916 | 545 | Yes | 797 |
| BPR-30221917 | 548 | Yes | 797 |
| BPR-30221918 | 547 | Yes | 797 |
| BPR-30221919 | 546 | Yes | 797 |
| BPR-30221920 | 545 | Yes | 795 |
| BPR-30221921 | 548 | Yes | 795 |
| BPR-30221922 | 547 | Yes | 795 |
| BPR-30221923 | 546 | Yes | 795 |
| BPR-30221924 | 545 | Yes | 798 |
| BPR-30221925 | 548 | Yes | 798 |
| BPR-30221926 | 547 | Yes | 798 |
| BPR-30221927 | 546 | Yes | 798 |
| BPR-30221928 | 545 | Yes | 806 |
| BPR-30221929 | 548 | Yes | 809 |
| BPR-30221930 | 548 | Yes | 808 |
| BPR-30221931 | 548 | Yes | 807 |
| BPR-30221932 | 547 | Yes | 809 |
| BPR-30221933 | 547 | Yes | 808 |
| BPR-30221934 | 547 | Yes | 807 |
| BPR-30221935 | 546 | Yes | 809 |
| BPR-30221936 | 546 | Yes | 808 |
| BPR-30221937 | 546 | Yes | 807 |
| BPR-30221938 | 545 | Yes | 804 |
| BPR-30221939 | 548 | Yes | 803 |
| BPR-30221940 | 548 | Yes | 802 |
| BPR-30221941 | 548 | Yes | 801 |
| BPR-30221942 | 547 | Yes | 803 |
| BPR-30221943 | 547 | Yes | 802 |
| BPR-30221944 | 547 | Yes | 801 |
| BPR-30221945 | 546 | Yes | 803 |
| BPR-30221946 | 546 | Yes | 802 |
| BPR-30221947 | 546 | Yes | 801 |
| BPR-30221948 | 545 | Yes | 811 |
| BPR-30221949 | 548 | Yes | 811 |
| BPR-30221950 | 547 | Yes | 811 |
| BPR-30221951 | 546 | Yes | 811 |
| BPR-30221952 | 545 | Yes | 794 |
| BPR-30221953 | 548 | Yes | 794 |
| BPR-30221954 | 547 | Yes | 794 |
| BPR-30221955 | 546 | Yes | 794 |
| BPR-30221956 | 545 | Yes | 799 |
| BPR-30221957 | 548 | Yes | 799 |
| BPR-30221958 | 547 | Yes | 799 |
| BPR-30221959 | 546 | Yes | 799 |
| BPR-30221960 | 542 | Yes | 800 |
| BPR-30221961 | 542 | Yes | 796 |
| BPR-30221962 | 549 | Yes | 800 |
| BPR-30221963 | 544 | Yes | 805 |
| BPR-30221964 | 544 | Yes | 810 |
| BPR-30221965 | 544 | Yes | 809 |
| BPR-30221966 | 544 | Yes | 808 |
| BPR-30221967 | 544 | Yes | 807 |
| BPR-30221968 | 544 | Yes | 803 |
| BPR-30221969 | 544 | Yes | 802 |
| BPR-30221970 | 544 | Yes | 801 |
| BPR-30222201 | 641 | Yes | 814 |
| BPR-30222202 | 552 | Yes | 814 |
| BPR-30222203 | 641 | Yes | 644 |
| BPR-30222204 | 641 | Yes | 815 |
| BPR-30222205 | 641 | Yes | 816 |
| BPR-30222206 | 641 | Yes | 817 |
| BPR-30222207 | 641 | Yes | 818 |
| BPR-30222208 | 641 | Yes | 819 |
| BPR-30222209 | 641 | Yes | 820 |
| BPR-30222210 | 553 | Yes | 821 |
| BPR-30222211 | 553 | Yes | 822 |
| BPR-30222212 | 757 | Yes | 655 |
| BPR-30222213 | 653 | Yes | 823 |
| BPR-30222214 | 653 | Yes | 814 |
| BPR-30222215 | 757 | Yes | 824 |
| BPR-30222216 | 653 | Yes | 817 |
| BPR-30222217 | 653 | Yes | 818 |
| BPR-30222218 | 757 | Yes | 644 |
| BPR-30222219 | 554 | Yes | 825 |
| BPR-30222220 | 653 | Yes | 820 |
| BPR-30222221 | 550 | Yes | 814 |
| BPR-30222222 | 551 | Yes | 821 |
| BPR-30222223 | 653 | Yes | 819 |
| BPR-30222224 | 558 | Yes | 814 |
| BPR-30222225 | 641 | Yes | 830 |
| BPR-30222226 | 641 | Yes | 827 |
| BPR-30222227 | 558 | Yes | 644 |
| BPR-30222228 | 550 | Yes | 644 |
| BPR-30222229 | 653 | Yes | 655 |
| BPR-30222230 | 758 | Yes | 655 |
| BPR-30222231 | 759 | Yes | 655 |
| BPR-30222232 | 757 | Yes | 823 |
| BPR-30222233 | 758 | Yes | 823 |
| BPR-30222234 | 759 | Yes | 823 |
| BPR-30222235 | 757 | Yes | 814 |
| BPR-30222236 | 758 | Yes | 814 |
| BPR-30222237 | 759 | Yes | 814 |
| BPR-30222238 | 653 | Yes | 824 |
| BPR-30222239 | 758 | Yes | 824 |
| BPR-30222240 | 759 | Yes | 824 |
| BPR-30222241 | 757 | Yes | 826 |
| BPR-30222242 | 757 | Yes | 828 |
| BPR-30222243 | 757 | Yes | 829 |
| BPR-30222244 | 758 | Yes | 826 |
| BPR-30222245 | 758 | Yes | 828 |
| BPR-30222246 | 758 | Yes | 829 |
| BPR-30222247 | 759 | Yes | 826 |
| BPR-30222248 | 759 | Yes | 828 |
| BPR-30222249 | 759 | Yes | 829 |
| BPR-30222250 | 757 | Yes | 831 |
| BPR-30222251 | 757 | Yes | 832 |
| BPR-30222252 | 757 | Yes | 833 |
| BPR-30222253 | 758 | Yes | 831 |
| BPR-30222254 | 758 | Yes | 832 |
| BPR-30222255 | 758 | Yes | 833 |
| BPR-30222256 | 759 | Yes | 831 |
| BPR-30222257 | 759 | Yes | 832 |
| BPR-30222258 | 759 | Yes | 833 |
| BPR-30222259 | 653 | Yes | 644 |
| BPR-30222260 | 557 | Yes | 825 |
| BPR-30222261 | 556 | Yes | 825 |
| BPR-30222262 | 555 | Yes | 825 |
| BPR-30222263 | 757 | Yes | 820 |
| BPR-30222264 | 758 | Yes | 820 |
| BPR-30222265 | 759 | Yes | 820 |
| BPR-30222266 | 641 | Yes | 655 |
| BPR-30222267 | 641 | Yes | 826 |
| BPR-30222268 | 641 | Yes | 828 |
| BPR-30222269 | 641 | Yes | 829 |
| BPR-30222270 | 641 | Yes | 831 |
| BPR-30222271 | 641 | Yes | 832 |
| BPR-30222272 | 641 | Yes | 833 |
| BPR-30222273 | 757 | Yes | 819 |
| BPR-30222274 | 758 | Yes | 819 |
| BPR-30222275 | 759 | Yes | 819 |
| BPR-30222276 | 758 | Yes | 644 |
| BPR-30222277 | 759 | Yes | 644 |
| BPR-30222601 | 560 | Yes | 841 |
| BPR-30222602 | 561 | Yes | 841 |
| BPR-30222603 | 560 | Yes | 852 |
| BPR-30222604 | 560 | Yes | 853 |
| BPR-30222605 | 560 | Yes | 854 |
| BPR-30222606 | 560 | Yes | 845 |
| BPR-30222607 | 560 | Yes | 844 |
| BPR-30222608 | 560 | Yes | 840 |
| BPR-30222609 | 560 | Yes | 851 |
| BPR-30222610 | 560 | Yes | 836 |
| BPR-30222611 | 560 | Yes | 834 |
| BPR-30222612 | 562 | Yes | 841 |
| BPR-30222613 | 565 | Yes | 841 |
| BPR-30222614 | 564 | Yes | 841 |
| BPR-30222615 | 563 | Yes | 841 |
| BPR-30222616 | 562 | Yes | 837 |
| BPR-30222617 | 565 | Yes | 837 |
| BPR-30222618 | 564 | Yes | 837 |
| BPR-30222619 | 563 | Yes | 837 |
| BPR-30222620 | 562 | Yes | 855 |
| BPR-30222621 | 565 | Yes | 855 |
| BPR-30222622 | 564 | Yes | 855 |
| BPR-30222623 | 563 | Yes | 855 |
| BPR-30222624 | 562 | Yes | 838 |
| BPR-30222625 | 565 | Yes | 838 |
| BPR-30222626 | 564 | Yes | 838 |
| BPR-30222627 | 563 | Yes | 838 |
| BPR-30222628 | 562 | Yes | 845 |
| BPR-30222629 | 565 | Yes | 847 |
| BPR-30222630 | 565 | Yes | 848 |
| BPR-30222631 | 565 | Yes | 849 |
| BPR-30222632 | 564 | Yes | 847 |
| BPR-30222633 | 564 | Yes | 848 |
| BPR-30222634 | 564 | Yes | 849 |
| BPR-30222635 | 563 | Yes | 847 |
| BPR-30222636 | 563 | Yes | 848 |
| BPR-30222637 | 563 | Yes | 849 |
| BPR-30222638 | 562 | Yes | 844 |
| BPR-30222639 | 565 | Yes | 843 |
| BPR-30222640 | 565 | Yes | 842 |
| BPR-30222641 | 565 | Yes | 839 |
| BPR-30222642 | 564 | Yes | 843 |
| BPR-30222643 | 564 | Yes | 842 |
| BPR-30222644 | 564 | Yes | 839 |
| BPR-30222645 | 563 | Yes | 843 |
| BPR-30222646 | 563 | Yes | 842 |
| BPR-30222647 | 563 | Yes | 839 |
| BPR-30222648 | 562 | Yes | 840 |
| BPR-30222649 | 565 | Yes | 840 |
| BPR-30222650 | 564 | Yes | 840 |
| BPR-30222651 | 563 | Yes | 840 |
| BPR-30222652 | 562 | Yes | 835 |
| BPR-30222653 | 565 | Yes | 835 |
| BPR-30222654 | 564 | Yes | 835 |
| BPR-30222655 | 563 | Yes | 835 |
| BPR-30222656 | 562 | Yes | 851 |
| BPR-30222657 | 565 | Yes | 851 |
| BPR-30222658 | 564 | Yes | 851 |
| BPR-30222659 | 563 | Yes | 851 |
| BPR-30222660 | 559 | Yes | 841 |
| BPR-30222661 | 559 | Yes | 836 |
| BPR-30222662 | 566 | Yes | 841 |
| BPR-30222663 | 560 | Yes | 846 |
| BPR-30222664 | 560 | Yes | 850 |
| BPR-30222665 | 560 | Yes | 847 |
| BPR-30222666 | 560 | Yes | 848 |
| BPR-30222667 | 560 | Yes | 849 |
| BPR-30222668 | 560 | Yes | 843 |
| BPR-30222669 | 560 | Yes | 842 |
| BPR-30222670 | 560 | Yes | 839 |
| BPR-30223101 | 569 | Yes | 873 |
| BPR-30223102 | 568 | Yes | 873 |
| BPR-30223103 | 569 | Yes | 859 |
| BPR-30223104 | 569 | Yes | 856 |
| BPR-30223105 | 569 | Yes | 857 |
| BPR-30223106 | 569 | Yes | 867 |
| BPR-30223107 | 569 | Yes | 872 |
| BPR-30223108 | 569 | Yes | 862 |
| BPR-30223109 | 569 | Yes | 874 |
| BPR-30223110 | 569 | Yes | 860 |
| BPR-30223111 | 569 | Yes | 714 |
| BPR-30223112 | 570 | Yes | 873 |
| BPR-30223113 | 570 | Yes | 875 |
| BPR-30223114 | 570 | Yes | 858 |
| BPR-30223115 | 570 | Yes | 748 |
| BPR-30223116 | 570 | Yes | 867 |
| BPR-30223117 | 573 | Yes | 863 |
| BPR-30223118 | 573 | Yes | 864 |
| BPR-30223119 | 573 | Yes | 866 |
| BPR-30223120 | 572 | Yes | 863 |
| BPR-30223121 | 572 | Yes | 864 |
| BPR-30223122 | 572 | Yes | 866 |
| BPR-30223123 | 571 | Yes | 863 |
| BPR-30223124 | 571 | Yes | 864 |
| BPR-30223125 | 571 | Yes | 866 |
| BPR-30223126 | 570 | Yes | 872 |
| BPR-30223127 | 573 | Yes | 871 |
| BPR-30223128 | 573 | Yes | 870 |
| BPR-30223129 | 573 | Yes | 869 |
| BPR-30223130 | 572 | Yes | 871 |
| BPR-30223131 | 572 | Yes | 870 |
| BPR-30223132 | 572 | Yes | 869 |
| BPR-30223133 | 571 | Yes | 871 |
| BPR-30223134 | 571 | Yes | 870 |
| BPR-30223135 | 571 | Yes | 869 |
| BPR-30223136 | 570 | Yes | 862 |
| BPR-30223137 | 573 | Yes | 862 |
| BPR-30223138 | 572 | Yes | 862 |
| BPR-30223139 | 571 | Yes | 862 |
| BPR-30223140 | 570 | Yes | 865 |
| BPR-30223141 | 573 | Yes | 865 |
| BPR-30223142 | 572 | Yes | 865 |
| BPR-30223143 | 571 | Yes | 865 |
| BPR-30223144 | 570 | Yes | 874 |
| BPR-30223145 | 573 | Yes | 874 |
| BPR-30223146 | 572 | Yes | 874 |
| BPR-30223147 | 571 | Yes | 874 |
| BPR-30223148 | 567 | Yes | 873 |
| BPR-30223149 | 567 | Yes | 860 |
| BPR-30223150 | 574 | Yes | 873 |
| BPR-30223151 | 569 | Yes | 868 |
| BPR-30223152 | 569 | Yes | 861 |
| BPR-30223153 | 573 | Yes | 875 |
| BPR-30223154 | 572 | Yes | 875 |
| BPR-30223155 | 571 | Yes | 875 |
| BPR-30223156 | 573 | Yes | 858 |
| BPR-30223157 | 572 | Yes | 858 |
| BPR-30223158 | 571 | Yes | 858 |
| BPR-30223159 | 573 | Yes | 748 |
| BPR-30223160 | 572 | Yes | 748 |
| BPR-30223161 | 571 | Yes | 748 |
| BPR-30223162 | 569 | Yes | 863 |
| BPR-30223163 | 569 | Yes | 864 |
| BPR-30223164 | 569 | Yes | 866 |
| BPR-30223165 | 569 | Yes | 871 |
| BPR-30223166 | 569 | Yes | 870 |
| BPR-30223167 | 569 | Yes | 869 |

**Table 6**

| SEQ ID NO | Modified antisense strand (5'-3') |
|---|---|
| 575 | VpUmsAfsCmCmdAUmdCAmGmAmGmGmAmCfAmCfUmUmGmGmAmsUmsUm |
| 576 | VpUmsAfsCmCmAmUfCmAmGmAmGmGmAmCfAmCfUmUmGmGmAmsUmsUm |
| 577 | VpUmsAfsCmCmAmUfCmAmGmAmG(moe)GmAmCfAmCfUmUmGmGmAmsUmsUm |
| 578 | UmsAfsCmCmdAUmdCAmGmAmGmGmAmCfAmCfUmUmGmGmAmsUmsUm |
| 579 | UmsAfsCmCmdAUfCmAmGmAmGmGmAmCfAmCfUmUmGmGmAmsUmsUm |
| 580 | UmsAfsCmCmAmUfdCAmGmAmGmGmAmCfAmCfUmUmGmGmAmsUmsUm |
| 581 | UmsAfsCmCmAmUfCmAmGmAmGmGmAmCfAmCfUmUmGmGmAmsUmsUm |
| 582 | UmsAfsCmCmAmUfCmAmGmAmGmGmAmCfAmCfUmUmGmGmAmsUmsUh₁₆ |
| 583 | UmsAfsCmCmAmUfCmAmGmAmGmGmAmCfAmCfUmUmGmGmAmsUmsUh₁₄ |
| 584 | UmsAfsCmCmAmUfCmAmGmAmGmGmAmCfAmCfUmUmGmGmAmsUmsUh₁₂ |
| 585 | UmsAfsCmCmAmUfCmAmGmAmGmGmAmCfAmCfUmUmGmGmAmsUmsUh₂₀ |
| 586 | UmsAfsCmCmAmUfCmAmGmAmGmGmAmCfAmCfUmUmGmGmAmsUmsTb |
| 587 | UmsAfsCmCmAmUfCmAmGmAmGmGmAmCfAmCfUmUmGmGmAmsUmsiU |
| 588 | UmsAfsCmCmAmUfCmAmGmAmGmGmAmCfAmCfUmUmGmGmAmsUh₁₀sUh₁₀ |
| 589 | UmsAfsCmCmAmUfCmAmGmAmGmGmAmCfAmCfUmUmGmGmAmsUh₁₆sUh₁₆ |
| 590 | UmsAfsCmCmAmUfCmAmGmAmGmGmAmCfAmCfUmUmGmGmAmsUh₁₄sUh₁₄ |
| 591 | UmsAfsCmCmAmUfCmAmGmAmGmGmAmCfAmCfUmUmGmGmAmsUh₁₂sUh₁₂ |
| 592 | UmsAfsCmCmAmUfCmAmGmAmGmGmAmCfAmCfUmUmGmGmAmsTbsTb |
| 593 | UmsAfsCmCmAmUfCmAmGmAmGmGmAmCfAmCfUmUmGmGmAmsiUsiU |
| 594 | UmsAfsCmCmAmUfCmAmGmAmG(moe)GmAmCfAmCfUmUmGmGmAmsUmsUm |
| 595 | UmsAfsCmCmAmUfCkAmGmAmGmGmAmCfAmCfUmUmGmGmAmsUmsUm |
| 596 | UmsAfsC(moe)CmAmUfCmAmGmAmGmGmAmCfAmCfUmUmGmGmAmsUmsUm |
| 597 | UmsAfsGmGmUmGfAmAmAmAmCmAmCmUfGmCfUmUmUmAmGmsiGsiG |
| 598 | VpUmsAfsGmAmAmCfUmUmUmGmAmCmCmAfUmCfAmGmAmGmGmsAmsCm |
| 599 | VpUmsAfsGmAmAmCfUmUmUmGmA(moe)CmCmAfUmCfAmGmAmGmGmsAmsCm |
| 600 | UmsAfsGmAmdACmdTUmUmGmAmCmCmAfUmCfAmGmAmGmGmsAmsCm |
| 602 | UmsAfsGmAmdACfUmUmUmGmAmCmCmAfUmCfAmGmAmGmGmsAmsCm |
| 604 | UmsAfsGmGmdTGmdAAmAmAmCmAmCmUfGmCfUmUmUmAmGmsGmsGm |
| 606 | AmsUfsAmUmGmAfGmGmUmGmAmAmAmAfCmAfCmUmGmCmUmsGmsGm |
| 607 | UmsAfsGmAmAmCfUmUmUmGmAmCmCmAfUmCfAmGmAmGmGmsiAsiC |
| 608 | UmsAfsGmAmAmCfUmUmUmGmAmCmCmAfUmCfAmGmAmGmGmsAmsiC |
| 609 | UmsAfsGmAmAmCfUmUmUmGmAmCmCmAfUmCfAmGmAmGmGmsAmsCm |
| 610 | UmsAfsGmAmAmCfUmUmUmGmAmCmCmAfUmCfAmGmAmGmGmsAmsCh₁₆ |
| 611 | UmsAfsGmAmAmCfUmUmUmGmAmCmCmAfUmCfAmGmAmGmGmsAmsCh₁₄ |
| 612 | UmsAfsGmAmAmCfUmUmUmGmAmCmCmAfUmCfAmGmAmGmGmsAmsCh₁₂ |
| 613 | UmsAfsGmAmAmCfUmUmUmGmAmCmCmAfUmCfAmGmAmGmGmsAmsCh₂₀ |
| 614 | UmsAfsGmAmAmCfUmUmUmGmAmCmCmAfUmCfAmGmAmGmGmsAmsCb |
| 615 | UmsAfsGmAmAmCfUmUmUmGmAmCmCmAfUmCfAmGmAmGmGmsAh₁₀sCh₁₀ |
| 616 | UmsAfsGmAmAmCfUmUmUmGmAmCmCmAfUmCfAmGmAmGmGmsAh₁₆sCh₁₆ |
| 618 | UmsAfsGmAmAmCfUmUmUmGmAmCmCmAfUmCfAmGmAmGmGmsAh₁₂sCh₁₂ |
| 619 | UmsAfsGmAmAmCfUmUmUmGmAmCmCmAfUmCfAmGmAmGmGmsAh₁₄sCh₁₄ |
| 620 | AmsUfsAmUmdGAmdGGmUmGmAmAmAmAfCmAfCmUmGmCmUmsGmsGm |
| 621 | VpUmsUfsAmUmdGAmdGGmUmGmAmAmAmAfCmAfCmUmGmCmUmsGmsGm |
| 622 | UmsAfsGmAmAmCfUmUmUmGmAmCmCmAfUmCfAmGmAmGmGmsAbsCb |
| 623 | UmsAfsGmAmAmCfUmUmUmGmA(moe)CmCmAfUmCfAmGmAmGmGmsAmsCm |
| 624 | UmsAfsGmAmAmCfUkUmUmGmAmCmCmAfUmCfAmGmAmGmGmsAmsCm |
| 625 | UmsAfsGmAmAmCfdTUmUmGmAmCmCmAfUmCfAmGmAmGmGmsAmsCm |
| 626 | UmsAfsG(moe)AmAmCfUmUmUmGmAmCmCmAfUmCfAmGmAmGmGmsAmsCm |
| 627 | VpUmsAfsGmAmdACmdTUmUmGmAmCmCmAfUmCfAmGmAmGmGmsAmsCm |
| 628 | AmsUfsCmUmAmGfdAAmCmUmUmUmGmAfCmCfAmUmCmAmGmsAmsGm |
| 629 | VpUmsUfsCmUmdAGmdAAmCmUmUmUmGmAfCmCfAmUmCmAmGmsAmsGm |
| 630 | VpUmsUfsCmUmAmGfAmAmCmUmUmUmGmAfCmCfAmUmCmAmGmsAmsGm |
| 631 | VpUmsUfsCmUmAmGfAmAmCmUmU(moe)UmGmAfCmCfAmUmCmAmGmsAmsGm |
| 632 | AmsUfsCmUmdAGmdAAmCmUmUmUmGmAfCmCfAmUmCmAmGmsAmsGm |
| 633 | AmsUfsCmUmdAGfAmAmCmUmUmUmGmAfCmCfAmUmCmAmGmsAmsGm |
| 634 | AmsUfsCmUmAmGfAmAmCmUmUmUmGmAfCmCfAmUmCmAmGmsiAsiG |
| 635 | AmsUfsCmUmAmGfAmAmCmUmUmUmGmAfCmCfAmUmCmAmGmsAmsiG |
| 636 | AmsUfsCmUmAmGfAmAmCmUmUmUmGmAfCmCfAmUmCmAmGmsAmsGm |
| 637 | AmsUfsCmUmAmGfAmAmCmUmUmUmGmAfCmCfAmUmCmAmGmsAmsGh₁₆ |
| 638 | AmsUfsCmUmAmGfAmAmCmUmUmUmGmAfCmCfAmUmCmAmGmsAmsGh₁₄ |
| 639 | AmsUfsCmUmAmGfAmAmCmUmUmUmGmAfCmCfAmUmCmAmGmsAmsGh₁₂ |
| 640 | AmsUfsCmUmAmGfAmAmCmUmUmUmGmAfCmCfAmUmCmAmGmsAmsGh₂₀ |
| 642 | AmsUfsCmUmAmGfAmAmCmUmUmUmGmAfCmCfAmUmCmAmGmsAmsGb |
| 643 | AmsUfsCmUmAmGfAmAmCmUmUmUmGmAfCmCfAmUmCmAmGmsAh₁₀sGh₁₀ |
| 644 | AmsAfsUmGmUmUfUmUmAmUmU(moe)GmUmCfUmCfUmGmCmCmUmsGmsGm |
| 645 | AmsUfsCmUmAmGfAmAmCmUmUmUmGmAfCmCfAmUmCmAmGmsAh₁₆sGh₁₆ |
| 646 | AmsUfsCmUmAmGfAmAmCmUmUmUmGmAfCmCfAmUmCmAmGmsAh₁₄sGh₁₄ |
| 647 | AmsUfsCmUmAmGfAmAmCmUmUmUmGmAfCmCfAmUmCmAmGmsAh₁₂sGh₁₂ |
| 648 | AmsUfsCmUmAmGfAmAmCmUmUmUmGmAfCmCfAmUmCmAmGmsAbsGb |
| 649 | AmsUfsCmUmAmGfAmAmCmUmU(moe)UmGmAfCmCfAmUmCmAmGmsAmsGm |
| 650 | AmsUfsC(moe)UmAmGfAmAmCmUmUmUmGmAfCmCfAmUmCmAmGmsAmsGm |
| 651 | AmsUfsCmUmAmGfAkAmCmUmUmUmGmAfCmCfAmUmCmAmGmsAmsGm |
| 652 | VpUmsCfsUmAmUmUfUmCmCmAmCmUmUmUfGmUfAmUmAmUmCmsCmsCm |
| 654 | VpUmsCfsUmAmUmUfUmCmCmAmC(moe)UmUmUfGmUfAmUmAmUmCmsCmsCm |
| 655 | AmsAfsUmGmUmUfdTUmAmUmUmGmUmCfUmCfUmGmCmCmUmsGmsGm |
| 656 | VpUmsCfsUmAmdTUmdTCmCmAmCmUmUmUfGmUfAmUmAmUmCmsCmsCm |
| 657 | UmsCfsUmAmUmUfUmCmCmAmCmUmUmUfGmUfAmUmAmUmCmsiCsiC |
| 658 | UmsCfsUmAmUmUfUmCmCmAmCmUmUmUfGmUfAmUmAmUmCmsCmsiC |
| 659 | UmsCfsUmAmUmUfUmCmCmAmCmUmUmUfGmUfAmUmAmUmCmsCmsCm |
| 660 | UmsCfsUmAmUmUfUmCmCmAmCmUmUmUfGmUfAmUmAmUmCmsCmsCh₁₆ |
| 661 | UmsCfsUmAmUmUfUmCmCmAmCmUmUmUfGmUfAmUmAmUmCmsCmsCh₁₄ |
| 662 | UmsCfsUmAmUmUfUmCmCmAmCmUmUmUfGmUfAmUmAmUmCmsCmsCh₁₂ |
| 663 | UmsCfsUmAmUmUfUmCmCmAmCmUmUmUfGmUfAmUmAmUmCmsCmsCh₂₀ |
| 664 | UmsCfsUmAmUmUfUmCmCmAmCmUmUmUfGmUfAmUmAmUmCmsCmsCb |
| 665 | UmsCfsUmAmUmUfUmCmCmAmCmUmUmUfGmUfAmUmAmUmCmsCh₁₀sCh₁₀ |
| 666 | UmsCfsUmAmUmUfUmCmCmAmCmUmUmUfGmUfAmUmAmUmCmsCh₁₆sCh₁₆ |
| 667 | UmsCfsUmAmUmUfUmCmCmAmCmUmUmUfGmUfAmUmAmUmCmsCh₁₄sCh₁₄ |
| 668 | UmsCfsUmAmUmUfUmCmCmAmCmUmUmUfGmUfAmUmAmUmCmsCh₁₂sCh₁₂ |
| 669 | UmsCfsUmAmUmUfUmCmCmAmCmUmUmUfGmUfAmUmAmUmCmsCbsCb |
| 670 | UmsCfsUmAmUmUfUmCmCmAmC(moe)UmUmUfGmUfAmUmAmUmCmsCmsCm |
| 671 | UmsCfsUmAmUmUfUkCmCmAmCmUmUmUfGmUfAmUmAmUmCmsCmsCm |
| 672 | UmsCfsUmAmUmUfdTCmCmAmCmUmUmUfGmUfAmUmAmUmCmsCmsCm |
| 673 | UmsCfsUmAmdTUmdTCmCmAmCmUmUmUfGmUfAmUmAmUmCmsCmsCm |
| 674 | UmsCfsUmAmdTUfUmCmCmAmCmUmUmUfGmUfAmUmAmUmCmsCmsCm |
| 675 | UmsCfsU(moe)AmUmUfUmCmCmAmCmUmUmUfGmUfAmUmAmUmCmsCmsCm |
| 676 | VpUmsAfsAmGmdAUmdTUmGmGmUmGmUmCfUmAfUmUmUmCmCmsAmsCm |
| 677 | VpUmsAfsAmGmAmUfUmUmGmGmUmGmUmCfUmAfUmUmUmCmCmsAmsCm |
| 678 | VpUmsAfsAmGmAmUfUmUmGmGmU(moe)GmUmCfUmAfUmUmUmCmCmsAmsCm |
| 679 | UmsAfsAmGmdAUmdTUmGmGmUmGmUmCfUmAfUmUmUmCmCmsAmsCm |
| 680 | UmsAfsAmGmdAUfUmUmGmGmUmGmUmCfUmAfUmUmUmCmCmsAmsCm |
| 681 | UmsAfsAmGmAmUfUmUmGmGmUmGmUmCfUmAfUmUmUmCmCmsiAsiC |
| 682 | UmsAfsAmGmAmUfUmUmGmGmUmGmUmCfUmAfUmUmUmCmCmsAmsiC |
| 683 | UmsAfsAmGmAmUfUmUmGmGmUmGmUmCfUmAfUmUmUmCmCmsAmsCm |
| 684 | UmsAfsAmGmAmUfUmUmGmGmUmGmUmCfUmAfUmUmUmCmCmsAmsCh₁₆ |
| 685 | UmsAfsAmGmAmUfUmUmGmGmUmGmUmCfUmAfUmUmUmCmCmsAmsCh₁₄ |
| 686 | UmsAfsAmGmAmUfUmUmGmGmUmGmUmCfUmAfUmUmUmCmCmsAmsCh₂₀ |
| 687 | UmsAfsAmGmAmUfUmUmGmGmUmGmUmCfUmAfUmUmUmCmCmsAmsCh₁₂ |
| 688 | UmsAfsAmGmAmUfUmUmGmGmUmGmUmCfUmAfUmUmUmCmCmsAh₁₀sCh₁₀ |
| 689 | UmsAfsAmGmAmUfUmUmGmGmUmGmUmCfUmAfUmUmUmCmCmsAmsCb |
| 690 | UmsAfsAmGmAmUfUmUmGmGmUmGmUmCfUmAfUmUmUmCmCmsAh₁₆sCh₁₆ |
| 691 | UmsAfsAmGmAmUfUmUmGmGmUmGmUmCfUmAfUmUmUmCmCmsAh₁₄sCh₁₄ |
| 692 | UmsAfsAmGmAmUfUmUmGmGmUmGmUmCfUmAfUmUmUmCmCmsAh₁₂sCh₁₂ |
| 693 | UmsAfsAmGmAmUfUmUmGmGmUmGmUmCfUmAfUmUmUmCmCmsAbsCb |
| 694 | UmsAfsAmGmAmUfUmUmGmGmU(moe)GmUmCfUmAfUmUmUmCmCmsAmsCm |
| 695 | UmsAfsAmGmAmUfUkUmGmGmUmGmUmCfUmAfUmUmUmCmCmsAmsCm |
| 696 | UmsAfsA(moe)GmAmUfUmUmGmGmUmGmUmCfUmAfUmUmUmCmCmsAmsCm |
| 697 | UmsAfsAmGmAmUfdTUmGmGmUmGmUmCfUmAfUmUmUmCmCmsAmsCm |
| 698 | VpUmsUfsGmUmGmAfAmUmAmCmC(moe)AmCmCfUmCfUmGmCmAmUmsGmsCm |
| 699 | VpUmsUfsGmUmGmAfAmUmAmCmCmAmCmCfUmCfUmGmCmAmUmsGmsCm |
| 700 | VpUmsUfsGmUmdGAmdAUmAmCmCmAmCmCfUmCfUmGmCmAmUmsGmsCm |
| 701 | UmsUfsGmUmGmAfAmUmAmCmCmAmCmCfUmCfUmGmCmAmUmsiGsiC |
| 702 | UmsUfsGmUmGmAfdAUmAmCmCmAmCmCfUmCfUmGmCmAmUmsGmsCm |
| 703 | UmsUfsGmUmGmAfAmUmAmCmCmAmCmCfUmCfUmGmCmAmUmsGmsiC |
| 704 | UmsUfsGmUmGmAfAmUmAmCmCmAmCmCfUmCfUmGmCmAmUmsGmsCm |
| 705 | UmsUfsGmUmGmAfAmUmAmCmCmAmCmCfUmCfUmGmCmAmUmsGmsCh₁₆ |
| 706 | UmsUfsGmUmGmAfAmUmAmCmCmAmCmCfUmCfUmGmCmAmUmsGmsCh₁₄ |
| 707 | UmsUfsGmUmGmAfAmUmAmCmCmAmCmCfUmCfUmGmCmAmUmsGmsCh₂₀ |
| 708 | UmsUfsGmUmGmAfAmUmAmCmCmAmCmCfUmCfUmGmCmAmUmsGh₁₀sCh₁₀ |
| 709 | UmsUfsGmUmGmAfAmUmAmCmCmAmCmCfUmCfUmGmCmAmUmsGmsCb |
| 710 | UmsUfsGmUmGmAfAmUmAmCmCmAmCmCfUmCfUmGmCmAmUmsGmsCh₁₂ |
| 711 | UmsUfsGmUmGmAfAmUmAmCmCmAmCmCfUmCfUmGmCmAmUmsGh₁₂sCh₁₂ |
| 712 | UmsUfsGmUmGmAfAmUmAmCmCmAmCmCfUmCfUmGmCmAmUmsGh₁₄sCh₁₄ |
| 713 | UmsUfsGmUmGmAfAmUmAmCmCmAmCmCfUmCfUmGmCmAmUmsGh₁₆sCh₁₆ |
| 714 | VpUmsUfsUmCmAmCfAmGmGmAmA(moe)UmGmUfUmUfUmAmUmUmGmsUmsCm |
| 715 | UmsUfsGmUmGmAfAmUmAmCmC(moe)AmCmCfUmCfUmGmCmAmUmsGmsCm |
| 716 | UmsUfsGmUmGmAfAkUmAmCmCmAmCmCfUmCfUmGmCmAmUmsGmsCm |
| 717 | UmsUfsGmUmdGAmdAUmAmCmCmAmCmCfUmCfUmGmCmAmUmsGmsCm |
| 718 | UmsUfsG(moe)UmGmAfAmUmAmCmCmAmCmCfUmCfUmGmCmAmUmsGmsCm |
| 719 | UmsUfsGmUmdGAfAmUmAmCmCmAmCmCfUmCfUmGmCmAmUmsGmsCm |
| 720 | VpUmsAfsGmGmAmGfUmAmGmGmGmGmCmUfCmAfGmCmAmGmGmsGmsCm |
| 721 | VpUmsAfsGmGmAmGfUmAmGmGmG(moe)GmCmUfCmAfGmCmAmGmGmsGmsCm |
| 722 | VpUmsAfsGmGmdAGmdTAmGmGmGmGmCmUfCmAfGmCmAmGmGmsGmsCm |
| 723 | UmsUfsGmUmGmAfAmUmAmCmCmAmCmCfUmCfUmGmCmAmUmsGbsCb |
| 724 | UmsAfsGmGmdAGmdTAmGmGmGmGmCmUfCmAfGmCmAmGmGmsGmsCm |
| 725 | UmsAfsGmGmdAGfUmAmGmGmGmGmCmUfCmAfGmCmAmGmGmsGmsCm |
| 726 | UmsAfsGmGmAmGfUmAmGmGmGmGmCmUfCmAfGmCmAmGmGmsGmsCm |
| 727 | UmsAfsGmGmAmGfUmAmGmGmGmGmCmUfCmAfGmCmAmGmGmsiGsiC |
| 728 | UmsAfsGmGmAmGfUmAmGmGmGmGmCmUfCmAfGmCmAmGmGmsGmsiC |
| 729 | UmsAfsGmGmAmGfUmAmGmGmGmGmCmUfCmAfGmCmAmGmGmsGmsCh₁₂ |
| 730 | UmsAfsGmGmAmGfUmAmGmGmGmGmCmUfCmAfGmCmAmGmGmsGmsCh₁₄ |
| 731 | UmsAfsGmGmAmGfUmAmGmGmGmGmCmUfCmAfGmCmAmGmGmsGmsCh₁₆ |
| 732 | UmsAfsGmGmAmGfUmAmGmGmGmGmCmUfCmAfGmCmAmGmGmsGmsCb |
| 733 | UmsAfsGmGmAmGfUmAmGmGmGmGmCmUfCmAfGmCmAmGmGmsGh₁₀sCh₁₀ |
| 734 | UmsAfsGmGmAmGfUmAmGmGmGmGmCmUfCmAfGmCmAmGmGmsGmsCh₂₀ |
| 735 | UmsAfsGmGmAmGfUmAmGmGmGmGmCmUfCmAfGmCmAmGmGmsGh₁₂sCh₁₂ |
| 736 | UmsAfsGmGmAmGfUmAmGmGmGmGmCmUfCmAfGmCmAmGmGmsGh₁₄sCh₁₄ |
| 737 | UmsAfsGmGmAmGfUmAmGmGmGmGmCmUfCmAfGmCmAmGmGmsGh₁₆sCh₁₆ |
| 738 | UmsAfsGmGmAmGfUmAmGmGmG(moe)GmCmUfCmAfGmCmAmGmGmsGmsCm |
| 739 | UmsAfsGmGmAmGfUkAmGmGmGmGmCmUfCmAfGmCmAmGmGmsGmsCm |
| 740 | UmsAfsGmGmAmGfUmAmGmGmGmGmCmUfCmAfGmCmAmGmGmsGbsCb |
| 741 | UmsAfsG(moe)GmAmGfUmAmGmGmGmGmCmUfCmAfGmCmAmGmGmsGmsCm |
| 742 | UmsAfsGmGmAmGfdTAmGmGmGmGmCmUfCmAfGmCmAmGmGmsGmsCm |
| 743 | VpUmsAfsGmGmUmGfAmAmAmAmCmAmCmUfGmCfUmUmUmAmGmsGmsGm |
| 745 | VpUmsAfsGmGmUmGfAmAmAmAmC(moe)AmCmUfGmCfUmUmUmAmGmsGmsGm |
| 746 | UmsAfsGmGmUmGfAmAmAmAmCmAmCmUfGmCfUmUmUmAmGmsGmsGm |
| 747 | UmsAfsGmGmUmGfAkAmAmAmCmAmCmUfGmCfUmUmUmAmGmsGmsGm |
| 748 | UmsUfsUmCmdACfAmGmGmAmAmUmGmUfUmUfUmAmUmUmGmsUmsCm |
| 749 | UmsAfsGmGmUmGfAmAmAmAmCmAmCmUfGmCfUmUmUmAmGmsGmsiG |
| 750 | UmsAfsGmGmUmGfAmAmAmAmCmAmCmUfGmCfUmUmUmAmGmsGmsGh₂₀ |
| 763 | UmsAfsGmGmUmGfdAAmAmAmCmAmCmUfGmCfUmUmUmAmGmsGmsGm |
| 764 | UmsAfsGmGmdTGfAmAmAmAmCmAmCmUfGmCfUmUmUmAmGmsGmsGm |
| 765 | UmsAfsGmGmUmGfAmAmAmAmCmAmCmUfGmCfUmUmUmAmGmsGmsGb |
| 766 | UmsAfsGmGmUmGfAmAmAmAmCmAmCmUfGmCfUmUmUmAmGmsGbsGb |
| 767 | UmsAfsGmGmUmGfAmAmAmAmC(moe)AmCmUfGmCfUmUmUmAmGmsGmsGm |
| 768 | UmsAfsG(moe)GmUmGfAmAmAmAmCmAmCmUfGmCfUmUmUmAmGmsGmsGm |
| 769 | UmsAfsGmGmUmGfAmAmAmAmCmAmCmUfGmCfUmUmUmAmGmsGh₁₂sGh₁₂ |
| 770 | UmsAfsGmGmUmGfAmAmAmAmCmAmCmUfGmCfUmUmUmAmGmsGh₁₄sGh₁₄ |
| 771 | UmsAfsGmGmUmGfAmAmAmAmCmAmCmUfGmCfUmUmUmAmGmsGh₁₆sGh₁₆ |
| 772 | VpUmsAfsGmGmdTGmdAAmAmAmCmAmCmUfGmCfUmUmUmAmGmsGmsGm |
| 773 | UmsAfsGmGmUmGfAmAmAmAmCmAmCmUfGmCfUmUmUmAmGmsGmsGh₁₄ |
| 774 | VpUmsUfsAmUmGmAfGmGmUmGmAmAmAmAfCmAfCmUmGmCmUmsGmsGm |
| 775 | AmsUfsA(moe)UmGmAfGmGmUmGmAmAmAmAfCmAfCmUmGmCmUmsGmsGm |
| 776 | AmsUfsAmUmGmAfGkGmUmGmAmAmAmAfCmAfCmUmGmCmUmsGmsGm |
| 777 | AmsUfsAmUmGmAfGmGmUmGmAmAmAmAfCmAfCmUmGmCmUmsGh₁₀sGh₁₀ |
| 778 | VpUmsUfsAmUmGmAfGmGmUmGmA(moe)AmAmAfCmAfCmUmGmCmUmsGmsGm |
| 779 | AmsUfsAmUmGmAfGmGmUmGmAmAmAmAfCmAfCmUmGmCmUmsiGsiG |
| 780 | AmsUfsAmUmGmAfGmGmUmGmAmAmAmAfCmAfCmUmGmCmUmsGmsGh₂₀ |
| 781 | AmsUfsAmUmdGAfGmGmUmGmAmAmAmAfCmAfCmUmGmCmUmsGmsGm |
| 782 | AmsUfsAmUmGmAfdGGmUmGmAmAmAmAfCmAfCmUmGmCmUmsGmsGm |
| 783 | AmsUfsAmUmGmAfGmGmUmGmAmAmAmAfCmAfCmUmGmCmUmsGmsiG |
| 784 | AmsUfsAmUmGmAfGmGmUmGmAmAmAmAfCmAfCmUmGmCmUmsGmsGb |
| 785 | AmsUfsAmUmGmAfGmGmUmGmA(moe)AmAmAfCmAfCmUmGmCmUmsGmsTb |
| 786 | AmsUfsAmUmGmAfGmGmUmGmAmAmAmAfCmAfCmUmGmCmUmsGbsGb |
| 787 | AmsUfsAmUmGmAfGmGmUmGmA(moe)AmAmAfCmAfCmUmGmCmUmsTbsTb |
| 788 | AmsUfsAmUmGmAfGmGmUmGmAmAmAmAfCmAfCmUmGmCmUmsGh₁₂sGh₁₂ |
| 789 | AmsUfsAmUmGmAfGmGmUmGmAmAmAmAfCmAfCmUmGmCmUmsGh₁₄sGh₁₄ |
| 790 | AmsUfsAmUmGmAfGmGmUmGmAmAmAmAfCmAfCmUmGmCmUmsGh₁₆sGh₁₆ |
| 791 | AmsUfsAmUmGmAfGmGmUmGmAmAmAmAfCmAfCmUmGmCmUmsGmsGh₁₂ |
| 792 | AmsUfsAmUmGmAfGmGmUmGmAmAmAmAfCmAfCmUmGmCmUmsGmsGh₁₄ |
| 793 | AmsUfsAmUmGmAfGmGmUmGmAmAmAmAfCmAfCmUmGmCmUmsGmsGh₁₆ |
| 794 | VpUmsUfsUmUmdAUmdTGmUmCmUmCmUmGfCmCfUmGmGmAmCmsUmsUm |
| 795 | UmsUfsUmUmAmUfdTGmUmCmUmCmUmGfCmCfUmGmGmAmCmsUmsUm |
| 796 | VpUmsUfsUmUmAmUfUmGmUmCmUmCmUmGfCmCfUmGmGmAmCmsUmsUm |
| 797 | UmsUfsUmUmdAUmdTGmUmCmUmCmUmGfCmCfUmGmGmAmCmsUmsUm |
| 798 | UmsUfsUmUmdAUfUmGmUmCmUmCmUmGfCmCfUmGmGmAmCmsUmsUm |
| 799 | UmsUfsUmUmAmUfUmGmUmCmUmCmUmGfCmCfUmGmGmAmCmUmsiU |
| 800 | UmsUfsUmUmAmUfUmGmUmCmUmCmUmGfCmCfUmGmGmAmCmsUmsUm |
| 801 | UmsUfsUmUmAmUfUmGmUmCmUmCmUmGfCmCfUmGmGmAmCmsUmsUh₁₆ |
| 802 | UmsUfsUmUmAmUfUmGmUmCmUmCmUmGfCmCfUmGmGmAmCmsUmsUh₁₄ |
| 803 | UmsUfsUmUmAmUfUmGmUmCmUmCmUmGfCmCfUmGmGmAmCmsUmsUh₁₂ |
| 804 | UmsUfsUmUmAmUfUmGmUmCmUmCmUmGfCmCfUmGmGmAmCmsUmsUh₂₀ |
| 805 | UmsUfsUmUmAmUfUmGmUmCmUmCmUmGfCmCfUmGmGmAmCmsUmsTb |
| 806 | UmsUfsUmUmAmUfUmGmUmCmUmCmUmGfCmCfUmGmGmAmCmsUh₁₀sUh₁₀ |
| 807 | UmsUfsUmUmAmUfUmGmUmCmUmCmUmGfCmCfUmGmGmAmCmsUh₁₆sUh₁₆ |
| 808 | UmsUfsUmUmAmUfUmGmUmCmUmCmUmGfCmCfUmGmGmAmCmsUh₁₄sUh₁₄ |
| 809 | UmsUfsUmUmAmUfUmGmUmCmUmCmUmGfCmCfUmGmGmAmCmsUh₁₂sUh₁₂ |
| 810 | UmsUfsUmUmAmUfUmGmUmCmUmCmUmGfCmCfUmGmGmAmCmsTbsTb |
| 811 | UmsUfsUmUmAmUfUmGmUmCmUmCmUmGfCmCfUmGmGmAmCmsiUsiU |
| 812 | UmsUfsUmUmAmUfUmGmUmCmU(moe)CmUmGfCmCfUmGmGmAmCmsUmsUm |
| 813 | UmsUfsUmUmAmUfUkGmUmCmUmCmUmGfCmCfUmGmGmAmCmsUmsUm |
| 814 | AmsAfsUmGmUmUfUmUmAmUmUmGmUmCfUmCfUmGmCmCmUmsGmsGm |
| 815 | AmsAfsU(moe)GmUmUfUmUmAmUmUmGmUmCfUmCfUmGmCmCmUmsGmsGm |
| 816 | AmsAfsUmGmUmUfUkUmAmUmUmGmUmCfUmCfUmGmCmCmUmsGmsGm |
| 817 | AmsAfsUmGmUmUfUmUmAmUmUmGmUmCfUmCfUmGmCmCmUmsGh₂₀sGh₂₀ |
| 818 | AmsAfsUmGmUmUfUmUmAmUmUmGmUmCfUmCfUmGmCmCmUmsGmsGh₁₀ |
| 819 | AmsAfsUmGmUmUfUmUmAmUmUmGmUmCfUmCfUmGmCmCmUmsiGsiG |
| 820 | AmsAfsUmGmUmUfUmUmAmUmUmGmUmCfUmCfUmGmCmCmUmsGmsiG |
| 821 | VpUmsAfsUmGmUmUfUmUmAmUmUmGmUmCfUmCfUmGmCmCmUmsGmsGm |
| 822 | VpUmsAfsUmGmUmUfUmUmAmUmU(moe)GmUmCfUmCfUmGmCmCmUmsGmsGm |
| 823 | AmsAfsUmGmdTUfUmUmAmUmUmGmUmCfUmCfUmGmCmCmUmsGmsGm |
| 824 | AmsAfsUmGmdTUmdTUmAmUmUmGmUmCfUmCfUmGmCmCmUmsGmsGm |
| 825 | VpUmsAfsUmGmdTUmdTUmAmUmUmGmUmCfUmCfUmGmCmCmUmsGmsGm |
| 826 | AmsAfsUmGmUmUfUmUmAmUmUmGmUmCfUmCfUmGmCmCmUmsGh₁₂sGh₁₂ |
| 827 | AmsAfsUmGmUmUfUmUmAmUmUmGmUmCfUmCfUmGmCmCmUmsGbsGb |
| 828 | AmsAfsUmGmUmUfUmUmAmUmUmGmUmCfUmCfUmGmCmCmUmsGh₁₄sGh₁₄ |
| 829 | AmsAfsUmGmUmUfUmUmAmUmUmGmUmCfUmCfUmGmCmCmUmsGh₁₆sGh₁₆ |
| 830 | AmsAfsUmGmUmUfUmUmAmUmUmGmUmCfUmCfUmGmCmCmUmsGmsGb |
| 831 | AmsAfsUmGmUmUfUmUmAmUmUmGmUmCfUmCfUmGmCmCmUmsGmsGh₁₂ |
| 832 | AmsAfsUmGmUmUfUmUmAmUmUmGmUmCfUmCfUmGmCmCmUmsGmsGh₁₄ |
| 833 | AmsAfsUmGmUmUfUmUmAmUmUmGmUmCfUmCfUmGmCmCmUmsGmsGh₁₆ |
| 834 | VpUmsAfsGmGmAmAfUmGmUmUmU(moe)UmAmUfUmGfUmCmUmCmUmsGmsCm |
| 835 | VpUmsAfsGmGmdAAmdTGmUmUmUmUmAmUfUmGfUmCmUmCmUmsGmsCm |
| 836 | VpUmsAfsGmGmAmAfUmGmUmUmUmUmAmUfUmGfUmCmUmCmUmsGmsCm |
| 837 | UmsAfsGmGmdAAmdTGmUmUmUmUmAmUfUmGfUmCmUmCmUmsGmsCm |
| 838 | UmsAfsGmGmdAAfUmGmUmUmUmUmAmUfUmGfUmCmUmCmUmsGmsCm |
| 839 | UmsAfsGmGmAmAfUmGmUmUmUmUmAmUfUmGfUmCmUmCmUmsGmsCh₁₆ |
| 840 | UmsAfsGmGmAmAfUmGmUmUmUmUmAmUfUmGfUmCmUmCmUmsiGsiC |
| 841 | UmsAfsGmGmAmAfUmGmUmUmUmUmAmUfUmGfUmCmUmCmUmsGmsCm |
| 842 | UmsAfsGmGmAmAfUmGmUmUmUmUmAmUfUmGfUmCmUmCmUmsGmsCh₁₄ |
| 843 | UmsAfsGmGmAmAfUmGmUmUmUmUmAmUfUmGfUmCmUmCmUmsGmsCh₁₂ |
| 844 | UmsAfsGmGmAmAfUmGmUmUmUmUmAmUfUmGfUmCmUmCmUmsGmsCh₂₀ |
| 845 | UmsAfsGmGmAmAfUmGmUmUmUmUmAmUfUmGfUmCmUmCmUmsGh₁₀sCh₁₀ |
| 846 | UmsAfsGmGmAmAfUmGmUmUmUmUmAmUfUmGfUmCmUmCmUmsGmsCb |
| 847 | UmsAfsGmGmAmAfUmGmUmUmUmUmAmUfUmGfUmCmUmCmUmsGh₁₂sCh₁₂ |
| 848 | UmsAfsGmGmAmAfUmGmUmUmUmUmAmUfUmGfUmCmUmCmUmsGh₁₄sCh₁₄ |
| 849 | UmsAfsGmGmAmAfUmGmUmUmUmUmAmUfUmGfUmCmUmCmUmsGh₁₆sCh₁₆ |
| 850 | UmsAfsGmGmAmAfUmGmUmUmUmUmAmUfUmGfUmCmUmCmUmsGbsCb |
| 851 | UmsAfsGmGmAmAfUmGmUmUmUmUmAmUfUmGfUmCmUmCmUmGmsiC |
| 852 | UmsAfsGmGmAmAfUmGmUmUmU(moe)UmAmUfUmGfUmCmUmCmUmsGmsCm |
| 853 | UmsAfsG(moe)GmAmAfUmGmUmUmUmUmAmUfUmGfUmCmUmCmUmsGmsCm |
| 854 | UmsAfsGmGmAmAfUkGmUmUmUmUmAmUfUmGfUmCmUmCmUmsGmsCm |
| 855 | UmsAfsGmGmAmAfdTGmUmUmUmUmAmUfUmGfUmCmUmCmUmsGmsCm |
| 856 | UmsUfsU(moe)CmAmCfAmGmGmAmAmUmGmUfUmUfUmAmUmUmGmsUmsCm |
| 857 | UmsUfsUmCmAmCfAkGmGmAmAmUmGmUfUmUfUmAmUmUmGmsUmsCm |
| 858 | UmsUfsUmCmAmCfdAGmGmAmAmUmGmUfUmUfUmAmUmUmGmsUmsCm |
| 859 | UmsUfsUmCmAmCfAmGmGmAmA(moe)UmGmUfUmUfUmAmUmUmGmsUmsCm |
| 860 | VpUmsUfsUmCmAmCfAmGmGmAmAmUmGmUfUmUfUmAmUmUmGmsUmsCm |
| 861 | UmsUfsUmCmAmCfAmGmGmAmAmUmGmUfUmUfUmAmUmUmGmsTbsCb |
| 862 | UmsUfsUmCmAmCfAmGmGmAmAmUmGmUfUmUfUmAmUmUmGmsiUsiC |
| 863 | UmsUfsUmCmAmCfAmGmGmAmAmUmGmUfUmUfUmAmUmUmGmsUh₁₂sCh₁₂ |
| 864 | UmsUfsUmCmAmCfAmGmGmAmAmUmGmUfUmUfUmAmUmUmGmsUh₁₄sCh₁₄ |
| 865 | VpUmsUfsUmCmdACmdAGmGmAmAmUmGmUfUmUfUmAmUmUmGmsUmsCm |
| 866 | UmsUfsUmCmAmCfAmGmGmAmAmUmGmUfUmUfUmAmUmUmGmsUh₁₆sCh₁₆ |
| 867 | UmsUfsUmCmAmCfAmGmGmAmAmUmGmUfUmUfUmAmUmUmGmsUh₁₀sCh₁₀ |
| 868 | UmsUfsUmCmAmCfAmGmGmAmAmUmGmUfUmUfUmAmUmUmGmsUmsCb |
| 869 | UmsUfsUmCmAmCfAmGmGmAmAmUmGmUfUmUfUmAmUmUmGmsUmsCh₁₆ |
| 870 | UmsUfsUmCmAmCfAmGmGmAmAmUmGmUfUmUfUmAmUmUmGmsUmsCh₁₄ |
| 871 | UmsUfsUmCmAmCfAmGmGmAmAmUmGmUfUmUfUmAmUmUmGmsUmsCh₁₂ |
| 872 | UmsUfsUmCmAmCfAmGmGmAmAmUmGmUfUmUfUmAmUmUmGmsUmsCh₂₀ |
| 873 | UmsUfsUmCmAmCfAmGmGmAmAmUmGmUfUmUfUmAmUmUmGmsUmsCm |
| 874 | UmsUfsUmCmAmCfAmGmGmAmAmUmGmUfUmUfUmAmUmUmGmsUmsiC |
| 875 | UmsUfsUmCmdACmdAGmGmAmAmUmGmUfUmUfUmAmUmUmGmsUmsCm |
| 877 | UmsCfsUmUmGmGfUmUmAfCmAmUmGmAfAmAfUmCmCmCmAmsUmsCm |
| 878 | UmsAfsGmGmUmGfAmAmAmAmCmAmCmUfGmCfUmUmUmAmGmsGmsGh₁₆ |
| 879 | UmsAfsGmGmUmGfAmAmAmAmCmAmCmUfGmCfUmUmUmAmGmsGh₁₀sGh₁₀ |
| 880 | UmsAfsGmGmUmGfAmAmAmAmCmAmCmUfGmCfUmUmUmAmGmsGmsGh₁₂ |
| 881 | AmsUfsAmUmGmAfGmGmUmGmA(moe)AmAmAfCmAfCmUmGmCmUmsGmsGm |
| 882 | UmsUfsU(moe)UmAmUfUmGmUmCmUmCmUmGfCmCfUmGmGmAmCmsUmsUm |
| 883 | VpUmsUfsUmUmAmUfUmGmUmCmU(moe)CmUmGfCmCfUmGmGmAmCmsUmsUm |
| 889 | AmsUfsAmUmdGAmdGGmUmGmAmAmAmAfCmAfCmUmGmCmUmsUmsUm |
| 890 | AmsUfsAmUmdGAmdGGmUmGmAmAmAmAfCmAfCmUmGmCmUmsAmsGm |
| 891 | AmsUfsAmUmdGAmdGGmUmGmAmAmAmAfCmAfCmUmGmCmUmsAmsUm |
| 892 | AmsUfsAmUmdGAmdGGmUmGmAmAmAmAfCmAfCmUmGmCmCmsAmsGm |
| 893 | AmsUfsAmUmdGAmdGGmUmGmAmAmAmAfCmAfCmUmGmCmGmsAmsGm |
| 894 | AmsUfsAmUmdGAmdGGmUmGmAmAmAmAfCmAfCmUmGmCmCmsGmsGm |
| 895 | AmsUfsAmUmdGAmdGGmUmGmAmAmAmAfCmAfCmUmGmCmGmsGmsGm |
| 896 | UmsAfsGmGmdTGmdAAmAmAmCmAmCmUfGmCfUmUmUmAmGmsAmsGm |
| 897 | UmsAfsGmGmdTGmdAAmAmAmCmAmCmUfGmCfUmUmUmAmGmsUmsGm |
| 898 | UmsAfsGmGmdTGmdAAmAmAmCmAmCmUfGmCfUmUmUmAmCmsGmsGm |
| 899 | UmsAfsGmGmdTGmdAAmAmAmCmAmCmUfGmCfUmUmUmAmCmsAmsGm |
| 900 | UmsAfsGmGmdTGmdAAmAmAmCmAmCmUfGmCfUmUmUmAmUmsGmsGm |
| 901 | UmsAfsGmGmdTGmdAAmAmAmCmAmCmUfGmCfUmUmUmAmUmsAmsGm |
| 902 | AmsUfsAmUmdGAmdGGmUmGmAmAmAmAfCmAfCmUmGmsCmsUm |
| 903 | AmsUfsAmUmdGAmdGGmUmGmAmAmAmAfCmAfCmUmGmsAmsGm |
| 904 | AmsUfsAmUmdGAmdGGmUmGmAmAmAmAfCmAfCmUmUmsAmsGm |
| 905 | UmsAfsGmGmdTGmdAAmAmAmCmAmCmUfGmCfUmUmUmsAmsGm |
| 906 | UmsAfsGmGmdTGmdAAmAmAmCmAmCmUfGmCfUmUmUmsGmsGm |

**Table 7**

| SEQ ID NO | Modified sense strand (5'-3') |
|---|---|
| 471 | UmsCmsCmAmAmGmUfGmUfCfCfUmCmUmGmAmUmGmGmUmAm |
| 472 | UmsCmsCmAmAmGmUfGmdTCfCfUmCmUmGmAmUmGmGmUmAm |
| 473 | UhsCmsCmAmAmGmUfGmUfCfCfUmCmUmGmAmUmGmGmUmAm |
| 474 | Uh₁₆sCmsCmAmAmGmUfGmUfCfCfUmCmUmGmAmUmGmGmUmAm |
| 475 | Uh₁₄sCmsCmAmAmGmUfGmUfCfCfUmCmUmGmAmUmGmGmUmAm |
| 476 | Uh₁₂sCmsCmAmAmGmUfGmUfCfCfUmCmUmGmAmUmGmGmUmAm |
| 477 | TbsCmsCmAmAmGmUfGmUfCfCfUmCmUmGmAmUmGmGmUmAm |
| 478 | iUsCmsCmAmAmGmUfGmUfCfCfUmCmUmGmAmUmGmGmUmAm |
| 479 | iCsCmsUmCmUmGmAfUmGfGfUfCmAmAmAmGmUmUmCmUmAm |
| 480 | CmsCmsUmCmUmGmAfUmGfGfUfCmAmAmAmGmUmUmCmUmAm |
| 481 | CmsCmsUmCmUmGmAfUmdGGfUfCmAmAmAmGmUmUmCmUmAm |
| 482 | ChsCmsUmCmUmGmAfUmGfGfUfCmAmAmAmGmUmUmCmUmAm |
| 483 | Ch₁₆sCmsUmCmUmGmAfUmGfGfUfCmAmAmAmGmUmUmCmUmAm |
| 484 | Ch₁₄sCmsUmCmUmGmAfUmGfGfUfCmAmAmAmGmUmUmCmUmAm |
| 485 | Ch₁₂sCmsUmCmUmGmAfUmGfGfUfCmAmAmAmGmUmUmCmUmAm |
| 486 | CbsCmsUmCmUmGmAfUmGfGfUfCmAmAmAmGmUmUmCmUmAm |
| 487 | iCsUmsGmAmUmGmGfUmCfAfAfAmGmUmUmCmUmAmGmAmUm |
| 488 | iCsUmsGmAmUmGmGfUmCfAfAfAmGmUmUmCmUmAmGmAmAm |
| 489 | CmsUmsGmAmUmGmGfUmdCAfAfAmGmUmUmCmUmAmGmAmUm |
| 490 | CmsUmsGmAmUmGmGfUmCfAfAfAmGmUmUmCmUmAmGmAmUm |
| 491 | CmsUmsGmAmUmGmGfUmCfAfAfAmGmUmUmCmUmAmGmAmAm |
| 492 | ChsUmsGmAmUmGmGfUmCfAfAfAmGmUmUmCmUmAmGmAmUm |
| 493 | ChsUmsGmAmUmGmGfUmCfAfAfAmGmUmUmCmUmAmGmAmAm |
| 494 | Ch₁₆sUmsGmAmUmGmGfUmCfAfAfAmGmUmUmCmUmAmGmAmUm |
| 495 | Ch₁₆sUmsGmAmUmGmGfUmCfAfAfAmGmUmUmCmUmAmGmAmAm |
| 496 | Ch₁₄sUmsGmAmUmGmGfUmCfAfAfAmGmUmUmCmUmAmGmAmUm |
| 497 | Ch₁₄sUmsGmAmUmGmGfUmCfAfAfAmGmUmUmCmUmAmGmAmAm |
| 498 | Ch₁₂sUmsGmAmUmGmGfUmCfAfAfAmGmUmUmCmUmAmGmAmUm |
| 499 | Ch₁₂sUmsGmAmUmGmGfUmCfAfAfAmGmUmUmCmUmAmGmAmAm |
| 500 | CbsUmsGmAmUmGmGfUmCfAfAfAmGmUmUmCmUmAmGmAmUm |
| 501 | iGsAmsUmAmUmAmCfAmAfAfGfUmGmGmAmAmAmUmAmGmAm |
| 502 | GmsAmsUmAmUmAmCfAmdAAfGfUmGmGmAmAmAmUmAmGmAm |
| 503 | GmsAmsUmAmUmAmCfAmAfAfGfUmGmGmAmAmAmUmAmGmAm |
| 504 | GhsAmsUmAmUmAmCfAmAfAfGfUmGmGmAmAmAmUmAmGmAm |
| 505 | Gh₁₆sAmsUmAmUmAmCfAmAfAfGfUmGmGmAmAmAmUmAmGmAm |
| 506 | Gh₁₄sAmsUmAmUmAmCfAmAfAfGfUmGmGmAmAmAmUmAmGmAm |
| 507 | Gh₁₂sAmsUmAmUmAmCfAmAfAfGfUmGmGmAmAmAmUmAmGmAm |
| 508 | GbsAmsUmAmUmAmCfAmAfAfGfUmGmGmAmAmAmUmAmGmAm |
| 509 | iGsGmsAmAmAmUmAfGmAfCfAfCmCmAmAmAmUmCmUmUmAm |
| 510 | GmsGmsAmAmAmUmAfGmdACfAfCmCmAmAmAmUmCmUmUmAm |
| 511 | GbsGmsAmAmAmUmAfGmAfCfAfCmCmAmAmAmUmCmUmUmAm |
| 512 | GmsGmsAmAmAmUmAfGmAfCfAfCmCmAmAmAmUmCmUmUmAm |
| 513 | GhsGmsAmAmAmUmAfGmAfCfAfCmCmAmAmAmUmCmUmUmAm |
| 514 | Gh₁₆sGmsAmAmAmUmAfGmAfCfAfCmCmAmAmAmUmCmUmUmAm |
| 515 | Gh₁₄sGmsAmAmAmUmAfGmAfCfAfCmCmAmAmAmUmCmUmUmAm |
| 516 | Gh₁₂sGmsAmAmAmUmAfGmAfCfAfCmCmAmAmAmUmCmUmUmAm |
| 517 | iAsUmsGmCmAmGmAfGmGfUfGfGmUmAmUmUmCmAmCmAmAm |
| 518 | AmsUmsGmCmAmGmAfGmGfUfGfGmUmAmUmUmCmAmCmAmAm |
| 519 | AmsUmsGmCmAmGmAfGmdGUfGfGmUmAmUmUmCmAmCmAmAm |
| 520 | AhsUmsGmCmAmGmAfGmGfUfGfGmUmAmUmUmCmAmCmAmAm |
| 521 | Ah₁₆sUmsGmCmAmGmAfGmGfUfGfGmUmAmUmUmCmAmCmAmAm |
| 522 | Ah₁₄sUmsGmCmAmGmAfGmGfUfGfGmUmAmUmUmCmAmCmAmAm |
| 523 | Ah₁₂sUmsGmCmAmGmAfGmGfUfGfGmUmAmUmUmCmAmCmAmAm |
| 524 | AbsUmsGmCmAmGmAfGmGfUfGfGmUmAmUmUmCmAmCmAmAm |
| 525 | iCsCmsUmGmCmUmGfAmGfCfCfCmCmUmAmCmUmCmCmUmAm |
| 526 | CmsCmsUmGmCmUmGfAmGfCfCfCmCmUmAmCmUmCmCmUmAm |
| 527 | CmsCmsUmGmCmUmGfAmdGCfCfCmCmUmAmCmUmCmCmUmAm |
| 528 | ChsCmsUmGmCmUmGfAmGfCfCfCmCmUmAmCmUmCmCmUmAm |
| 529 | Ch₁₆sCmsUmGmCmUmGfAmGfCfCfCmCmUmAmCmUmCmCmUmAm |
| 530 | Ch₁₄sCmsUmGmCmUmGfAmGfCfCfCmCmUmAmCmUmCmCmUmAm |
| 531 | Ch₁₂sCmsUmGmCmUmGfAmGfCfCfCmCmUmAmCmUmCmCmUmAm |
| 532 | CbsCmsUmGmCmUmGfAmGfCfCfCmCmUmAmCmUmCmCmUmAm |
| 533 | iCsUmsAmAmAmGmCfAmdGUfGfUmUmUmUmCmAmCmCmUmAm |
| 534 | CmsUmsAmAmAmGmCfAmGfUfGfUmUmUmUmCmAmCmCmUmAm |
| 535 | CmsUmsAmAmAmGmCfAmdGUfGfUmUmUmUmCmAmCmCmUmAm |
| 536 | CbsUmsAmAmAmGmCfAmGfUfGfUmUmUmUmCmAmCmCmUmAm |
| 537 | iAsGmsCmAmGmUmGfUmUfUfUfCmAmCmCmUmCmAmUmAmUm |
| 538 | iAsGmsCmAmGmUmGfUmUfUfUfCmAmCmCmUmCmAmUmAmAm |
| 539 | AmsGmsCmAmGmUmGfUmUfUfUfCmAmCmCmUmCmAmUmAmAm |
| 540 | AmsGmsCmAmGmUmGfUmdTUfUfCmAmCmCmUmCmAmUmAmUm |
| 541 | AbsGmsCmAmGmUmGfUmUfUfUfCmAmCmCmUmCmAmUmAmUm |
| 542 | iGsUmsCmCmAmGmGfCmAfGfAfGmAmCmAmAmUmAmAmAmAm |
| 543 | GmsUmsCmCmAmGmGfCmdAGfAfGmAmCmAmAmUmAmAmAmAm |
| 544 | GmsUmsCmCmAmGmGfCmAfGfAfGmAmCmAmAmUmAmAmAmAm |
| 545 | GhsUmsCmCmAmGmGfCmAfGfAfGmAmCmAmAmUmAmAmAmAm |
| 546 | Gh₁₆sUmsCmCmAmGmGfCmAfGfAfGmAmCmAmAmUmAmAmAmAm |
| 547 | Gh₁₄sUmsCmCmAmGmGfCmAfGfAfGmAmCmAmAmUmAmAmAmAm |
| 548 | Gh₁₂sUmsCmCmAmGmGfCmAfGfAfGmAmCmAmAmUmAmAmAmAm |
| 549 | GbsUmsCmCmAmGmGfCmAfGfAfGmAmCmAmAmUmAmAmAmAm |
| 550 | iAsGmsGmCmAmGmAfGmAfCfAfAmUmAmAmAmAmCmAmUmUm |
| 551 | iAsGmsGmCmAmGmAfGmAfCfAfAmUmAmAmAmAmCmAmUmAm |
| 552 | AmsGmsGmCmAmGmAfGmdACfAfAmUmAmAmAmAmCmAmUmUm |
| 553 | AmsGmsGmCmAmGmAfGmAfCfAfAmUmAmAmAmAmCmAmUmAm |
| 554 | AhsGmsGmCmAmGmAfGmAfCfAfAmUmAmAmAmAmCmAmUmAm |
| 555 | Ah₁₆sGmsGmCmAmGmAfGmAfCfAfAmUmAmAmAmAmCmAmUmAm |
| 556 | Ah₁₄sGmsGmCmAmGmAfGmAfCfAfAmUmAmAmAmAmCmAmUmAm |
| 557 | Ah₁₂sGmsGmCmAmGmAfGmAfCfAfAmUmAmAmAmAmCmAmUmAm |
| 558 | AbsGmsGmCmAmGmAfGmAfCfAfAmUmAmAmAmAmCmAmUmUm |
| 559 | iAsGmsAmGmAmCmAfAmUfAfAfAmAmCmAmUmUmCmCmUmAm |
| 560 | AmsGmsAmGmAmCmAfAmUfAfAfAmAmCmAmUmUmCmCmUmAm |
| 561 | AmsGmsAmGmAmCmAfAmdTAfAfAmAmCmAmUmUmCmCmUmAm |
| 562 | AhsGmsAmGmAmCmAfAmUfAfAfAmAmCmAmUmUmCmCmUmAm |
| 563 | Ah₁₆sGmsAmGmAmCmAfAmUfAfAfAmAmCmAmUmUmCmCmUmAm |
| 564 | Ah₁₄sGmsAmGmAmCmAfAmUfAfAfAmAmCmAmUmUmCmCmUmAm |
| 565 | Ah₁₂sGmsAmGmAmCmAfAmUfAfAfAmAmCmAmUmUmCmCmUmAm |
| 566 | AbsGmsAmGmAmCmAfAmUfAfAfAmAmCmAmUmUmCmCmUmAm |
| 567 | iCsAmsAmUmAmAmAfAmCfAfUfUmCmCmUmGmUmGmAmAmAm |
| 568 | CmsAmsAmUmAmAmAfAmdCAfUfUmCmCmUmGmUmGmAmAmAm |
| 569 | CmsAmsAmUmAmAmAfAmCfAfUfUmCmCmUmGmUmGmAmAmAm |
| 570 | ChsAmsAmUmAmAmAfAmCfAfUfUmCmCmUmGmUmGmAmAmAm |
| 571 | Ch₁₆sAmsAmUmAmAmAfAmCfAfUfUmCmCmUmGmUmGmAmAmAm |
| 572 | Ch₁₄sAmsAmUmAmAmAfAmCfAfUfUmCmCmUmGmUmGmAmAmAm |
| 573 | Ch₁₂sAmsAmUmAmAmAfAmCfAfUfUmCmCmUmGmUmGmAmAmAm |
| 574 | CbsAmsAmUmAmAmAfAmCfAfUfUmCmCmUmGmUmGmAmAmAm |
| 601 | ChsUmsAmAmAmGmCfAmGfUfGfUmUmUmUmCmAmCmCmUmAm |
| 603 | iCsUmsAmAmAmGmCfAmGfUfGfUmUmUmUmCmAmCmCmUmAm |
| 605 | AmsGmsCmAmGmUmGfUmUfUfUfCmAmCmCmUmCmAmUmAmUm |
| 617 | AhsGmsCmAmGmUmGfUmUfUfUfCmAmCmCmUmCmAmUmAmUm |
| 641 | AmsGmsGmCmAmGmAfGmAfCfAfAmUmAmAmAmAmCmAmUmUm |
| 653 | AhsGmsGmCmAmGmAfGmAfCfAfAmUmAmAmAmAmCmAmUmUm |
| 744 | AhsGmsCmAmGmUmGfUmUfUfUfCmAmCmCmUmCmAmUmAmAm |
| 751 | Ah₁₂sGmsCmAmGmUmGfUmUfUfUfCmAmCmCmUmCmAmUmAmUm |
| 752 | Ah₁₄sGmsCmAmGmUmGfUmUfUfUfCmAmCmCmUmCmAmUmAmUm |
| 753 | Ah₁₂sGmsCmAmGmUmGfUmUfUfUfCmAmCmCmUmCmAmUmAmAm |
| 754 | Ah₁₄sGmsCmAmGmUmGfUmUfUfUfCmAmCmCmUmCmAmUmAmAm |
| 755 | Ch₁₄sUmsAmAmAmGmCfAmGfUfGfUmUmUmUmCmAmCmCmUmAm |
| 756 | Ch₁₂sUmsAmAmAmGmCfAmGfUfGfUmUmUmUmCmAmCmCmUmAm |
| 757 | Ah₁₂sGmsGmCmAmGmAfGmAfCfAfAmUmAmAmAmAmCmAmUmUm |
| 758 | Ah₁₄sGmsGmCmAmGmAfGmAfCfAfAmUmAmAmAmAmCmAmUmUm |
| 759 | Ah₁₆sGmsGmCmAmGmAfGmAfCfAfAmUmAmAmAmAmCmAmUmUm |
| 760 | Ah₁₆sGmsCmAmGmUmGfUmUfUfUfCmAmCmCmUmCmAmUmAmUm |
| 761 | Ah₁₆sGmsCmAmGmUmGfUmUfUfUfCmAmCmCmUmCmAmUmAmAm |
| 762 | Ch₁₆sUmsAmAmAmGmCfAmGfUfGfUmUmUmUmCmAmCmCmUmAm |
| 876 | UmsGmsGmGmAmUmUfUmCfAfUfGmUmAmAmCmCmAmAmGmAm |
| 907 | Gh12sGmsCmAmGmUmGfUmUfUfUfCmAmCmCmUmCmAmUmAmUm |
| 908 | Ch12sGmsCmAmGmUmGfUmUfUfUfCmAmCmCmUmCmAmUmAmUm |
| 909 | Gh12sUmsAmAmAmGmCfAmGfUfGfUmUmUmUmCmAmCmCmUmAm |
| 910 | Ah12sUmsAmAmAmGmCfAmGfUfGfUmUmUmUmCmAmCmCmUmAm |
| 911 | CmsAmsGmUmGfUmUfUfUfCmAmCmCmUmCmAmUmAmUm |
| 912 | AmsAmsGmUmGfUmUfUfUfCmAmCmCmUmCmAmUmAmUm |
| 913 | AmsAmsAmGmCfAmGfUfGfUmUmUmUmCmAmCmCmUmAm |

In Table 6 and Table 7, the specific meanings of the various symbols in the sequences are shown in Table 8.

**Table 8**

| Symbol | Meaning |
|---|---|
| G | Representing a ribonucleotide containing guanine as a base |
| C | Representing a ribonucleotide containing cytosine as a base |
| A | Representing a ribonucleotide containing adenine as a base |
| U | Representing a ribonucleotide containing uracil as a base |
| T | Representing a deoxyribonucleotide containing thymine as a base |
| m | Representing that one nucleotide adjacent to the letter m on the left side is a 2'-O-methyl-modified nucleotide |
| f | Representing that one nucleotide adjacent to the letter f on the left side is a 2'-fluoro-modified nucleotide |
| k | Representing that one nucleotide adjacent to the letter k on the left side is a UNA-modified nucleotide |
| moe | Representing that one nucleotide adjacent to the letter moe on the left side is a 2'-O-methoxyethyl-modified nucleotide |
| h | Representing that one nucleotide adjacent to the letter h on the left side is a 4'-modified threose nucleic acid, with the subscript of h being the number of Cs in R |
| b | Representing that one nucleotide adjacent to the letter b on the left side is an L-2'-deoxyribonucleotide |
| i | Representing that one nucleotide adjacent to the letter i on the right side is a reverse nucleotide |
| d | Representing that one nucleotide adjacent to the letter d on the right side is a 2'-deoxyribonucleotide |
| Vp | Representing that one nucleotide adjacent to the letter Vp on the right side is a 5'-vinylphosphonate nucleotide |
| VpXm | Representing that nucleotide X between Vp and m is a 5'-vinylphosphonate-2'-O-methyl-modified nucleotide, with X being A, T, C, G, or U |
| s | Representing that a linkage between two nucleotides adjacent to the letter s on the left and right sides is a phosphorothioate group linkage |

### Example 5. Assay of Free Uptake Activity of Conjugates in Primary Hepatocytes

On day 0, cryopreserved primary human hepatocytes (PHH) or primary cynomogus hepatocytes (PCH) were thawed. The cells were adjusted to 6 × 10⁵ cells/mL, and then 90 µL of the cell suspension was inoculated into a 96-well plate. The culture medium was Invitro GROCP Medium (BioIVT, Cat. No. Z990005) containing 10% FBS and 1% P/S. At the time of cell inoculation, 10 µL of the test GalNAc-siRNA was added to the wells of the plate; meanwhile, a control well without GalNAc-siRNA (PBS negative control group) was set. Each sample was tested at 3 or 2 concentrations, with 3 replicate wells for each concentration. After 48 h of culture, cells were collected to extract RNA. The mRNA expression level of the TTR gene (probe ID: Hs00174914) and the mRNA expression level of the internal reference gene GAPDH (probe ID: Hs02786624) in the samples were determined by RT-qPCR. Intracellular RNA was extracted using the RNeasy kit (QIAGEN-74182) according to the instructions, and reverse transcribed into cDNA using the Fast King RT Kit (with gDNase) (TIANGEN-KR116), followed by qPCR quantification.

The relative expression level of TTR mRNA was represented by 2^{-ΔΔCT}, and the calculation formulas were as follows:$Relative expression level of TTR mRNA = {2}^{- ΔΔCT}$ 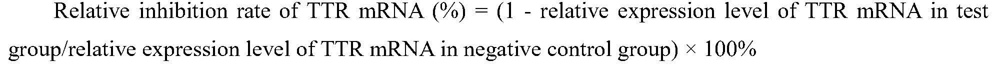 Relative inhibition rate of TTR mRNA (%) = (1 - relative expression level of TTR mRNA in test group/relative expression level of TTR mRNA in negative control group) × 100%

The results of the free uptake activity assay of the conjugates in primary hepatocytes are shown in Table 9, Table 10, and Table 11.

**Table 9. Free uptake activity results of conjugates in primary human hepatocytes**

| Conjugate No. | Mean inhibition rate in PHH (%) | | | Mean inhibition rate in PHH SD | | |
|---|---|---|---|---|---|---|
| | 20 nM | 10 nM | 5 nM | 20 nM | 10 nM | 5 nM |
| BPR-30203301 | 57.13 | 50.94 | 24.02 | 4.59 | 4.05 | 34.85 |
| BPR-30204501 | 77.74 | 65.28 | 60.60 | 5.70 | 2.37 | 4.42 |
| BPR-30204502 | 81.71 | 74.15 | 60.03 | 2.17 | 5.89 | 4.31 |
| BPR-30204503 | 38.68 | 11.84 | -4.95 | 4.98 | 3.62 | 21.27 |
| BPR-30204504 | 37.73 | -6.54 | -4.95 | 26.52 | 17.06 | 7.25 |
| BPR-30204505 | 67.71 | 28.80 | 18.45 | 7.55 | 19.29 | 14.61 |
| BPR-30213022 | 76.88 | 67.38 | 64.00 | 6.31 | 7.84 | 6.08 |
| BPR-30213002 | 82.15 | 76.05 | 69.33 | 3.55 | 1.18 | 2.86 |
| BPR-30213003 | 85.53 | 76.92 | 70.65 | 2.90 | 1.33 | 0.90 |
| BPR-30213004 | 75.70 | 61.98 | 50.30 | 1.50 | 0.70 | 6.01 |
| BPR-30213005 | 59.79 | 38.24 | 27.12 | 11.92 | 15.40 | 10.31 |
| BPR-30221618 | 86.91 | 74.51 | 68.17 | 4.15 | 3.44 | 3.28 |
| BPR-30221602 | 87.34 | 80.69 | 73.21 | 3.30 | 3.10 | 1.01 |
| BPR-30221603 | 89.91 | 82.47 | 69.49 | 0.41 | 1.45 | 4.72 |
| BPR-30221604 | 92.35 | 88.19 | 84.86 | 1.85 | 0.97 | 0.77 |
| BPR-30221605 | 94.45 | 92.05 | 88.55 | 0.35 | 0.81 | 0.24 |
| BPR-30220701 | 77.61 | 61.96 | 55.22 | 2.83 | 10.78 | 0.74 |
| BPR-30221201 | 65.41 | 58.44 | 33.81 | 13.12 | 11.81 | 1.89 |
| BPR-30221202 | 72.85 | 45.36 | 29.85 | 11.26 | 15.63 | 13.20 |
| BPR-30221203 | 77.59 | 50.31 | 52.36 | 1.37 | 5.01 | 13.17 |
| BPR-30221204 | 75.96 | 56.38 | 36.78 | 11.72 | 5.95 | 4.16 |
| BPR-30221205 | 77.91 | 75.06 | 56.59 | 3.33 | 3.29 | 3.24 |
| BPR-30222201 | 86.38 | 85.56 | 78.94 | 4.14 | 0.84 | 1.63 |
| BPR-30222202 | 82.58 | 79.23 | 63.25 | 5.03 | 1.62 | 6.58 |
| BPR-30222203 | 90.18 | 88.79 | 84.13 | 1.05 | 0.80 | 2.69 |
| BPR-30222204 | 57.54 | 48.91 | 33.38 | 7.80 | 4.52 | 5.56 |

The results in Table 9 show that: in the free uptake assay of primary human hepatocytes, at a dose of 20 nM, the mean value of the maximum inhibition rate of the TTR gene was 94.45%; at a dose of 10 nM, the mean value of the maximum inhibition rate of the TTR gene was 92.05%; at a dose of 5 nM, the mean value of the maximum inhibition rate of the TTR gene was 88.55%.

**Table 10. Free uptake activity results of conjugates in primary cynomogus hepatocytes**

| Conjugate No. | Mean inhibition rate in PCH (%) | | Mean inhibition rate in PCH SD | |
|---|---|---|---|---|
| | 20 nM | 10 nM | 20 nM | 10 nM |
| BPR-30221223 | 75.9 | 63.52 | 0.83 | 4.21 |
| BPR-30221615 | 84.24 | 62.66 | 7.11 | 5.26 |
| BPR-30221686 | 91.01 | 81.99 | 13.51 | 9.18 |
| BPR-30221618 | 80.34 | 67.73 | 0.96 | 5.74 |
| BPR-30221619 | 85.23 | 73.02 | 3.81 | 5.65 |
| BPR-30221677 | 89.81 | 79.59 | 15.39 | 14.28 |
| BPR-30221678 | 88.65 | 76.87 | 17.16 | 15.24 |
| BPR-30221679 | 87.21 | 81.33 | 8.86 | 4.27 |
| BPR-30221680 | 90.16 | 81.42 | 12.31 | 10.09 |
| BPR-30221681 | 89.59 | 82.54 | 7.11 | 8.37 |
| BPR-30221682 | 88.91 | 80.43 | 12.04 | 7.53 |
| BPR-30221683 | 92.61 | 84.26 | 10.82 | 7.61 |
| BPR-30221684 | 86.76 | 76.87 | 13.54 | 6.41 |
| BPR-30221685 | 88.06 | 75.38 | 9.66 | 16.37 |
| BPR-302 21228 | 80.52 | 69.18 | 10.22 | 8.56 |
| BPR-30221273 | 79.52 | 68.57 | 7.14 | 5.23 |
| BPR-30221275 | 78.16 | 69.64 | 3.21 | 4.16 |
| BPR-30221276 | 82.14 | 66.40 | 1.29 | 4.89 |
| BPR-30221277 | 80.07 | 67.89 | 6.77 | 8.49 |
| BPR-30221278 | 80.95 | 67.17 | 9.13 | 2.17 |
| BPR-30221279 | 79.14 | 65.78 | 10.58 | 12.36 |
| BPR-30221280 | 82.58 | 66.17 | 0.56 | 8.14 |
| BPR-30221281 | 81.56 | 68.18 | 3.72 | 5.33 |

The results in Table 10 show that: in the free uptake assay of primary cynomogus hepatocytes, at a dose of 20 nM, the mean value of the maximum inhibition rate of the TTR gene was 92.61%; at a dose of 10 nM, the mean value of the maximum inhibition rate of the TTR gene was 84.26%.

**Table 11. Free uptake activity results of conjugates in primary cynomogus hepatocytes**

| Conjugate No. | Mean inhibition rate in PCH (%) | | Mean inhibition rate in PCH SD | |
|---|---|---|---|---|
| | 20 nM | 10 nM | 20 nM | 10 nM |
| AD-649282 | 79.33 | 62.18 | 11.23 | 5.89 |
| BPR-30221201 | 81.92 | 68.79 | 9.38 | 12.17 |
| BPR-30221223 | 83.51 | 76.63 | 16.45 | 9.61 |
| AD-649286 | 75.41 | 56.38 | 5.96 | 8.47 |
| BPR-30221617 | 80.56 | 59.15 | 8.62 | 12.78 |
| BPR-30221618 | 76.89 | 60.15 | 4.52 | 6.55 |
| BPR-30221672 | 79.65 | 65.91 | 18.33 | 17.86 |
| BPR-30221676 | 80.54 | 68.62 | 10.21 | 11.33 |
| BPR-30221678 | 81.77 | 67.25 | 19.36 | 15.15 |
| BPR-30221686 | 82.31 | 72.66 | 9.37 | 9.29 |
| BPR-30222201 | 85.26 | 76.37 | 15.76 | 12.81 |
| BPR-30222203 | 87.19 | 80.24 | 17.39 | 15.67 |
| BPR-30222218 | 88.02 | 81.41 | 10.08 | 11.94 |

| | | | | |
|---|---|---|---|---|
| Note: AD-649282 and AD-649286 were derived from patent WO2023014677A1. | | | | |

The results in Table 11 show that: in the free uptake assay of primary cynomogus hepatocytes, at different screening concentrations, the conjugates screened in the present disclosure had equivalent or superior TTR gene inhibition effects compared with the positive controls.

### Example 6: Efficacy Evaluation in Humanized Mice

In this example, TTR humanized mice B6-hTTR (Jiangsu GemPharmatech Co., Ltd.) were injected subcutaneously in a single dose to study the ability of the pharmaceutical compositions to inhibit TTR gene expression *in vivo*. At least one week in advance, male mice aged 6-8 weeks were brought into the facility for adaptive rearing. During the adaptive rearing, blood collection (as pre-administration blood samples) and serum TTR protein assay were performed. After the adaptive rearing was completed, the mice were randomly grouped according to the body weight and pre-administration serum TTR protein content, with 3-5 animals per group. The AD-65492 (see Table 3 in CN108138182A) positive control group, the PBS negative control group, the BPR-30213022 experimental group, the BPR-30221223 experimental group, the BPR-30221686 experimental group, the BPR-30221617 experimental group, the BPR-30221618 experimental group, the BRP-30222201 experimental group, and the BPR-30222218 experimental group were set. According to the body weight of the mice, the administration dose of the single-dose subcutaneous injection was 3 mg/kg, and the administration volume was 10 µL/g; the day of administration was day 0. In addition, the BPR-30221672 experimental group, the BPR-30221676 experimental group, the BPR-30221678 experimental group, and the BPR-30221686 experimental group were set. According to the body weight of the mice, the administration dose of the single-dose subcutaneous injection was 1 mg/kg, and the administration volume was 10 µL/g; the day of administration was day 0. Blood samples were collected at different time points (day 7, day 14, day 21, day 28, day 35/day 42, etc.) after administration. About 100 µL of blood was collected and separated by centrifugation to obtain serum. Quantitative serum TTR protein assay was performed using an ELISA kit (Abcam ab231920), and the specific operations were as described in the kit instructions. The calculation formula for the relative inhibition rate of the serum TTR protein for each animal at each time point after administration was as follows: 100% × (TTR serum protein content at each time point after administration/TTR serum protein content of corresponding individual before administration - 1). The results are shown in FIG. 1 and FIG. 2.

FIG. 1 shows that on day 7 after administration, BPR-30213022, BPR-30221223, BPR-30221686, BPR-30221617, BPR-30221618, BRP-30222201, and BPR-30222218 can all knock down the serum TTR protein content in mice, with a maximum knockdown rate of 96%. On day 42 after administration, the maximum knockdown rate of the serum TTR protein in mice was 90%.Some of the conjugates had activity superior to that of AD-65492 in the patent CN108138182A.

FIG. 2 shows that on day 28 and day 35 after administration, the TTR protein inhibition rate of BPR-30221676 was weaker than those of BPR-30221672, BPR-30221678, and BPR-30221686, and the TTR protein inhibition rate of BPR-30221672 was weaker than those of BPR-30221678 and BPR-30221686.

### Example 7: Efficacy Evaluation in Cynomolgus Monkeys

In this example, non-human primates were injected subcutaneously in a single dose to study the ability of the pharmaceutical compositions to inhibit TTR gene expression *in vivo*. Wild male cynomolgus monkeys were arranged in advance for adaptive rearing. During the adaptive rearing, blood collection (as pre-administration blood samples) and serum TTR protein assay were performed. After the adaptive rearing was completed, the cynomolgus monkeys were randomly grouped according to the body weight and pre-administration serum TTR protein content, with 2-3 animals per group. The AD-65492 (see Table 3 in CN108138182A) positive control group, the PBS negative control group, the BPR-30221223 experimental group, the BPR-30221228 experimental group, the BPR-30221617 experimental group, the BPR-30221618 experimental group, the BRP-30221620 experimental group, the BPR-30221678 experimental group, the BPR-30221686 experimental group, the BRP-30222201 experimental group, and the BPR-30222218 experimental group were set. The administration dose of the single-dose subcutaneous injection was 1 g/kg, and the administration volume was 1 mL/kg. The day of administration was day 0. Blood samples were collected at different time points (day 7, day 14, day 21, day 28, day 35, day 42, etc.) after administration and separated by centrifugation to obtain serum. Quantitative serum TTR protein assay was performed using an ELISA kit (Abcam ab231920), and the specific operations were as described in the kit instructions. The calculation formula for the relative inhibition rate of the serum TTR protein for each animal at each time point after administration was as follows: 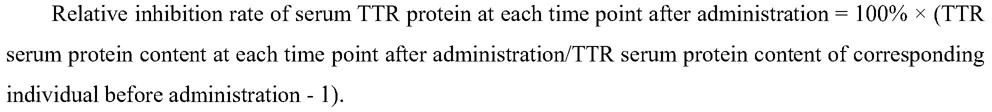 Relative inhibition rate of serum TTR protein at each time point after administration = 100% × (TTR serum protein content at each time point after administration/TTR serum protein content of corresponding individual before administration - 1).

The serum TTR protein content of the mice in each group decreased significantly over time. On day 35 after administration, the maximum inhibition rate of the experimental groups relative to that before administration reached up to 90%. Details of some experimental groups are shown in FIG. 3.

The specific embodiments of the present disclosure have been described in detail above, but the present disclosure is not limited to the specific details of the embodiments described above. Within the scope of the technical concept of the present disclosure, various simple modifications may be made to the technical solutions of the present disclosure. These simple modifications all belong to the protection scope of the present disclosure.

In addition, it should be noted that the various specific technical features described in the specific embodiments described above can be combined in any suitable manner provided that no contradiction arises. In order to avoid unnecessary repetition, the various possible combinations are not illustrated separately in the present disclosure.

In addition, various embodiments of the present disclosure may be combined arbitrarily, as long as they do not violate the idea of the present disclosure, and they should also be regarded as the content disclosed in the present disclosure.

## Claims

1. A double-stranded RNAi reagent, wherein the RNAi comprises a sense strand and an antisense strand capable of forming a double-stranded region, wherein the antisense strand is complementary to at least a portion of an mRNA encoding transthyretin (TTR), and the antisense strand comprises at least 15, 16, 17, 18, 19, 20, 21, 22, or 23 consecutive nucleotides differing by no more than 5, 4, 3, 2, or 1 nucleotide from any one of antisense strand nucleotide sequence among the antisense strand nucleotide sequences listed in Table 1;
preferably, the antisense strand is complementary to a fragment at positions 118-140, a fragment at positions 411-433, a fragment at positions 542-566, and/or a fragment at positions 581-603 of the TTR mRNA sequence as shown in SEQ ID NO: 884;
more preferably, the antisense strand comprises at least 15, 16, 17, 18, 19, 20, 21, 22, or 23 consecutive nucleotides differing by no more than 3, 2, or 1 nucleotide from any one of the following sequences: SEQ ID NOs: 90, 340, 424, 432, 444, 914, and 915.

2. The double-stranded RNAi reagent according to claim 1, wherein the RNAi comprises a sense strand and an antisense strand, wherein the sense strand comprises at least 15, 16, 17, 18, 19, 20, or 21 consecutive nucleotides differing by no more than 5, 4, 3, 2, or 1 nucleotide from any nucleotide sequence among the sense strand nucleotide sequences in Table 1;
preferably, the sense strand comprises at least 15, 16, 17, 18, 19, 20, or 21 consecutive nucleotides from a fragment at positions 118-140, a fragment at positions 411-433, a fragment at positions 542-566, and/or a fragment at positions 581-603 of the TTR mRNA sequence as shown in SEQ ID NO: 884;
preferably, the sense strand comprises at least 15, 16, 17, 18, 19, 20, or 21 consecutive nucleotides differing by no more than 3, 2, or 1 nucleotide from any one of the following sequences: SEQ ID NOs: 89, 339, 423, 431, and 443.

3. The double-stranded RNAi reagent according to claim 1 or 2, wherein the sense strand/antisense strand comprise, or consist of, the nucleotide sequences as shown in SEQ ID NOs: 89/90, SEQ ID NOs: 339/340, SEQ ID NOs: 417/418, SEQ ID NOs: 419/420, SEQ ID NOs: 421/422, SEQ ID NOs: 423/424, SEQ ID NOs: 425/426, SEQ ID NOs: 427/428, SEQ ID NOs: 429/430, SEQ ID NOs: 431/432, SEQ ID NOs: 431/914, SEQ ID NOs: 431/915, SEQ ID NOs: 433/434, SEQ ID NOs: 435/436, SEQ ID NOs: 437/438, SEQ ID NOs: 439/440, SEQ ID NOs: 441/442, SEQ ID NOs: 443/444, SEQ ID NOs: 445/446, SEQ ID NOs: 447/448, SEQ ID NOs: 449/450, SEQ ID NOs: 451/452, SEQ ID NOs: 453/454, SEQ ID NOs: 455/456, SEQ ID NOs: 457/458, SEQ ID NOs: 459/460, SEQ ID NOs: 461/462, SEQ ID NOs: 463/464, SEQ ID NOs: 465/466, SEQ ID NOs: 467/468, or SEQ ID NOs: 469/470, respectively.

4. The double-stranded RNAi reagent according to any one of claims 1-3, wherein the sense strand/antisense strand comprise, or consist of, the nucleotide sequences as shown in SEQ ID NOs: 89/90, SEQ ID NOs: 339/340, SEQ ID NOs: 423/424, SEQ ID NOs: 431/432, SEQ ID NOs: 431/914, SEQ ID NOs: 431/915, or SEQ ID NOs: 443/444, respectively.

5. The double-stranded RNAi reagent according to any one of claims 1-4, comprising at least one modified nucleotide,
preferably, at least one modified nucleotide in the modified nucleotides is selected from one or more of the following: a 2'-O-methyl-modified nucleotide, a 2'-fluoro-modified nucleotide, a 2'-O-methoxyethyl-modified nucleotide, an L-2'-fluoro-modified nucleotide, a 2'-deoxyribonucleotide, a 2'-amino-modified nucleotide, a 2'-alkoxy-modified nucleotide, a 2',3'-seco-nucleotide mimic, an L-2'-O-methyl-modified nucleotide, an L-2'-deoxyribonucleotide, a locked nucleotide, a threose nucleic acid, a 4'-modified threose nucleic acid, a reverse nucleotide, a reverse 2'-O-methyl-modified nucleotide, a reverse 2'-deoxyribonucleotide, a glycol nucleotide, and a 5'-vinylphosphate nucleotide;
more preferably, at least one modified nucleotide in the modified nucleotides is selected from one or more of the following: a 4'-modified threose nucleic acid, a 2'-O-methyl-modified nucleotide, a 2'-fluoro-modified nucleotide, and a 2'-deoxy-modified nucleotide.

6. The double-stranded RNAi reagent according to any one of claims 1-5, comprising at least one 4'-modified threose nucleic acid,
wherein preferably, the 4'-modified threose nucleic acid has a structure of formula (A1) below: 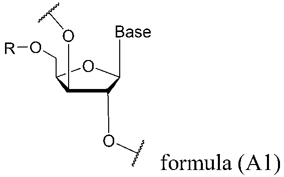
wherein Base represents a natural or modified nucleobase, the natural nucleobase being A, T, C, G, or U, and R represents an alkyl group having 1-30 carbon atoms;
preferably, the threose nucleic acid is located at the 5'-terminal end of the sense strand of the RNAi reagent, and R represents an alkyl group having 10-30 carbon atoms;
more preferably, the threose nucleic acid is a 4'-modified threose nucleic acid having a structure of formula (A1') below: 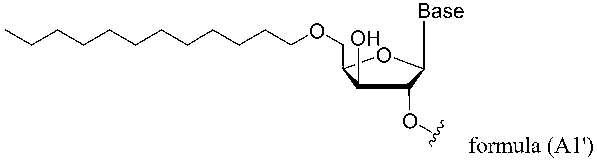
wherein Base is a natural nucleobase A, T, C, G, or U.

7. The double-stranded RNAi reagent according to any one of claims 1-6, wherein at least one nucleotide is linked to one or two nucleotides adjacent thereto via a phosphoester group or a phosphorothioate group.

8. A double-stranded RNAi reagent for inhibiting transthyretin (TTR) gene expression, comprising a sense strand and an antisense strand, wherein at least 18 nucleotides are reverse complementary between the sense strand and the antisense strand;
the sense strand comprises, consists of, or consists substantially of a sequence represented by general formula (I) below:
5'-n₁-n₂-n₃-n₄-n₅-n₆-n₇-n₈-n₉-n₁₀-n₁₁-n₁₂-n₁₃-n₁₄-n₁₅-n₁₆₋n₁₇-n₁₈-n₁₉-n₂₀-n₂₁-3' general formula (I);
the antisense strand comprises, consists of, or consists substantially of a sequence represented by general formula (II) below:
5'-N₁-N₂-N₃-N₄-N₅-N₆-N₇-N₈-N₉-N₁₀-N₁₁-N₁₂-N₁₃-N₁₄-N₁₅-N₁₆-N₁₇-N₁₈-N₁₉-N₂₀-N₂₁-N₂₂-N₂₃-3' general formula (II),
wherein n₁ to n₂₁ in general formula (I) represent 21 consecutive nucleotides comprised in the sense strand, N₁ to N₂₃ in general formula (II) represent 23 consecutive nucleotides comprised in the antisense strand, and each n and N is independently a modified nucleotide.

9. The RNAi reagent according to claim 8, wherein a sequence of the antisense strand comprises a sequence motif, the sequence motif being complementary to a portion of an mRNA sequence of a TTR gene.

10. The RNAi reagent according to claim 8 or 9, wherein the complementarity is partial, substantial, or complete reverse complementarity.

11. The RNAi reagent according to claim 9 or 10, wherein the sequence motif is complementary to a fragment at positions 118-140, a fragment at positions 411-433, a fragment at positions 542-566, and/or a fragment at positions 581-603 of the TTR mRNA sequence as shown in SEQ ID NO: 884;
preferably, the motif comprises at least 15, 16, 17, 18, 19, 20, 21, 22, or 23 consecutive nucleotides differing by no more than 3, 2, or 1 nucleotide from any one of the following sequences: SEQ ID NOs: 90, 340, 424, 432, 444, 914, and 915.

12. The RNAi reagent according to any one of claims 8-11, wherein the modified nucleotide is selected from: a 2'-O-methyl-modified nucleotide, a 2'-fluoro-modified nucleotide, a 2'-O-methoxyethyl-modified nucleotide, an L-2'-fluoro-modified nucleotide, a 2'-deoxyribonucleotide, a 2'-amino-modified nucleotide, a 2'-alkoxy-modified nucleotide, a 2',3'-seco-nucleotide mimic, an L-2'-O-methyl-modified nucleotide, an L-2'-deoxyribonucleotide, a locked nucleotide, a threose nucleic acid, a 4'-modified threose nucleic acid, a reverse nucleotide, a reverse 2'-O-methyl-modified nucleotide, a reverse 2'-deoxyribonucleotide, a glycol nucleotide, and a 5'-vinylphosphate nucleotide.

13. The RNAi reagent according to any one of claims 8-12, wherein nucleotides at positions n₁ and n₂₁ are each a threose nucleic acid, a 4'-modified threose nucleic acid, a reverse 2'-deoxyribonucleotide, a reverse nucleotide, an L-2'-O-methyl-modified nucleotide, an L-2'-deoxyribonucleotide, or a 2'-O-methyl-modified nucleotide.

14. The RNAi reagent according to any one of claims 8-13, wherein positions n₇, n₉, n₁₀, and n₁₁ are each a 2'-deoxyribonucleotide or a 2'-fluoro-modified nucleotide.

15. The RNAi reagent according to any one of claims 8-14, wherein positions n₂, n₃, n₄, n₅, n₆, n₈, n₁₂, n₁₃, n₁₄, n₁₅, n₁₆, n₁₇, n₁₈, n₁₉, and n₂₀ are each a 2'-O-methyl-modified nucleotide.

16. The RNAi reagent according to any one of claims 8-15, wherein the 5' end of the sequence represented by general formula (I) comprises no less than 1 phosphorothioate group; preferably, one or more of a linkage between n₁ and n₂ and a linkage between n₂ and n₃ are achieved by a phosphorothioate group.

17. The RNAi reagent according to any one of claims 8-16, wherein position N₁ is a 5'-vinylphosphate-2'-O-methyl-modified nucleotide, a 2'-O-methyl-modified nucleotide, a threose nucleic acid, a 4'-modified threose nucleic acid, or a reverse nucleotide.

18. The RNAi reagent according to any one of claims 8-17, wherein position N₃ is a 2'-O-methoxyethyl-modified nucleotide or a 2'-O-methyl-modified nucleotide.

19. The RNAi reagent according to any one of claims 8-18, wherein position N₅ is a 2'-deoxyribonucleotide or a 2'-O-methyl-modified nucleotide.

20. The RNAi reagent according to any one of claims 8-19, wherein positions N₆ and N₁₁ are each a 2'-O-methyl-modified nucleotide, a 2'-O-methoxyethyl-modified nucleotide, or a 2'-fluoro-modified nucleotide.

21. The RNAi reagent according to any one of claims 8-20, wherein position N₇ is a 2'-deoxyribonucleotide, a 2'-O-methyl-modified nucleotide, a 2',3'-seco-nucleotide mimic, or a glycol nucleotide.

22. The RNAi reagent according to any one of claims 8-21, wherein the RNAi reagent comprises a 4'-modified threose nucleic acid, e.g., a 4'-modified threose nucleic acid having formula (A1) below: 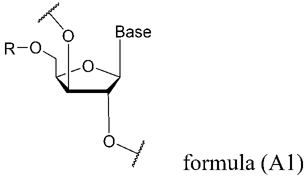
wherein Base represents a natural or modified nucleobase, the natural nucleobase being A, T, C, G, or U, and R represents an alkyl group having 1-30 carbon atoms;
preferably, the threose nucleic acid is located at the 5'-terminal end of the sense strand of the RNAi reagent, and R represents an alkyl group having 10-30 carbon atoms;
more preferably, the threose nucleic acid is a 4'-modified threose nucleic acid having a structure of formula (A1') below: 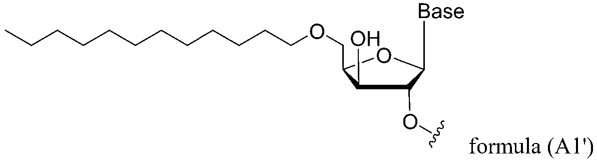
wherein Base is a natural nucleobase A, T, C, G, or U.

23. The RNAi reagent according to any one of claims 8-22, wherein one or two of positions N₂₂ to N₂₃ are each an L-2'-O-methyl-modified nucleotide, an L-2'-deoxyribonucleotide, a threose nucleic acid, a 4'-modified threose nucleic acid, a reverse 2'-deoxyribonucleotide, a reverse nucleotide, a reverse 2'-O-methyl-modified nucleotide, or a 2'-O-methyl-modified nucleotide;
preferably, the 4'-modified threose nucleic acid is as defined in claim 6 or 22.

24. The RNAi reagent according to any one of claims 8-23, wherein positions N₂, N₁₄, and N₁₆ are each a 2'-deoxyribonucleotide or a 2'-fluoro-modified nucleotide.

25. The RNAi reagent according to any one of claims 8-24, wherein N₄, N₈, N₉, N₁₀, N₁₂, N₁₃, N₁₅, N₁₇, N₁₈, N₁₉, N₂₀, and N₂₁ are each a 2'-O-methyl-modified nucleotide.

26. The RNAi reagent according to any one of claims 8-25, wherein the 5' end and the 3' end of the sequence represented by general formula (II) each comprise no less than 1 phosphorothioate group; preferably, one or more of a linkage between N₁ and N₂, a linkage between N₂ and N ₃, a linkage between N₂₁ and N₂₂, and a linkage between N₂₂ and N₂₃ are achieved by a phosphorothioate group.

27. The RNAi reagent according to any one of claims 1-26, wherein the antisense strand comprises at least 15 consecutive nucleotides (preferably at least 16, 17, 18, or 19 consecutive nucleotides, and more preferably at least 20, 21, 22, or 23 consecutive nucleotides) from any one of the modified antisense strands listed in Table 6; or the antisense strand differs by no more than 3, 2, or 1 nucleotide within at least 15 consecutive nucleotides (preferably at least 16, 17, 18, or 19 consecutive nucleotides, and more preferably at least 20, 21, 22, or 23 consecutive nucleotides) from the modified antisense strand; or the antisense strand comprises at least 15 consecutive nucleotides differing by no more than 3, 2, or 1 nucleotide from the modified antisense strand;
preferably, the antisense strand comprises, consists of, or consists substantially of any one of the modified antisense strands listed in Table 6.

28. The RNAi reagent according to any one of claims 1-26, wherein the sense strand comprises at least 15 consecutive nucleotides (preferably at least 16, 17, 18, or 19 consecutive nucleotides, and more preferably at least 20 or 21 consecutive nucleotides) from any one of the modified sense strands listed in Table 7; or the sense strand differs by no more than 3, 2, or 1 nucleotide within at least 15 consecutive nucleotides (preferably at least 16, 17, 18, or 19 consecutive nucleotides, and more preferably at least 20 or 21 consecutive nucleotides) from the modified sense strand; or the sense strand comprises at least 15 consecutive nucleotides differing by no more than 3, 2, or 1 nucleotide from the modified sense strand;
preferably, the sense strand comprises, consists of, or consists substantially of any one of the modified sense strands listed in Table 7.

29. The RNAi reagent according to any one of claims 1-28, wherein the sense strand consists of 21 nucleotides, and the antisense strand consists of 23 nucleotides.

30. The RNAi reagent according to any one of claims 1-29, wherein the RNAi reagent is obtained by independently modifying nucleotides at each position of the sense strand and the antisense strand shown in Table 2.

31. The RNAi reagent according to any one of claims 1-30, wherein the sense strand and the antisense strand are as shown in Table 7 and Table 6, respectively.

32. The RNAi reagent according to any one of claims 1-31, wherein the antisense strand comprises, consists of, or consists substantially of a sequence obtained by independently modifying nucleotides at each position of a nucleotide sequence differing by 0, 1, 2, or 3 nucleotides from one of the following nucleotide sequences:
5'-AUCUAGAACUUUGACCAUCAGAG-3' (SEQ ID NO: 90)
5'-UAGGAGUAGGGGCUCAGCAGGGC-3' (SEQ ID NO: 340)
5'-UAGGUGAAAACACUGCUUUAGGG-3' (SEQ ID NO: 424)
5'-AUAUGAGGUGAAAACACUGCUGG-3' (SEQ ID NO: 432)
5'-AAUGUUUUAUUGUCUCUGCCUGG-3' (SEQ ID NO: 444)
5'-AUAUGAGGUGAAAACACUGCUUU-3' (SEQ ID NO: 914)
5'-AUAUGAGGUGAAAACACUGCUAU-3' (SEQ ID NO: 915).

33. The RNAi reagent according to any one of claims 1-32, wherein the sense strand comprises, consists of, or consists substantially of a sequence obtained by independently modifying nucleotides at each position of a nucleotide sequence differing by 0, 1, 2, or 3 nucleotides from one of the following nucleotide sequences:
5'-CUGAUGGUCAAAGUUCUAGAU-3' (SEQ ID NO: 89)
5'-CCUGCUGAGCCCCUACUCCUA-3' (SEQ ID NO: 339)
5'-CUAAAGCAGUGUUUUCACCUA-3' (SEQ ID NO: 423)
5'-AGCAGUGUUUUCACCUCAUAU-3' (SEQ ID NO: 431)
5'-AGGCAGAGACAAUAAAACAUU-3' (SEQ ID NO: 443).

34. The RNAi reagent according to any one of claims 1-33, comprising a duplex obtained by independently modifying nucleotides at each postion of each strand of any pair of the following double strands:
sense strand: 5'-CUGAUGGUCAAAGUUCUAGAU-3' (SEQ ID NO: 89) and
antisense strand: 5'-AUCUAGAACUUUGACCAUCAGAG-3' (SEQ ID NO: 90);
sense strand: 5'-CCUGCUGAGCCCCUACUCCUA-3' (SEQ ID NO: 339) and
antisense strand: 5'-UAGGAGUAGGGGCUCAGCAGGGC-3' (SEQ ID NO: 340);
sense strand: 5'-CUAAAGCAGUGUUUUCACCUA-3' (SEQ ID NO: 423) and
antisense strand: 5'-UAGGUGAAAACACUGCUUUAGGG-3' (SEQ ID NO: 424);
sense strand: 5'-AGCAGUGUUUUCACCUCAUAU-3' (SEQ ID NO: 431) and
antisense strand: 5'-AUAUGAGGUGAAAACACUGCUGG-3' (SEQ ID NO: 432);
sense strand: 5'-AGCAGUGUUUUCACCUCAUAU-3' (SEQ ID NO: 431) and
antisense strand: 5'-AUAUGAGGUGAAAACACUGCUUU-3' (SEQ ID NO: 914);
sense strand: 5'-AGCAGUGUUUUCACCUCAUAU-3' (SEQ ID NO: 431) and
antisense strand: 5'-AUAUGAGGUGAAAACACUGCUAU-3' (SEQ ID NO: 915);
sense strand: 5'-AGGCAGAGACAAUAAAACAUU-3' (SEQ ID NO: 443) and
antisense strand: 5'-AAUGUUUUAUUGUCUCUGCCUGG-3' (SEQ ID NO: 444).

35. The RNAi reagent according to any one of claims 1-34, wherein the antisense strand comprises, consists of, or consists substantially of a modified nucleotide sequence differing by 0, 1, 2, or 3 nucleotides from one of the following modified nucleotide sequences:
5'-UmsAfsGmGmUmGfAmAmAmAmCmAmCmUfGmCfUmUmUmAmGmsiGsiG-3' (SEQ ID NO: 597)
5'-UmsAfsGmGmdTGmdAAmAmAmCmAmCmUfGmCfUmUmUmAmGmsGmsGm-3' (SEQ ID NO: 604)
5'-AmsUfsAmUmGmAfGmGmUmGmAmAmAmAfCmAfCmUmGmCmUmsGmsGm-3' (SEQ ID NO: 606)
5'-AmsUfsAmUmdGAmdGGmUmGmAmAmAmAfCmAfCmUmGmCmUmsGmsGm-3' (SEQ ID NO: 620)
5'-VpUmsUfsAmUmdGAmdGGmUmGmAmAmAmAfCmAfCmUmGmCmUmsGmsGm-3' (SEQ ID NO: 621)
5'-AmsAfsUmGmUmUfUmUmAmUmU(moe)GmUmCfUmCfUmGmCmCmUmsGmsGm-3' (SEQ ID NO: 644)
5'-AmsAfsUmGmUmUfdTUmAmUmUmGmUmCfUmCfUmGmCmCmUmsGmsGm-3' (SEQ ID NO: 655)
5'-AmsUfsAmUmdGAmdGGmUmGmAmAmAmAfCmAfCmUmGmCmUmsUmsUm-3' (SEQ ID NO: 889)
5'-AmsUfsAmUmdGAmdGGmUmGmAmAmAmAfCmAfCmUmGmCmUmsAmsUm-3' (SEQ ID NO: 891)
wherein the uppercase letters "G", "C", "A", and "U" represent ribonucleotides containing guanine, cytosine, adenine, and uracil as bases, respectively; "T" represents a deoxyribonucleotide containing thymine as a base; the lowercase letter "m" represents that one nucleotide adjacent to the letter m on the left side is a 2'-O-methyl-modified nucleotide; the lowercase letter "f" represents that one nucleotide adjacent to the letter f on the left side is a 2'-fluoro-modified nucleotide; "moe" represents that one nucleotide adjacent to the letter moe on the left side is a 2'-O-methoxyethyl-modified nucleotide; "Vp" represents that one nucleotide adjacent to the letter Vp on the right side is a 5'-vinylphosphate nucleotide; the lowercase letter "i" represents that one nucleotide adjacent to the letter i on the right side is a reverse nucleotide; the lowercase letter "d" represents that one nucleotide adjacent to the letter d on the right side is a 2'-deoxyribonucleotide; the lowercase letter "s" represents that a linkage between two nucleotides adjacent to the letter s on the left and right sides is a phosphorothioate group linkage.

36. The RNAi reagent according to any one of claims 1-35, wherein the sense strand comprises, consists of, or consists substantially of a modified nucleotide sequence differing by 0, 1, 2, or 3 nucleotides from one of the following modified nucleotide sequences:
5'-ChsUmsAmAmAmGmCfAmGfUfGfUmUmUmUmCmAmCmCmUmAm-3' (SEQ ID NO: 601)
5'-iCsUmsAmAmAmGmCfAmGfUfGfUmUmUmUmCmAmCmCmUmAm-3' (SEQ ID NO: 603)
5'-AmsGmsCmAmGmUmGfUmUfUfUfCmAmCmCmUmCmAmUmAmUm-3' (SEQ ID NO: 605)
5'-AhsGmsCmAmGmUmGfUmUfUfUfCmAmCmCmUmCmAmUmAmUm-3' (SEQ ID NO: 617)
5'-AmsGmsGmCmAmGmAfGmAfCfAfAmUmAmAmAmAmCmAmUmUm-3' (SEQ ID NO: 641)
5'-AhsGmsGmCmAmGmAfGmAfCfAfAmUmAmAmAmAmCmAmUmUm-3' (SEQ ID NO: 653)
5'-AhsGmsCmAmGmUmGfUmUfUfUfCmAmCmCmUmCmAmUmAmAm-3' (SEQ ID NO: 744)
wherein the uppercase letters "G", "C", "A", and "U" represent ribonucleotides containing guanine, cytosine, adenine, and uracil as bases, respectively; the lowercase letter "m" represents that one nucleotide adjacent to the letter m on the left side is a 2'-O-methyl-modified nucleotide; the lowercase letter "f" represents that one nucleotide adjacent to the letter f on the left side is a 2'-fluoro-modified nucleotide; the lowercase letter "h" represents that one nucleotide adjacent to the letter h on the left side is a 4'-modified threose nucleic acid; "i" represents that one nucleotide adjacent to the letter i on the right side is a reverse nucleotide; the lowercase letter "s" represents that a linkage between two nucleotides adjacent to the letter s on the left and right sides is a phosphorothioate group linkage.

37. The RNAi reagent according to any one of claims 1-36, comprising any one of the following groups: SEQ ID NOs: 601 and 597, SEQ ID NOs: 601 and 604, SEQ ID NOs: 603 and 597, SEQ ID NOs: 603 and 604, SEQ ID NOs: 605 and 606, SEQ ID NOs: 605 and 620, SEQ ID NOs: 605 and 889, SEQ ID NOs: 617 and 606, SEQ ID NOs: 617 and 620, SEQ ID NOs: 744 and 621, SEQ ID NOs: 641 and 644, SEQ ID NOs: 641 and 655, SEQ ID NOs: 653 and 644, SEQ ID NOs: 653 and 655, SEQ ID NOs: 751 and 620, and SEQ ID NOs: 751 and 891.

38. The RNAi reagent according to any one of claims 1-37, wherein the sense strand and/or the antisense strand are/is linked to a targeting ligand to form a conjugate.

39. The RNAi reagent according to claim 38, wherein the targeting ligand comprises one or more (e.g., 1, 2, 3, or 4) N-acetylgalactosamine and/or a derivative thereof, wherein the N-acetylgalactosamine has a structural formula represented by formula (B-I):

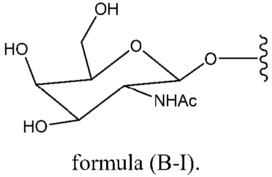

40. The RNAi reagent according to claim 38 or 39, wherein the targeting ligand has a structural formula represented by formula (B-II):

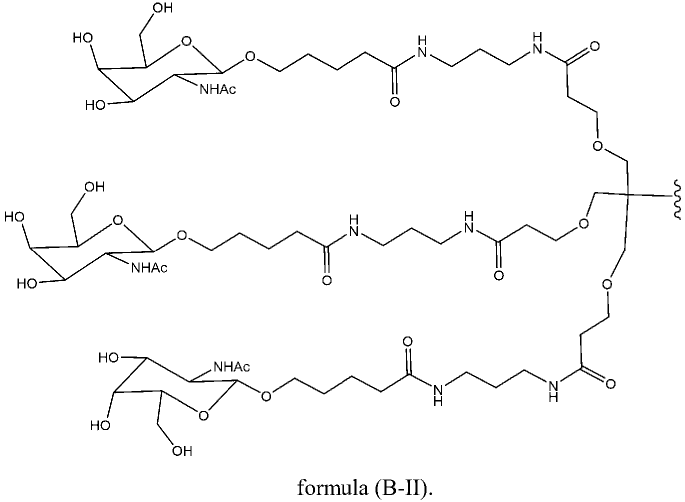

41. The RNAi reagent according to any one of claims 38-40, wherein the targeting ligand is conjugated to the sense strand; preferably, the targeting ligand is conjugated to the 3' end or the 5' end of the sense strand.

42. The RNAi reagent according to any one of claims 38-41, wherein the conjugate has a structural formula represented by formula (B-III): 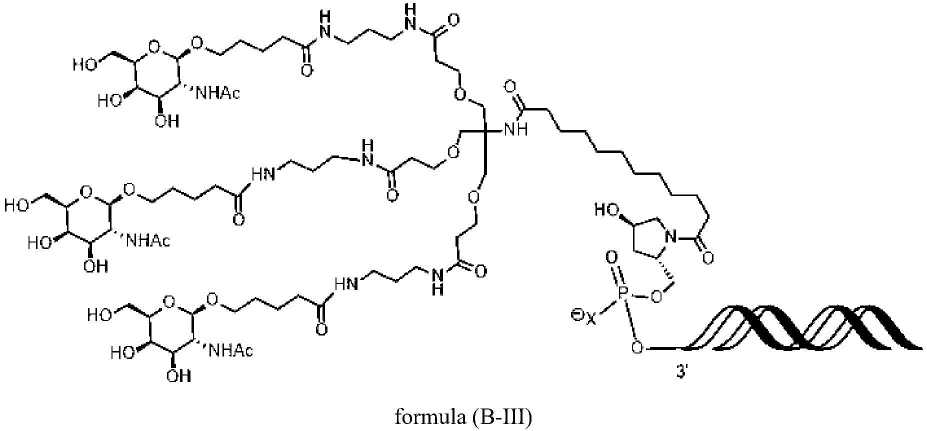 wherein 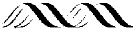 represents a double-stranded oligonucleotide of the RNAi, 3' represents the 3' end of the sense strand, and X is O or S.

43. The RNAi reagent according to any one of claims 38-42, wherein the conjugate is as shown in Table 5, preferably BPR-30221218, BPR-30221221, BPR-30221223, BPR-30221228, BPR-30221686, BPR-30221617, BPR-30221618, BPR-30221620, BPR-30221672, BPR-30221676, BPR-30221678, BPR-30222203, BPR-30222212, and BPR-30222218.

44. A cell, comprising the RNAi reagent according to any one of claims 1-43.

45. A composition, comprising the RNAi reagent according to any one of claims 1-43 and a pharmaceutically acceptable diluent, carrier, and/or excipient; wherein preferably, the diluent is a PBS buffer, normal saline, or water; more preferably, the composition further comprises one or more additional therapeutic agents.

46. A pharmaceutical combination, comprising the RNAi reagent according to any one of claims 1-43 and one or more additional therapeutic agents, wherein the additional therapeutic agents are, for example, any therapeutic agent effective in preventing or treating a disease and/or condition mediated by TTR gene expression.

47. A method for inhibiting TTR gene expression in a cell *in vivo* or *in vitro*, comprising introducing an effective amount of the RNAi reagent according to any one of claims 1-43 or the composition according to claim 45 or the pharmaceutical combination according to claim 46 into the cell.

48. The method according to claim 47, wherein the cell is in a subject; preferably, the subject is a human.

49. The method according to claim 47 or 48, wherein the TTR gene expression is inhibited by at least about 50%.

50. Use of the RNAi reagent according to any one of claims 1-43 or the composition according to claim 45 in preparing a medicament for treating and/or preventing a disease, condition, or symptom mediated at least in part by TTR gene expression.

51. The use according to claim 50, wherein the disease comprises ATTR-PN (transthyretin amyloid polyneuropathy), ATTR-CM (transthyretin amyloid cardiomyopathy), senile systemic amyloidosis diseases, and leptomeningeal/central nervous system amyloidosis diseases.

52. A method for treating and/or preventing a disease, condition, or symptom mediated at least in part by TTR gene expression, comprising administering to a patient in need thereof a therapeutically effective amount of the RNAi reagent according to any one of claims 1-38 or the composition according to claim 40, wherein preferably, the patient is a human.

53. The method according to claim 52, wherein the disease comprises ATTR-PN (transthyretin amyloid polyneuropathy), ATTR-CM (transthyretin amyloid cardiomyopathy), senile systemic amyloidosis diseases, and leptomeningeal/central nervous system amyloidosis diseases.

54. The method according to claim 52 or 53, wherein the RNAi reagent or the composition is administered to the patient by subcutaneous, intravenous, intramuscular, intrabronchial, intrapleural, intraperitoneal, intraarterial, lymphatic, and/or cerebrospinal administration.

55. The method according to any one of claims 52-54, wherein the RNAi reagent or the composition is delivered to the liver, choroid plexus, retina, and/or pancreas of the patient.

56. The method according to any one of claims 52-55, wherein an administration dose of the RNAi reagent is about 1-300 mg/kg body weight.

57. The method according to any one of claims 52-56, wherein an administration cycle of the RNAi reagent is once or more times every day, every week, every two weeks, every three weeks, every month, every 2 months, every 3 months, every 4 months, every 5 months, every 6 months, every 7 months, every 8 months, every 9 months, every 10 months, every 11 months, or every 12 months.

58. A kit, comprising the RNAi reagent according to any one of claims 1-43 or the composition according to claim 45.
